# EUROPEAN PATENT APPLICATION

(11) **EP 4 388 972 A1**
(43) Date of publication of application: **26.06.2024**
(21) Application number: 22858663.2
(22) Date of filing: 09.08.2022
(51) Int. Cl.: A47L 23/20, F26B 21/08, F26B 5/16, F26B 3/06, F26B 21/00, A61L 2/07, A61L 2/26, A47B 61/04

(54) **SHOE CARE APPARATUS**

(30) Priority: 17.08.2021 KR 20210108271
(71) Applicant: LG Electronics Inc., Yeongdeungpo-gu Seoul 07336 (KR)
(72) Inventor: KIM, Ji Hun, Seoul 08592 (KR); LEE, Young Jae, Seoul 08592 (KR); CHOI, Kyoung Min, Seoul 08592 (KR)
(74) Representative: Schornack, Oliver
(86) International application number: PCT/KR2022/011824
(87) International publication number: WO 2023/022425

(57) **Abstract**

Provided is a shoe care device for processing shoes by air circulation. A shoe care device according to an aspect of the present disclosure includes: an inner cabinet configured to accommodate shoes therein; a steam generator configured to supply steam into the inner cabinet; an inlet through which air is sucked from the inner cabinet; a nozzle installed inside the inner cabinet to be insertable into the shoe and configured to spray at least one of steam and air into the shoe; a connecting passage connected from the inlet to the nozzle; a blowing fan installed on the connecting passage to blow air from the inlet toward the nozzle; and a dehumidifying material installed on the connecting passage to dehumidify the blown air.

## Description

### TECHNICAL FIELD

The present disclosure relates to a shoe care device and, more specifically, to a shoe care device for processing shoes through air circulation.

### BACKGROUND

Shoes may get wet by a wearer's sweat, external contaminants, or rain or snow.

Wearing such shoes may make the wearer uncomfortable, and in this condition, germs may also breed or the shoes may stink.

For the shoes care device, Korean Patent No. 1037245 (hereinafter, referred to as "Prior Art Document 1"), entitled, "Apparatus for sterilization disposal of shoes", includes a main body, an ultraviolet emission module, and a deodorization module.

According to Prior Art Document 1 above, the shoes are disposed in a sterilization chamber of the main body, and the ultraviolet emission module is driven to remove germs and odors of the shoes. Furthermore, the air in the sterilization chamber is sucked into a ventilation pipe and discharged to the outside of the main body through an exhaust port through the deodorization module.

Here, the deodorization module includes a deodorization column made up of materials such as zeolite, activated carbon, and charcoal, and removes contaminants from the air discharged from the inside of the main body to the outside by the deodorization column.

According to Prior Art Document 1 above, the air from which moisture has been removed by a deodorization module including zeolite and activated carbon can be discharged to the outside of the apparatus for sterilization disposal of shoes.

However, in Prior Art Document 1 above, since the air is discharged to the outside of the apparatus for sterilization disposal of shoes, the air that has not sufficiently removed moisture or odors may be discharged to the outside of the apparatus for sterilization disposal of shoes, and such air may be discharged to the inside where the wearer resides.

In addition, Korean Patent Unexamined Publication No. 10-2000-0009653 (hereinafter referred to as "Prior Art Document 2"), entitled, "The shoes cabinet for sanitation", discloses a shoe closet that includes a main body, a far infrared radiation part, a circulation fan, an air circulation passage, and a sanitary filter portion.

According to Prior Art Document 2 above, while storing shoes in shoe closet, hygiene treatments such as dehumidification, sterilization, and deodorization can be performed on the shoes by far-infrared rays and a filter during the storing of the shoes.

Here, since the sanitary filter portion is filled with excellent adsorptive materials such as charcoal, it can serve to adsorb moisture in the process of ventilating air and filter bacteria, as well as to capture malodorous substances.

According to Prior Art Document 2 above, the air is circulated by a circulating fan in a shoe closet, and the sanitary filter portion is disposed on a circulation path of the air to remove germs and odors in the air.

However, since Prior Art Document 2 above does not consider a technology to prevent the reduction of the performance of the sanitary filter portion configured to remove moisture or odors, a user may not be satisfied because the shoes are not properly treated at the time of using a shoes care device.

As described above, for the shoes care device that removes germs or odors by treating the shoes in a predetermined manner, a task that has to be solved to ensure proper performance for shoes treatment while preventing the air used for dehumidification and deodorization from being exposed to the user is present in in the process of treating the shoes.

However, there is a limitation in that the conventional shoes care device cannot properly solve such a task.

In addition, when designing and manufacturing devices including a dehumidifier such as zeolite, it is necessary to consider how the dehumidification efficiency of a dehumidifying material can be further improved, whether the dehumidifying material can be effectively regenerated, whether water vapor generated during the regeneration of the dehumidifying material can be effectively discharged, whether moisture is left in an unintended area during the regeneration of the dehumidifying, whether components are properly disposed in a limited space, and where the devices provides excellent use convenience. There is a need to develop a shoes care device that takes these matters into consideration.

### DISCLOSURE

### TECHNICAL PROBLEM

The present disclosure describes a shoe care device configured to treat shoes by circulating air.

For example, the present disclosure describes a shoe care device that dehumidifies and deodorizes shoes using a dehumidifying material to refresh the shoes, and regenerates the used dehumidifying material, thereby always maintaining appropriate performance of processing shoes.

The present disclosure further describes a shoe care device that has an air circulation structure in which the air inside the inner cabinet where the shoes are placed is dehumidified using a dehumidifying material and in which the dehumidified air is supplied back into the inner cabinet, thereby preventing the user from being exposed to the air used in dehumidification and deodorization of shoes.

The present disclosure further describes a shoe care device that operates in the most appropriate mode in consideration of the condition of the shoes to be treated, thereby further improving the processing efficiency of shoes.

The technical problems to be solved in the present disclosure are not limited to the technical problems mentioned above, and other technical problems that have not been mentioned will be clearly understood by those of ordinary skill in the art to which the present disclosure pertains from the description below.

### TECHNICAL SOLUTION

In some implementations, the shoe care device can be configured to dehumidify and deodorize shoes using a dehumidifying material and regenerate the used dehumidifying material. Specifically, the dehumidifying material may be disposed in an air supplier to capture moisture and bacteria in the blown air, and the dehumidifying material can be regenerated by being heated by the air supplier.

In addition, the shoe care device can be configured to have an air circulation structure in which the air used in dehumidification and deodorization of shoes circulates inside the shoe care device. Specifically, the shoe care device can be configured such that a connection path through which air circulates is formed between an inlet and an outlet formed inside an inner cabinet.

In addition, the shoe care device according to an aspect of the present disclosure is configured such that the direction of spraying steam and air onto the shoes may be adjusted in consideration of the condition of the shoes. Specifically, the nozzle is installed to be inserted into the shoe so as to spray at least one of steam and air into the shoe, and is configured such that the insertion direction of the nozzle may be adjusted.

In addition, in the shoe care device according to an aspect of the present disclosure, the nozzle may be inserted into the shoe from above and may spray both steam and air into the shoe for treatment.

In addition, in the shoe care device according to an aspect of the present disclosure, the nozzle may be inserted into the shoe from the bottom and may spray only air into the shoe for treatment.

In addition, in the shoe care device according to an aspect of the present disclosure, the nozzle may be hinge-coupled to the nozzle duct installed to protrude to the inside of the inner cabinet on the connecting passage.

In addition, in the shoe care device according to an aspect of the present disclosure, the nozzle duct may be hinge-coupled to the inner cabinet.

In addition, in the shoe care device according to an aspect of the present disclosure, the nozzle duct may be installed on one upper side of the inner cabinet.

In addition, in the shoe care device according to an aspect of the present disclosure, spraying may be performed in the opposite directions from the end of the nozzle.

In addition, in the shoe care device according to an aspect of the present disclosure, a pair of nozzles may be installed to branch off from the nozzle duct.

In addition, in the shoe care device according to an aspect of the present disclosure, a plurality of nozzles may be disposed to branch off from the distribution housing disposed to extend in one direction on the connecting passage.

In addition, the shoe care device according to an aspect of the present disclosure may be configured to further include a dry air duct coupled to a portion of the distribution housing on the connecting passage.

In addition, in the shoe care device according to an aspect of the present disclosure, the steam generator may be connected to the steam inlet formed in another portion of the distribution housing.

In addition, in the shoe care device according to an aspect of the present disclosure, the dehumidifying material may be heated by the heater and the regeneration passage may be formed to allow the air generated according thereto to move therethrough.

In addition, the shoe care device according to an aspect of the present disclosure may be configured to further include a dehumidifying-material housing accommodating the dehumidifying material.

In addition, in the shoe care device according to an aspect of the present disclosure, a pair of dehumidifying materials may be disposed and the connecting passage and the regeneration passage may be formed in each dehumidifying material.

In addition, in the shoe care device according to an aspect of the present disclosure, the angle of the nozzle duct protruding to the inside of the inner cabinet may be adjusted by the first rotation-angle adjusting unit.

In addition, in the shoe care device according to an aspect of the present disclosure, the angle of the nozzle coupled to the nozzle duct may be adjusted by the second rotation-angle adjusting unit.

In addition, the shoe care device according to an aspect of the present disclosure may hold the nozzle duct in a holder installed in the upper portion of the inner cabinet.

In addition, in the shoe care device according to an aspect of the present disclosure, the holder may be configured to include a holding base and a holding wall.

Additional scope of applicability of the present disclosure will become apparent from the following detailed description. However, it should be understood that the detailed description and specific implementations such as implementations of the present disclosure are given by way of example only because various changes and modifications within the spirit and scope of the present disclosure can be clearly understood by those skilled in the art.

### BRIEF DESCRIPTION OF THE DRAWINGS

FIG. 1 is a perspective view illustrating a shoe care device according to an embodiment of the present disclosure.
FIG. 2A is a perspective view illustrating the shoe care device in FIG. 1 from which a door is removed to show the inside of the shoe care device in FIG. 1.
FIG. 2B is a front view illustrating the shoe care device in FIG. 2A.
FIG. 3A is a cross-sectional view of the shoe care device taken along line A-A' in FIG. 2B.
FIG. 3B is a cross-sectional view of the shoe care device taken along line B-B' in FIG. 2B.
FIG. 4 is a diagram illustrating the shoe care device in FIG. 2A from which an outer cabinet, a rack, and shoes are removed.
FIG. 5 is a diagram illustrating a portion of the inner cabinet in FIG. 2B.
FIG. 6 is a view illustrating the shoe care device shown in FIG. 4 viewed from the opposite side.
FIG. 7 is a diagram illustrating an air movement cycle in a shoe care device according to an embodiment of the present disclosure.
FIG. 8 is a diagram illustrating a machine room of a shoe care device viewed from above according to an embodiment of the present disclosure.
FIG. 9 is a cross-sectional view of the shoe care device taken along line C-C' in FIG. 2B.
FIG. 10 is a diagram illustrating the interior of the machine room of the shoe care device shown in FIG. 2A viewed from the bottom.
FIG. 11 is a bottom perspective view illustrating some elements provided inside a machine room of a shoe care device.
FIG. 12 is a perspective view illustrating some elements of the shoe care device shown in FIG. 11 when viewed in another direction.
FIG. 13 is a perspective view illustrating the shoe care device shown in FIG. 12 in which some elements are separated from each other.
FIG. 14 is a cross-sectional view illustrating some configurations of the shoe care device taken along line E-E' in FIG. 8.
FIG. 15A is a perspective view illustrating a suction duct according to an embodiment of the present disclosure.
FIG. 15B is a cross-sectional view illustrating the suction duct taken along line F-F' in FIG. 8.
FIG. 16 is a diagram illustrating some configurations of the machine room taken along line D-D' in FIG. 2B.
FIG. 17 is a perspective view illustrating the dehumidifying-material block shown in FIG. 13 viewed in another direction.
FIG. 18A is a transverse cross-sectional view illustrating a dehumidifying-material block according to an embodiment of the present disclosure.
FIG. 18B is a longitudinal cross-sectional view illustrating a dehumidifying-material block according to an embodiment of the present disclosure.
FIG. 18C is a diagram illustrating a dehumidifying-material block viewed from the front in the first direction according to an embodiment of the present disclosure.
FIG. 18D is a diagram illustrating a dehumidifying-material block viewed from the back in the first direction according to an embodiment of the present disclosure.
FIG. 19A is a perspective view illustrating a dehumidifying-material block according to an embodiment of the present disclosure.
FIG. 19B is a transverse cross-sectional view illustrating a dehumidifying-material block and a heater according to an embodiment of the present disclosure.
FIGS. 20A, 20B, and 20C are transverse cross-sectional views illustrating a dehumidifying-material block and a heater, respectively, according to an embodiment of the present disclosure.
FIG. 21 is a cross-sectional view illustrating some elements provided in a machine room of a shoe care device.
FIG. 22 is a perspective view illustrating a condensate water separator according to an embodiment of the present disclosure.
FIG. 23 is a diagram illustrating some configurations of a shoe care device according to an embodiment of the present disclosure.
FIG. 24 is a diagram illustrating a shoe care device from which an outer back plate of an outer cabinet is removed.
FIG. 25A is a perspective view separately illustrating the condenser shown in FIG. 24.
FIG. 25B is a diagram illustrating the inside of the condenser in FIG. 25A.
FIG. 25C is a front view illustrating the condenser in FIG. 25B.
FIG. 26 is a diagram illustrating a sump according to an embodiment of the present disclosure.
FIG. 27 is a diagram illustrating some configurations of a shoe care device according to an embodiment of the present disclosure, which shows the connection and positional relationships of a condensate water separator, a condenser, and a sump.
FIG. 28 is a diagram illustrating some configurations of a shoe care device according to an embodiment different from that in FIG. 27.

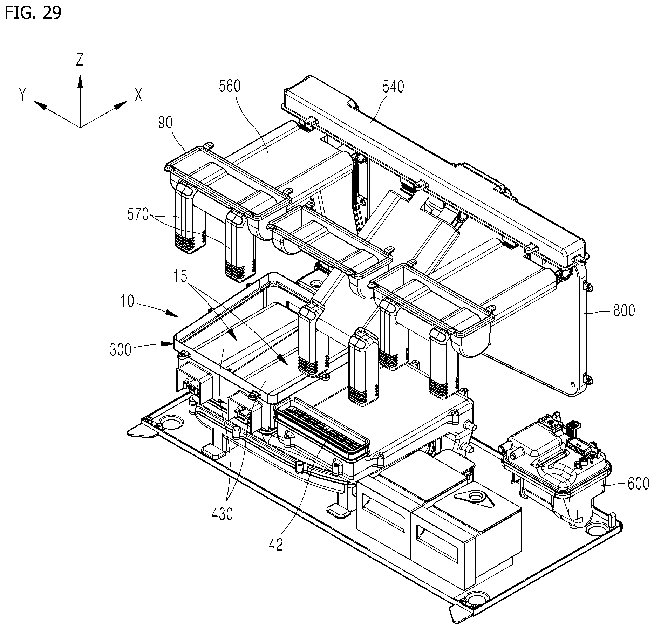

FIG. 29 is a perspective view illustrating the internal structure of a shoe care device according to an embodiment of the present disclosure.

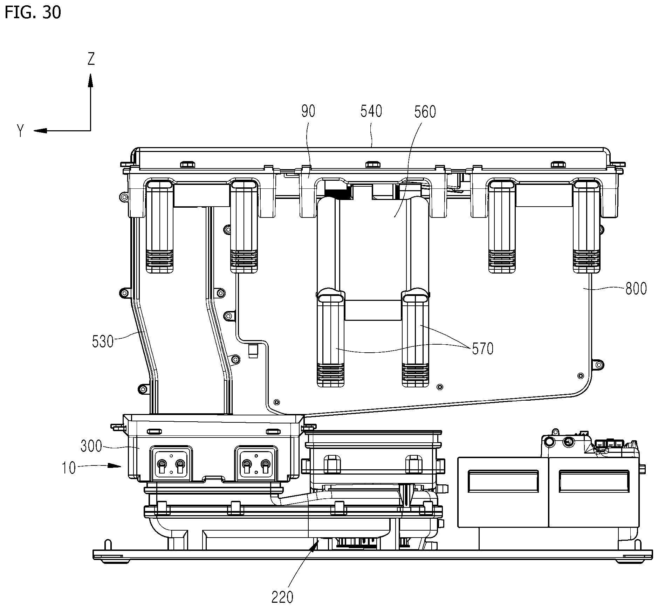

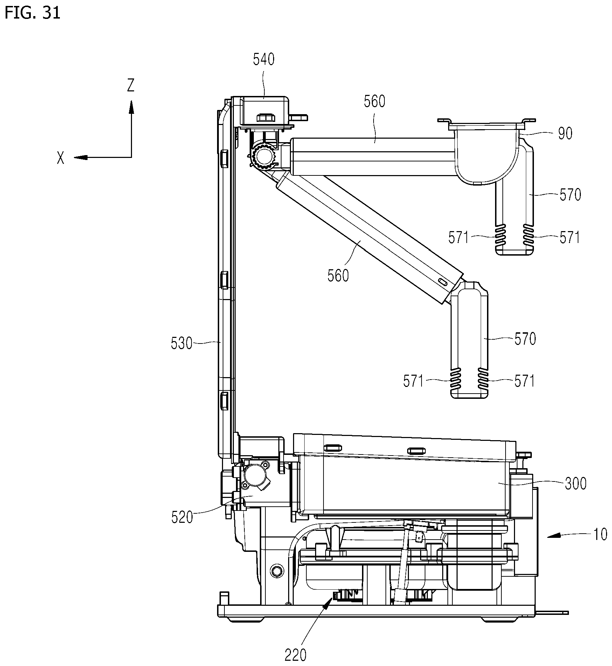

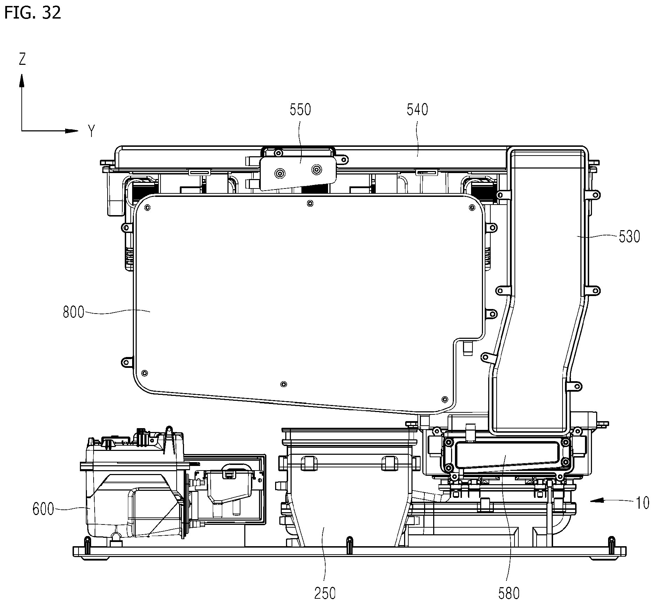

FIGS. 30 to 32 are views illustrating the front, side, and back of the shoe care device shown in FIG. 29.
FIG. 33 is a diagram illustrating an example of using a shoe care device according to an embodiment of the present disclosure.
FIG. 34 is a diagram illustrating another example of using a shoe care device according to an embodiment of the present disclosure.

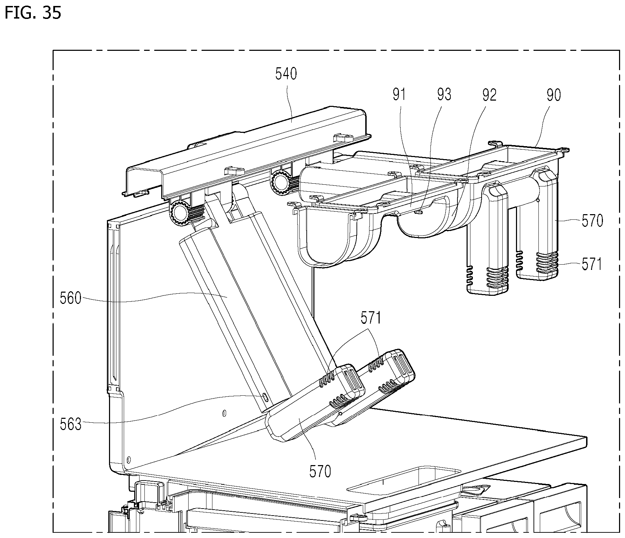

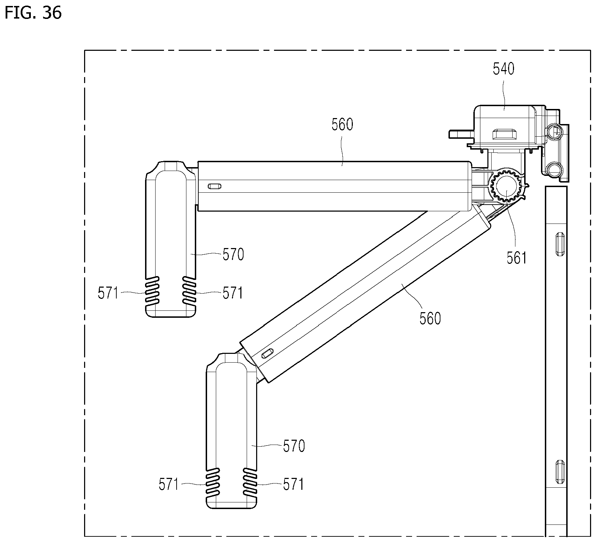

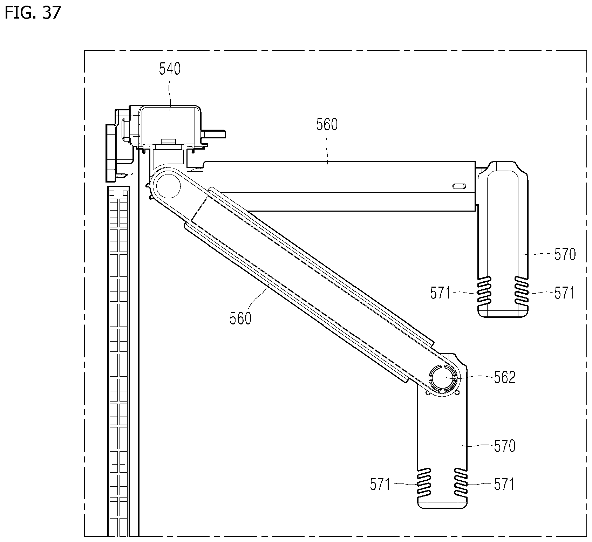

FIGS. 35 to 37 are diagrams illustrating a nozzle duct and a nozzle in a shoe care device in more detail according to an embodiment of the present disclosure.

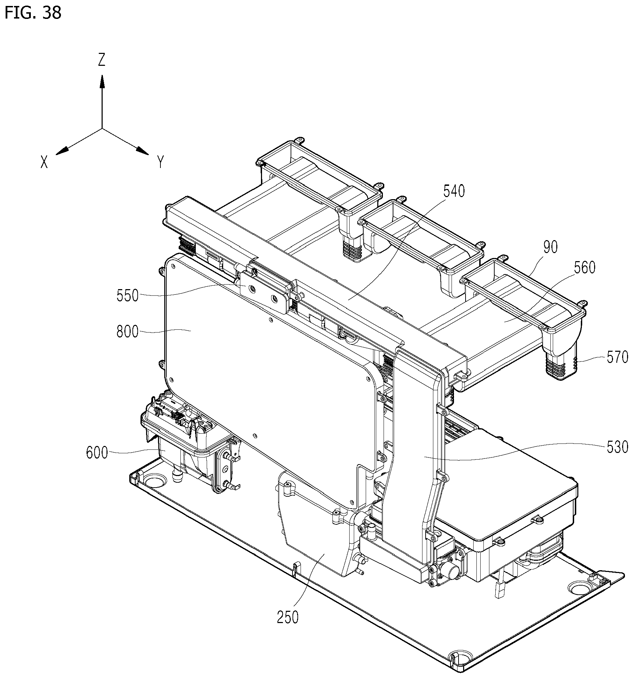

FIG. 38 is a perspective view of the shoe care device shown in FIG. 29 viewed in another direction.

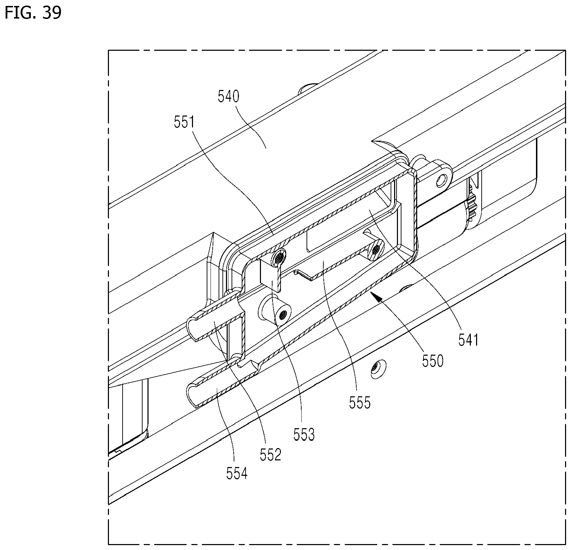

FIG. 39 is a cross-sectional view illustrating the inside of a steam separator in a shoe care device according to an embodiment of the present disclosure.

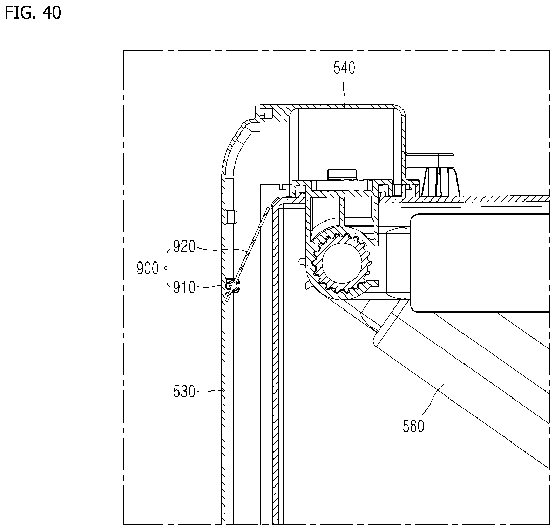

FIG. 40 is a cross-sectional view illustrating the inside of a dry air duct in a shoe care device according to an embodiment of the present disclosure.

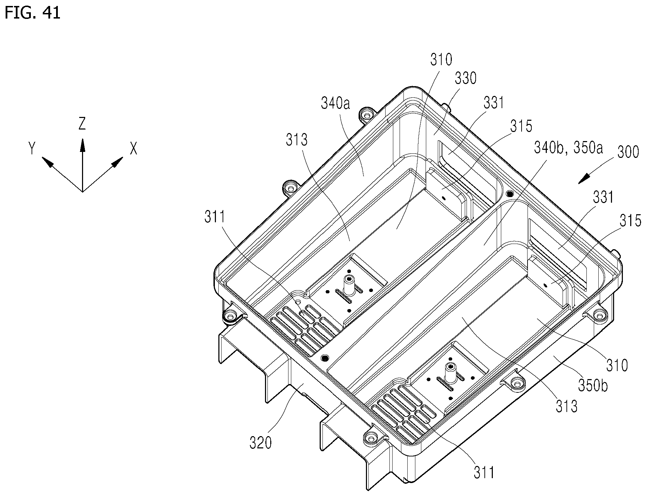

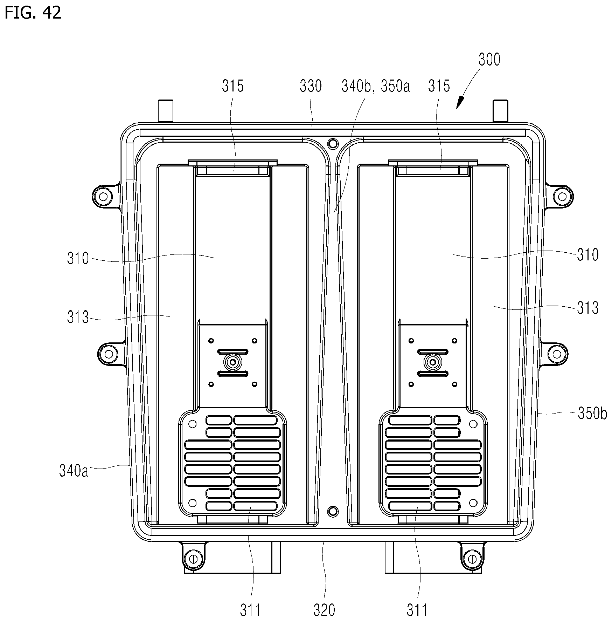

FIGS. 41 and 42 are diagrams illustrating a dehumidifying-material housing in a shoe care device in more detail according to an embodiment of the present disclosure.

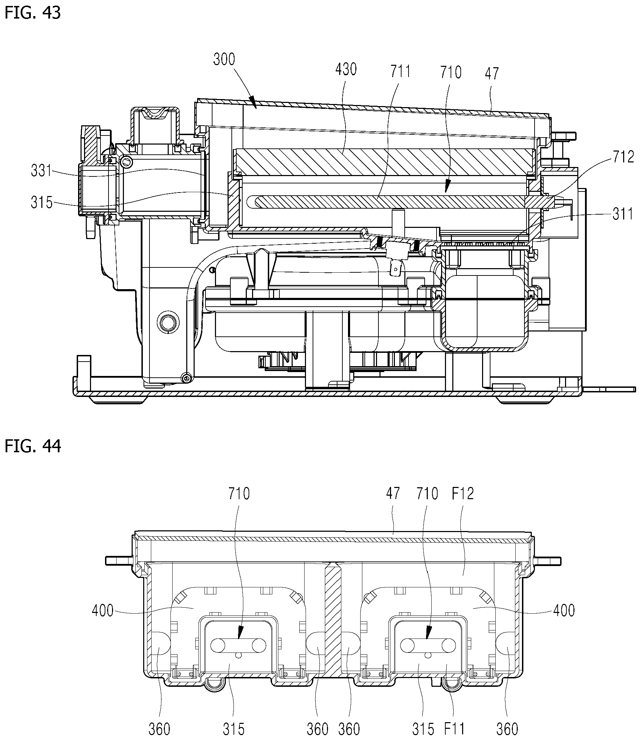

FIGS. 43 and 44 are cross-sectional views illustrating a state in which a dehumidifying material is accommodated in a dehumidifying-material housing of a shoe care device according to an embodiment of the present disclosure.

### DETAILED DESCRIPTION

Hereinafter, the embodiments disclosed in the present specification will be described in detail with reference to the accompanying drawings, and the same or similar elements are given the same and similar reference numerals, so duplicate descriptions thereof will be omitted. The terms "module" and "unit" used for the elements in the following description are given or interchangeably used in consideration of only the ease of writing the specification, and do not have distinct meanings or roles by themselves. In addition, in relation to describing the embodiments disclosed in the present specification, when the detailed description of the relevant known technology is determined to unnecessarily obscure the gist of the present disclosure, the detailed description may be omitted. Further, the accompanying drawings are provided only for easy understanding of the embodiments disclosed in the present specification, and the technical spirit disclosed herein is not limited to the accompanying drawings, and it should be understood that all changes, equivalents, or substitutes thereof are included in the spirit and scope of the present disclosure.

Terms including an ordinal number such as "first", "second", or the like may be used to describe various elements, but the elements are not limited to the terms. The above terms are used only for the purpose of distinguishing one element from another element.

The expression that one element is "connected" or "coupled" to the other element should be understood that another element may be provided therebetween, as well as that one element may be directly connected or coupled to the other element. On the other hand, the expression that one element is "directly connected" or "directly coupled" to the other element should be understood that no other element is present therebetween.

The singular forms are intended to include the plural forms as well, unless the context clearly indicates otherwise.

In the present application, the term "comprise", "have", or the like is intended to indicate that features, numbers, steps, operations, elements, or parts, which are described in the specification, or combinations thereof exist, and is not intended to preclude the possibility of existence or addition of one or more other features, numbers, steps, operations, elements, parts, or combinations thereof.

A first direction X, a second direction Y, and a third direction Z described in the embodiment of the present disclosure may be directions perpendicular to each other.

The first direction X and the second direction Y may be directions parallel to the horizontal direction, respectively, and the third direction Z may be a direction parallel to the vertical direction. When the first direction X is parallel to the left-right direction, the second direction Y may be parallel to the front-back direction. When the first direction X is parallel to the front-back direction, the second direction Y may be parallel to the left-right direction.

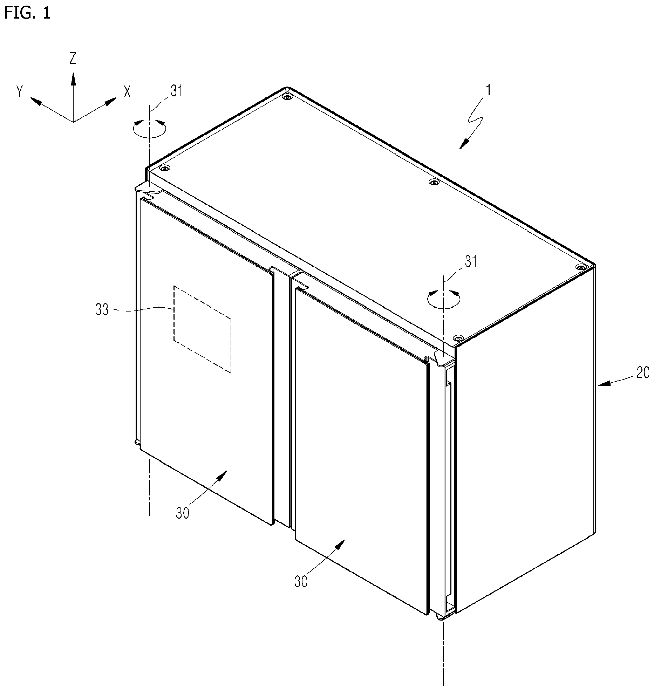

FIG. 1 is a perspective view illustrating a shoe care device 1 according to an embodiment of the present disclosure.

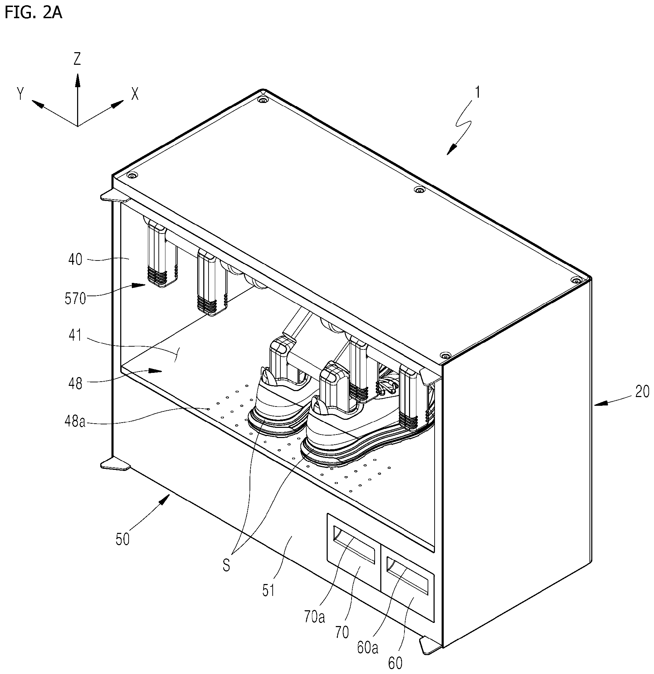

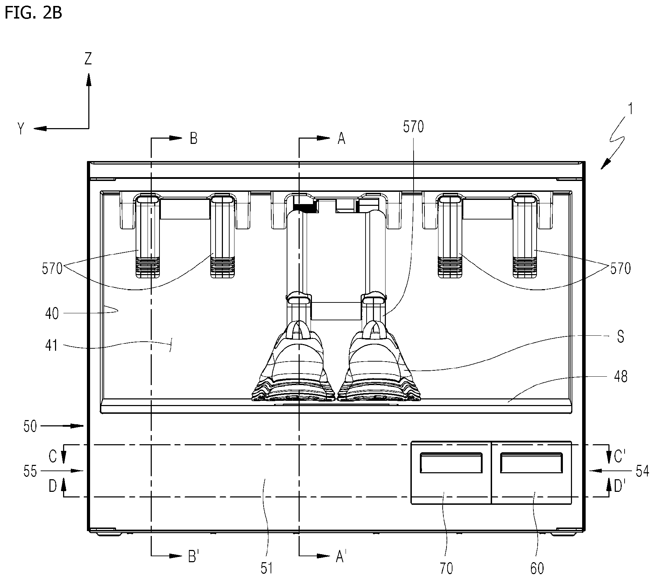

FIG. 2A is a perspective view illustrating the shoe care device 1 in FIG. 1 from which a door 30 is removed to show the inside of the shoe care device 1 in FIG. 1. FIG. 2B is a front view illustrating the shoe care device 1 in FIG. 2A.

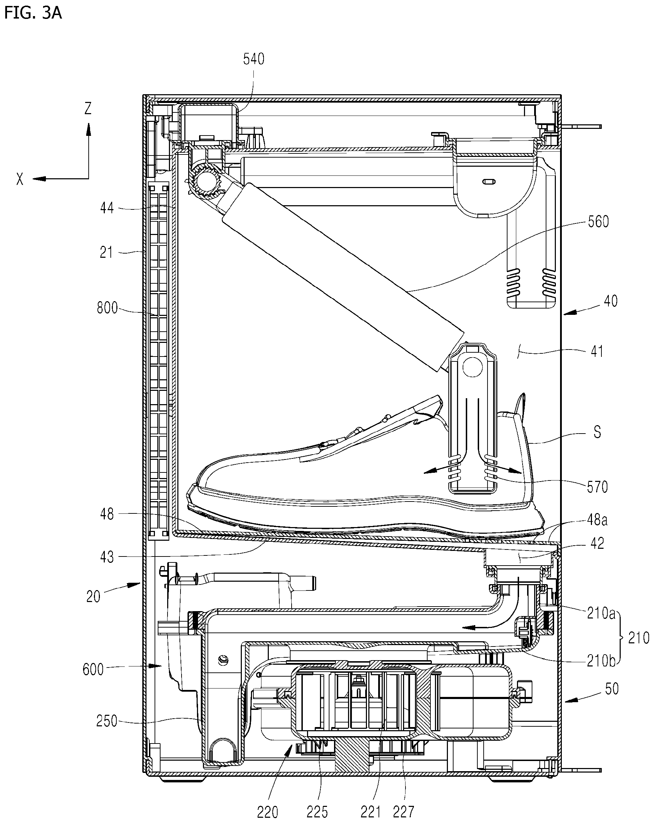

FIG. 3A is a cross-sectional view of the shoe care device 1 taken along line A-A' in FIG. 2B.

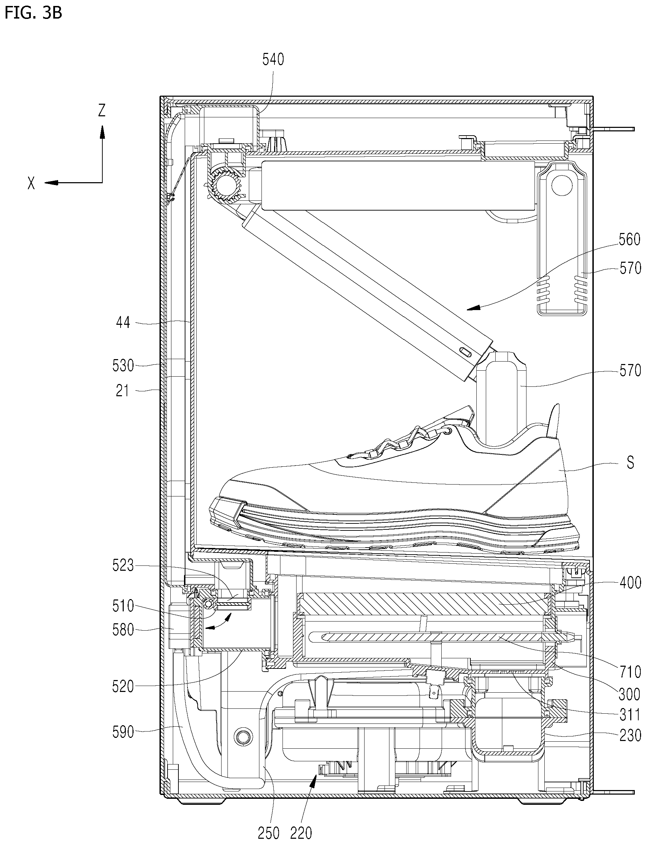

FIG. 3B is a cross-sectional view of the shoe care device 1 taken along line B-B' in FIG. 2B.

The shoe care device 1 according to an embodiment of the present disclosure may be configured to include an outer cabinet 20, a door 30, an inner cabinet 40, a machine room 50, and a controller 80.

The shoe care device 1 may be configured to include a dehumidifying housing 300, a dehumidifying-material block 400, and a heater 710.

The dehumidifying-material block 400 is configured to include a dehumidifying material 430. The entirety or a portion of the dehumidifying-material block 400 may be configured as a dehumidifying material.

The shoe care device 1 may include an inlet 42 and a nozzle 570.

The shoe care device 1 may be configured to include a connecting passage F10.

The shoe care device 1 may be configured to include a suction duct 210, a blower 220, and a blowing duct 230.

The shoe care device 1 may be configured to include a regeneration passage F20.

The shoe care device 1 may be configured to include a damper 510, a damper housing 520, a condensate water separator 580, a condenser 800, a sump 250, and a discharge pipe 590.

The shoe care device 1 may be configured to include a steam generator 600, a water supply tank 60, and a drain tank 70.

The outer cabinet 20 and the door 30 may constitute the overall appearance of the shoe care device 1.

The shoe care device 1 may have a hexahedral shape. That is, in the state where the outer cabinet 20 and the door 30 are coupled to each other and where the door 30 is closed, the outer shape of the shoe care device 1 may be configured in the form of a hexahedron.

The door 30 may be configured to open and close the inner space of the shoe care device 1. The door 30 may constitute any one face of the shoe care device 1. The door 30 may constitute the left face or right face of the shoe care device 1, or may constitute the front face of the shoe care device 1.

In an embodiment, the shoe care device 1 may be configured to include one door 30. In another embodiment, as shown in FIG. 1, the shoe care device 1 may be configured to include two doors 30.

A first direction X described in the embodiment of the present disclosure may be parallel to the horizontal direction, or may be substantially parallel to the horizontal direction.

In an embodiment, the first direction X may be a direction from the front to the back of the shoe care device 1. In another embodiment, the first direction X may be a direction from the left to the right of the shoe care device 1, or may be a direction from the right to the left of the shoe care device 1.

Hereinafter, the face where the door 30 is formed in the shoe care device 1 will be described as the front face of the shoe care device 1, unless otherwise specifically stated.

In addition, hereinafter, unless otherwise specifically limited, a description will be made on the assumption that a first direction X is parallel to the forward and backward directions, that a second direction Y is parallel to the left and right directions, and that a third direction Z is parallel to the upward and downward directions.

The inner cabinet 40 and the machine room 50 may be provided inside the outer cabinet 20. The outer cabinet 20 may configure outer wall surfaces of the inner cabinet 40 and the machine room 50, respectively. In the case where a separate cabinet for the machine room 50 is not provided in the shoe care device 1, the outer cabinet 20 may configure walls that separate the machine room 50 from the outside thereof.

An inner space is provided inside the shoe care device 1 as an accommodation space 41 in which shoes S are accommodated. The inner cabinet 40 may be configured in the form of a box, and the accommodation space 41 inside the inner cabinet 40 configures the inner space of the shoe care device 1. That is, the inner cabinet 40 may be configured to accommodate shoes S therein.

The inner cabinet 40 may be configured in the form of a horizontally long box, and a plurality of shoes S may be arranged in the left and right directions inside the inner cabinet 40.

The inner cabinet 40 may be configured in the form of a box having an opening on one side thereof. The opening of the inner cabinet 40 may be closed or opened by the door 30. The inner cabinet 40 may be configured to be open on the front face of the shoe care device 1.

The inner cabinet 40 has a predetermined size along the first direction X, second direction Y, and third direction Z.

A rack 48 may be provided in the inner space 41 of the inner cabinet 40. The rack 48 may be configured such that the shoes S are seated on the upper surface thereof.

The rack 48 may be configured in the form of a plate having a predetermined area, or may be configured in the form of a grill in which a plurality of bars is spaced apart from each other.

A single or a plurality of racks 48 may be provided.

The rack 48 may be configured in a plate having a flat upper surface and placed on the bottom of the inner cabinet 40. The rack 48 may be placed so as to be stacked on the upper surface of a cabinet bottom plate 43 of the inner cabinet 40.

The inlet 42 forms an entrance through which air is sucked from the accommodation space 41 of the inner cabinet 40, and this inlet 42 may be located below the rack 48. The rack 48 may be configured so as not to interfere with the air sucked from the accommodation space 41 into the inlet 42. To this end, in the case where the rack 48 is configured as a plate, a plurality of holes 48a vertically penetrating the rack 48 may be provided to allow air to move.

The outer cabinet 20, along with the door 30, may constitute the outer faces of the shoe care device 1. The outer cabinet 20 may be located outside the inner cabinet 40 and may also be located outside the machine room 50.

In the case where the door 30 constitute the front face of the shoe care device 1, the outer cabinet 20 may constitute the top face, left face, right face, back face, and bottom face of the shoe care device 1.

The entirety or part of the outer cabinet 20 may be spaced apart from the inner cabinet 40, and accordingly, a predetermined gap may be formed between the inner cabinet 40 and the outer cabinet 20.

The outer cabinet 20 may be configured to include an outer back plate 21. The outer back plate 21 may constitute the front face of the shoe care device 1 in the first direction X. The outer back plate 21 may constitute the entire outer back face of the shoe care device 1.

The outer back plate 21 may be spaced apart from the surface of the inner cabinet 40 so as to be parallel thereto. The outer back plate 21 may be spaced apart from the inner back plate 44 of the inner cabinet 40 so as to be parallel thereto.

A condenser 800 may be accommodated between the outer back plate 21 and the inner back plate 44.

The inner cabinet 40 and the machine room 50 may have separate spaces from each other inside the shoe care device 1. The inner cabinet 40 may form a space for accommodating objects (shoes S) subject to management, and the machine room 50 may form a space for accommodating elements for operation of the shoe care device 1.

The machine room 50 may be configured to accommodate the connecting passage F10, a blower 220, a dehumidifying-material housing 300, a dehumidifying-material block 400 (and a dehumidifying material 430), a heater 710, a sump 250, a regeneration passage F20, a condensate water separator 580, and a steam generator 600. The machine room 50 may be configured to accommodate a water supply tank 60 and a drain tank 70.

Elements that are coupled to or accommodated in the machine room 50 may be fixedly coupled to the machine room 50.

The machine room 50 may be configured to include a first wall 51.

The first wall 51 may configure any one wall surface of the machine room 50. The first wall 51 may be erected in the vertical direction or erected in the substantially vertical direction. In an embodiment, the first wall 51 may form a wall surface perpendicular to or inclined to the first direction X. In another embodiment, the first wall 51 may form a wall surface perpendicular to or inclined to the second direction Y.

The first wall 51 may form a front wall surface of the machine room 50, form a left wall of the machine room 50, or form a right wall of the machine room 50.

The machine room 50 may be configured to include a second wall 54 and a third wall 55. The second wall 54 and the third wall 55 form opposite wall surfaces in the machine room 50. The second wall 54 and the third wall 55 may be erected in the vertical direction or erected in the substantially vertical direction.

In the case where the first wall 51 forms the front wall of the machine room 50, the second wall 54 may form the left wall of the machine room 50, and the third wall 55 may form the right wall of the machine room 50.

The water supply tank 60 and the drain tank 70 may be configured in the form of a container for accommodating water.

The water supply tank 60 may be configured to store water, supplied to the inside of the shoe care device 1, therein. In particular, the water supply tank 60 may be configured to store water, supplied to the steam generator 600, therein.

In order to supply water from the water supply tank 60 into the shoe care device 1, a water pump (a first water pump 61) may be connected to the water supply tank 60.

The drain tank 70 may be configured to store water discharged from the shoe care device 1 therein. The drain tank 70 may store the water condensed inside the shoe care device 1. The drain tank 70 may be configured to store the water drained from the sump 250.

A water pump (a second water pump 71) may be connected to the drain tank 70 to discharge water to the drain tank 70.

The water supply tank 60 and the drain tank 70 may be coupled to the machine room 50 so as to be exposed to the outside through any one wall surface of the machine room 50.

The water supply tank 60 and the drain tank 70 may be located at the front of the machine room 50.

The water supply tank 60 and the drain tank 70 may configure one wall surface of the machine room 50 together with the first wall 51. In the case where the first wall 51 configures the front surface of the machine room 50, the water supply tank 60 and the drain tank 70 may be coupled to the machine room 50 so as to be exposed through the front side of the machine room 50 and so as to be exposed to the outside of the first wall 51.

Since the water supply tank 60 and the drain tank 70 are exposed to the outside of the first wall 51, the user may put water into the water supply tank 60 or discharge water from the drain tank 70.

The water supply tank 60 and the drain tank 70 may be configured to be detachable from the machine room 50. The water supply tank 60 and the drain tank 70 may be attached to and detached from the first wall 51. In order to facilitate attachment and detachment of the water supply tank 60 and the drain tank 70, a handle 60a of the water supply tank may be formed on the outer surface of the water supply tank 60, and a handle 70a of the drain tank 70 may be formed on the outer surface of the drain tank 70.

The water supply tank 60 and the drain tank 70 may be configured to be separated from the machine room 50 in the outer direction of the first wall 51, respectively.

The controller 80 may be configured to control the operations of the respective elements in connection with respective elements constituting the shoe care device 1.

The door 30 may be configured to open and close both the inner cabinet 40 and the machine room 50.

The door 30 may be configured to be rotatable in both directions around the rotation axis 31 in the vertical direction in the shoe care device 1. In the case where two doors 30 are provided in the shoe care device 1, the respective doors 30 may rotate around their own rotation axes 31 independently. The door 30 may be coupled to the outer cabinet 20 by a hinge. The door 30 may be coupled to the inner cabinet 40 and/or the machine room 50 by a hinge.

The door 30 may be coupled to the inner cabinet 40 and the machine room 50 on the same side as the first wall 51. That is, in the case where the door 30 configures the front face of the shoe care device 1, the first wall 51 may configure the front surface of the machine room 50, and the door 30 may be located right outside the first wall 51.

The door 30 may be configured to expose or shield the inner cabinet 40, the water supply tank 60, and the drain tank 70. The door 30 may be configured to open and close the front side of the inner cabinet 40, the water supply tank 60, and the drain tank 70.

In the shoe care device 1, the door 30, the water supply tank 60, and the drain tank 70 may be provided on the same side, and when the door 30 is opened, the water supply tank 60 and the drain tank 70 may be exposed and separated from the shoe care device 1.

With the configuration above, even if the left and right faces and the back face of the shoe care device 1 are blocked by other items or structures, the door 30 may be opened from the front side of the shoe care device 1, and furthermore, the water supply tank 60 and the drain tank 70 may be separated from or attached to the shoe care device 1.

A control panel 33 for controlling the shoe care device 1 is provided outside the door 30. A control unit (a controller 80) for controlling respective elements of the shoe care device 1 in connection with the control panel 33 may be provided in the inner space of the door 30. The controller 80 may be provided inside the machine room 50.

As shown in FIGS. 1 and 2A, the door 30 according to an embodiment may be configured to open and close both the inner cabinet 40 and the machine room 50.

In another embodiment, the door 30 may be configured to open and close only the inner cabinet 40. In this case, the machine room 50 may be configured not to be shielded by the door 30. Furthermore, in this case, the shoe care device 1 according to the embodiment of the present disclosure may further include a dedicated door for the machine room 50 provided to close the machine room 50 separately from the door 30.

A steam generator 600 is provided in the shoe care device 1 as a device for generating moisture inside the inner cabinet 40. The steam generator 600 may be provided inside the machine room 50. The steam generator 600 may be configured to generate steam and selectively supply moisture and steam into the inner cabinet 40.

Humid air generated by the steam generator 600 ("air" described in the embodiment of the present disclosure may be "air containing moisture") may be supplied to the inner space of the shoe care device 1, and the moisture may circulate through the inner space, thereby providing moisture to the shoes S.

The shoe care device 1 according to an embodiment of the present disclosure may be a refresher device for refreshing shoes.

Here, "refreshing" may indicate a process of removing contaminants from shoes, deodorizing shoes, sanitizing shoes, preventing static electricity of shoes, or warming shoes by providing air, heated air, water, mist, steam, or the like to the shoes.

The steam generator 600 may supply steam to the accommodation space 41 of the inner cabinet 40 in which shoes S are accommodated to perform steam treatment on the shoes, and furthermore, it is intended to exert a refreshing effect by inflating the shoe material, as well as a sterilization effect by high-temperature steam.

The steam generator 600 may be provided with a separate heater 610 for heating water therein, and may generate steam by heating water and supply the steam to the accommodation space 41 of the inner cabinet 40.

As a water supply source for supplying water to the steam generator 600, an external faucet or the like may be used, or a container-type water supply tank provided on one side of the machine room 50 may be used. The steam generator 600 may generate steam by receiving water from the water supply tank 60.

In the shoe care device 1 according to the embodiment of the present disclosure, the dehumidifying-material block 400 may be used as a means for dehumidifying air.

The dehumidifying-material block 400 may be provided in the machine room 50.

The dehumidifying-material block 400 may be configured to have a predetermined volume. The dehumidifying-material block 400 may be configured to be porous by itself. A plurality of pores may be formed in the dehumidifying-material block 400 over the entire volume, and air may pass through the dehumidifying-material block 400 through the pores.

As will be described below, in the case where the dehumidifying-material block 400 is configured as a combination of a plurality of dehumidifying materials 430, the plurality of dehumidifying materials 430 may be fixed to each other by separate fixing means, or fixed to each other by adhesion.

The dehumidifying-material block 400 may be configured to include a dehumidifying material.

The dehumidifying material 430 according to an embodiment of the present disclosure may be configured to include a material capable of lowering humidity by absorbing moisture from the air. The dehumidifying material 430 may be formed of various materials or combinations of materials capable of absorbing or adsorbing moisture from the air, and may have various shapes and structures.

The dehumidifying material 430 according to the embodiment of the present disclosure may be referred to as "desiccant", "absorbent", or "adsorbent".

The dehumidifying material 430 according to the embodiment of the present disclosure may be made of a microporous material. The dehumidifying material 430 according to the embodiment of the present disclosure may be configured to include silica gel, activated carbon, activated alumina (AL2O3), diatomaceous earth, and the like.

In particular, the dehumidifying material 430 according to the embodiment of the present disclosure may be made of zeolite or may be configured to include zeolite.

Zeolite is a natural and synthetic silicate mineral in which tunnels (or open channels) having a size of about 3 to 10 angstroms (A) are regularly arranged, and may perform a dehumidification function by adsorbing moisture from the air.

In the case of heating zeolite, the moisture adsorbed onto the zeolite may be separated into a large amount of vapor. Zeolite may perform a dehumidification function of removing moisture from the air according to the above characteristic, and the moisture adsorbed onto the zeolite may be separated by heating the zeolite, thereby regenerating the zeolite into the state capable of the dehumidification function.

Hereinafter, a description will be made based on that the dehumidifying material 430 according to the embodiment of the present disclosure is made of zeolite.

Zeolite may be made in the form of a small grain (or stone) having a size (diameter) of several millimeters (mm) to several tens of millimeter (mm), and the dehumidifying material 430 described in the embodiment of the present disclosure may be configured in a form in which these grains (or stones) are combined. Each of the grains (or stones) may be aggregated or combined to form a single structure.

The heater 710 may be provided on one side of the dehumidifying material (zeolite) 430, and the heater 710 may be selectively heated so that dehumidification by the dehumidifying material 430 or regeneration of the dehumidifying material 430 may be performed.

The dehumidifying material (zeolite) 430 and the heater 710 may be configured as a set. A plurality of sets of dehumidifying material and heater may be provided. Two sets of dehumidifying material 430 and heater 710 may be provided in the shoe care device 1 according to an embodiment.

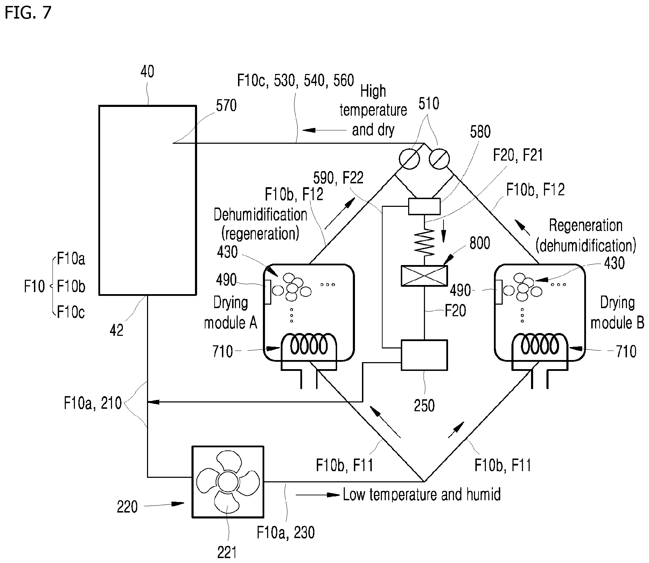

Such a set may be referred to as a "drying module" in the shoe care device 1 according to the embodiment of the present disclosure. In the shoe care device 1 according to the embodiment of the present disclosure, a plurality of drying modules may be provided or a pair of drying modules may be provided. In the case where a pair of drying modules is provided in the shoe care device 1, one drying module may be "drying module A", and the other drying module may be "drying module B" (see FIG. 7).

In the shoe care device 1 according to the embodiment of the present disclosure, the drying module A and the drying module B may be configured to operate in different modes from each other. When the drying module A is operated in a dehumidifying mode (in which the dehumidifying material 430 adsorbs moisture from the air), the drying module B may be operated in a regeneration mode (in which the moisture adsorbed onto the dehumidifying material is separated by heating the dehumidifying material). On the other hand, when the drying module A is operated in a regeneration mode, the drying module B may be operated in a dehumidifying mode.

Both the drying module A and the drying module B may also be operated in the dehumidifying mode or in the regeneration mode.

The shoe care device 1 according to an embodiment of the present disclosure may have a structure in which the dehumidifying-material block 400 is not separable from the machine room 50.

The shoe care device 1 according to another embodiment of the present disclosure may have a structure in which the dehumidifying-material block 400 is separable from the machine room 50. This structure of the shoe care device 1 may provide an advantage in overall maintenance and management of the dehumidifying-material block 400 and the shoe care device 1.

Meanwhile, the dehumidifying-material block 400 may be repeatedly used by regeneration, but may need to be replaced due to repeated use.

In consideration of this, the shoe care device 1 according to a specific embodiment of the present disclosure may be configured to enable separation and replacement of the dehumidifying-material block 400.

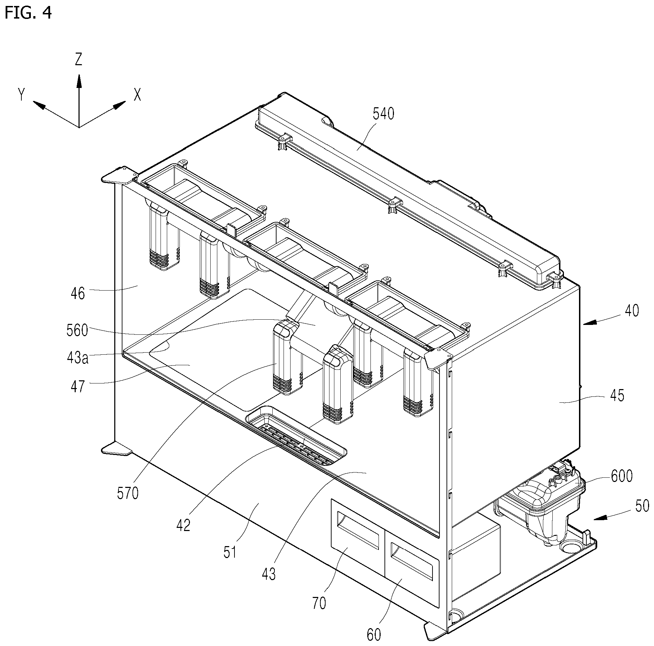

FIG. 4 is a diagram illustrating the shoe care device 1 in FIG. 2A from which the outer cabinet 20, the rack 48, and the shoes S are removed.

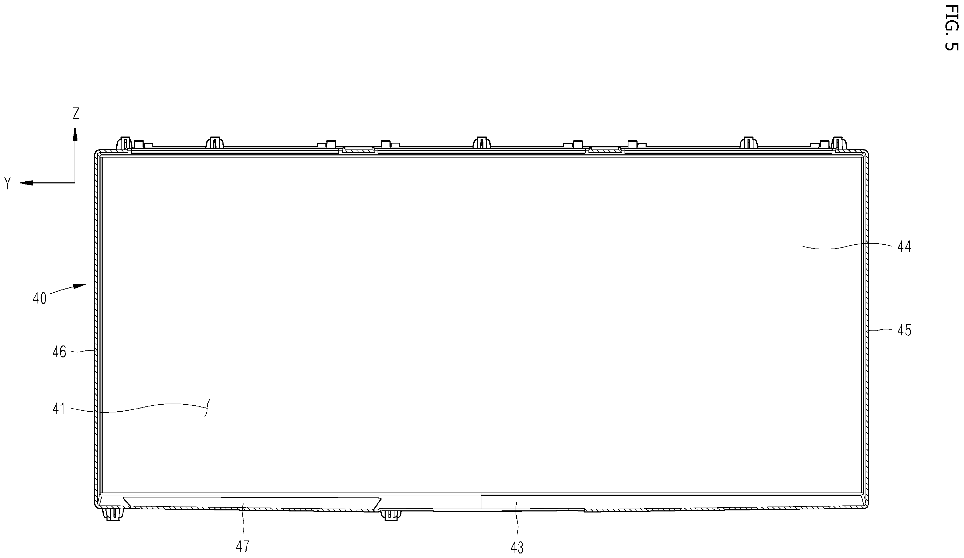

FIG. 5 is a diagram illustrating a portion of the inner cabinet 40 in FIG. 2B.

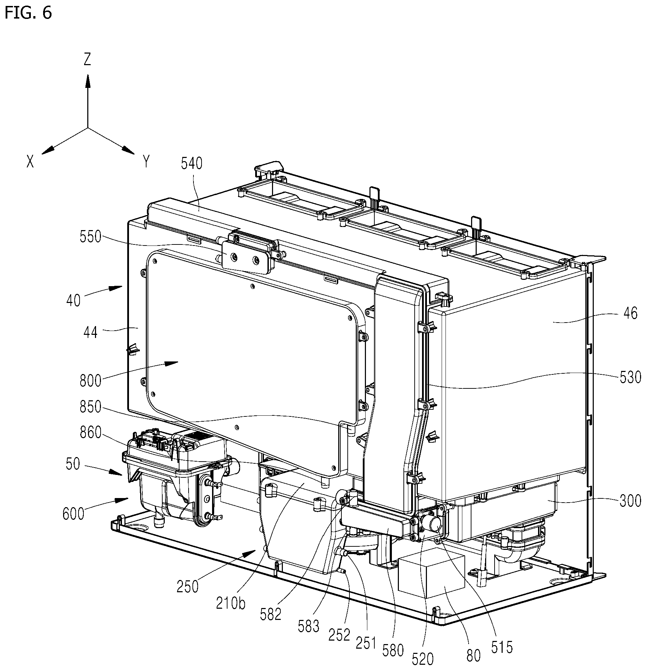

FIG. 6 is a view illustrating the shoe care device 1 shown in FIG. 4 viewed from the opposite side.

The inner cabinet 40 may be configured to include a cabinet bottom plate 43 forming its bottom surface.

Additionally, the inner cabinet 40 may be configured to include an inner back plate 44, an inner left plate 45, and an inner right plate 46.

The cabinet bottom plate 43 may form the boundary surface between the inner cabinet 40 and the machine room 50. The cabinet bottom plate 43 may have a rectangular shape.

The cabinet bottom plate 43 may be configured to be parallel to the horizontal direction.

Alternatively, the cabinet bottom plate 43 may be configured to be inclined to either side. In this case, water (e.g., condensate water) on the upper surface of the cabinet bottom plate 43 may flow in the inclined direction.

In an embodiment, the cabinet bottom plate 43 may be configured such that the front thereof is inclined downwards.

In another embodiment, the cabinet bottom plate 43 may be configured to be inclined downwards to the left, or may be configured to be inclined downwards to the right.

The cabinet bottom plate 43 may configured to be inclined upwards along the first direction X, or the cabinet bottom plate 43 may be configured to be inclined upwards along the second direction Y.

The shoe care device 1 may be configured to include a dehumidifying-material cover 47.

The dehumidifying-material cover 47 configures a portion of the cabinet bottom plate 43, which is the bottom of the inner cabinet 40. In addition, the dehumidifying-material cover 47 may be detachably coupled to the cabinet bottom plate 43 of the inner cabinet 40, or may be connected to the cabinet bottom plate 43 by a hinge.

The cabinet bottom plate 43 may have an opening 43a formed to have a shape and a size corresponding to the dehumidifying-material cover 47. The dehumidifying-material cover 47 may be configured to open and close the opening 43a. The dehumidifying-material cover 47 may be tightly coupled to the opening 43a. The dehumidifying-material cover 47 may be at least partially separable from the cabinet bottom plate 43. In an embodiment, the dehumidifying-material cover 47 may be completely separated from the cabinet bottom plate 43, so that the opening 43a of the cabinet bottom plate 43 may be open, and in another embodiment, the dehumidifying-material cover 47 may rotate about a hinge axis, thereby opening the opening 43a of the cabinet bottom plate 43. The dehumidifying-material block 400 may be inserted into or drawn out of the machine room 50 through the opening 43a.

In addition, if the dehumidifying-material cover 47 is separated from the cabinet bottom plate 43, the dehumidifying-material housing 300 positioned under the cabinet bottom plate 43 may be exposed through the opening 43a of the cabinet bottom plate 43, so that the dehumidifying-material block 400 may be placed inside the dehumidifying-material housing 300 or the dehumidifying-material block 400 may be separated from the dehumidifying-material housing 300.

The dehumidifying-material cover 47 and the opening 43a may have any size and shape as long as insertion and taking out of the dehumidifying-material block 400 are possible.

The dehumidifying-material cover 47 may be formed of a plate in a rectangular shape.

The length of the dehumidifying-material cover 47 may be equal to or greater than the length of the dehumidifying-material block 400 in the first direction X, and the length of the dehumidifying-material cover 47 may be equal to or greater than the length of the dehumidifying-material block 400 in the second direction Y.

In the case where a pair of dehumidifying-material blocks 400 is provided, a pair of dehumidifying-material covers 47 may be provided to respectively close or open the dehumidifying-material blocks 400, or one dehumidifying-material cover 47 may be provided to simultaneously close or open the pair of dehumidifying-material blocks 400.

The dehumidifying-material cover 47 may be configured to close the dehumidifying-material block 400 to be spaced apart therefrom. A space between the dehumidifying-material cover 47 and the dehumidifying-material block 400 may form an outer passage F12.

The inlet 42 is configured to communicate with the accommodation space 41 of the inner cabinet 40.

The inlet 42, which is a hole through which the air is sucked from the inner cabinet 40, may configure the beginning of the connecting passage F10. The inlet 42 may be formed on the bottom (the cabinet bottom plate 43) of the inner cabinet 40 or may be formed adjacent to the bottom of the inner cabinet 40. The inlet 42 may be located below the bottom (the cabinet bottom plate 43) of the inner cabinet 40.

A net such as a grid type, a mesh type, or the like may be provided in the inlet 42.

The inlet 42 may be formed parallel to the second direction Y. That is, the inlet 42 may be configured in the form of a long slot on the cabinet bottom plate 43 along the second direction Y.

The inlet 42 may be formed at the edge of the cabinet bottom plate 43.

The inlet 42 may be formed along the second direction Y at the edge of the cabinet bottom plate 43.

The inlet 42 may be formed in the front portion or rear portion of the cabinet bottom plate 43 in the first direction X.

The inlet 42 may be located on the cabinet bottom plate 43 to be relatively close to the door 30. That is, the inlet 42 may be located relatively at the front of the cabinet bottom plate 43.

The cabinet bottom plate 43 may be configured to be inclined downwards to the inlet 42. That is, the portion where the inlet 42 is formed in the cabinet bottom plate 43 may be the lowest portion. Accordingly, if there is water on the cabinet bottom plate 43, this water may flow along the surface of the cabinet bottom plate 43 by gravity and flow into the inlet 42.

The inner back plate 44 may be configured to extend upwards from one end of the cabinet bottom plate 43. The inner back plate 44 may be the surface forming the front surface of the inner cabinet 40 in the first direction X.

The inner back plate 44 may be configured to extend upwards from the rear end of the cabinet bottom plate 43. The inner back plate 44 may constitute the entirety of the inner rear surface of the shoe care device 1.

The inner left plate 45 may extend upwards from the left end of the cabinet bottom plate 43, and the inner right plate 46 may extend upwards from the right end of the cabinet bottom plate 43.

FIG. 7 is a diagram illustrating an air movement cycle in a shoe care device 1 according to an embodiment of the present disclosure.

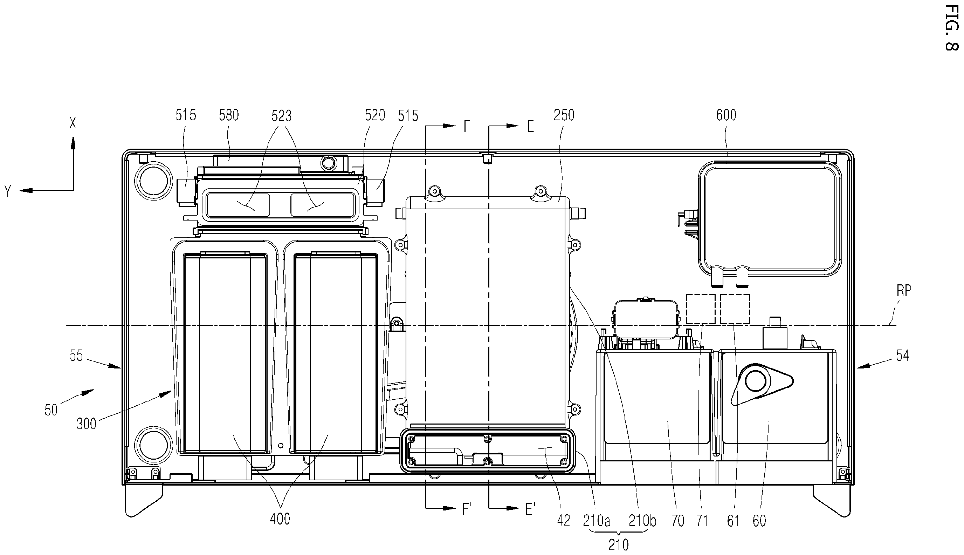

FIG. 8 is a diagram illustrating a machine room 50 of a shoe care device 1 a viewed from above according to an embodiment of the present disclosure.

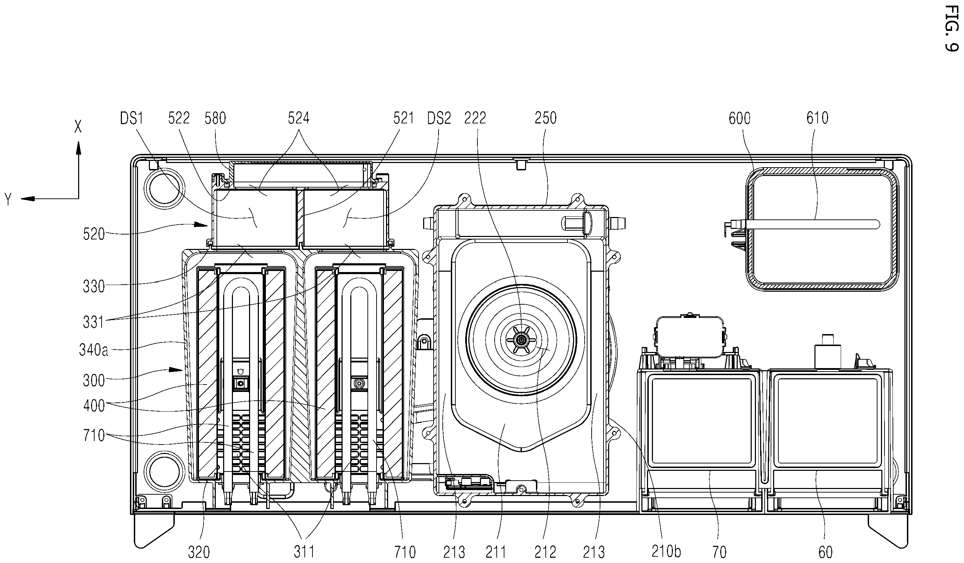

FIG. 9 is a cross-sectional view of the shoe care device 1 taken along line C-C' in FIG. 2B. In FIG. 9, the dehumidifying-material block 400 is shown in cross-section while the ceiling part 401 is removed therefrom.

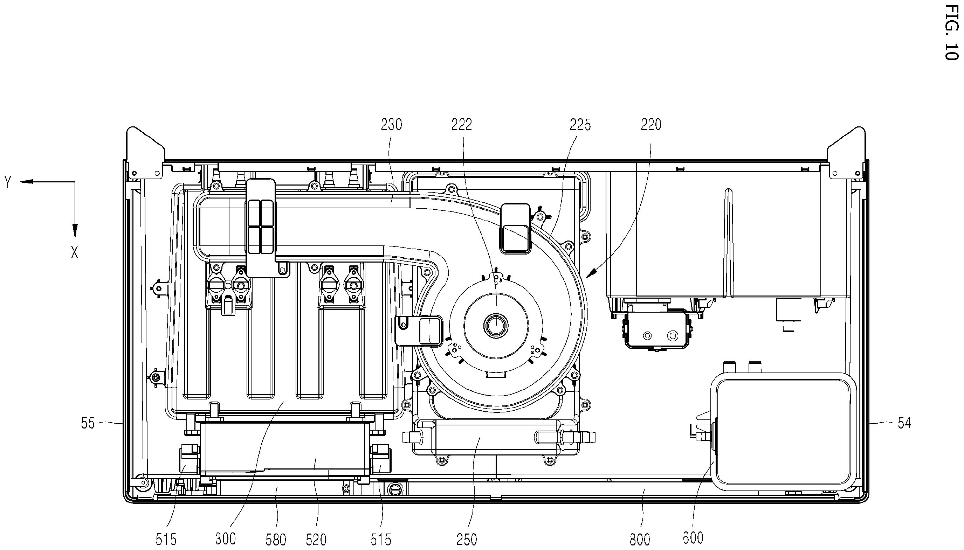

FIG. 10 is a diagram illustrating the interior of the machine room 50 of the shoe care device 1 shown in FIG. 2A viewed from the bottom.

The connecting passage F10 configures a movement passage for fluid.

The connecting passage F10 may form a passage through which the air moves inside the shoe care device 1.

The shoe care device 1 according to the embodiment of the present disclosure has an air circulation structure in which the air inside the inner cabinet 40 where shoes are placed is sucked into the machine room 50 and dehumidified using a dehumidifying material 430 and in which the dehumidified air is supplied back into the inner cabinet 40.

The connecting passage F10 may be used as a means for making such an air circulation structure in the shoe care device 1. The entirety or part of the connecting passage F10 may be configured in the form of a pipe, a tube, a duct, or a combination thereof.

The connecting passage F10 may configure an air moving passage connected from the inlet 42 to the nozzle 570. That is, the inlet 42 may configure the entrance to the connecting passage F10, and the nozzle 570 may configure the exit of the connecting passage F10.

The inlet 42 and the nozzle 570 may be provided in the inner cabinet 40. A portion of the connecting passage F10, excluding the inlet 42 and the nozzle 570, may be provided in the inner cabinet 40, and another portion thereof may be provided in the machine room 50.

The air inside the inner cabinet 40 may move to the connecting passage F10 through the inlet 42, and the air that has passed through the connecting passage F10 may move back to the inner cabinet 40 through the nozzle 570. As such air flow is repeated, air circulation may occur in the shoe care device 1.

A hole through which air is discharged into the inner cabinet 40 is formed in the nozzle 570, and the nozzle 570 may form the last part of the connecting passage F10.

In the shoe care device 1 according to the embodiment of the present disclosure, the nozzle 570 may be configured to be movable to various positions inside the inner cabinet 40, so that shoe care may be performed at various positions.

The dehumidifying-material block 400 is disposed on the connecting passage F10. The air moving through the connecting passage F10 may pass through the dehumidifying-material block 400, and the dehumidifying-material block 400 may absorb moisture from the air moving through the connecting passage F10, so that the dehumidified air may be supplied to the inner cabinet 40.

The blower 220 is provided inside the machine room 50 to generate air flow in the shoe care device 1.

In particular, the blower 220 makes the air flow through the connecting passage F10. That is, the blower 220 causes the air inside the inner cabinet 40 to be sucked into the inlet 42 and the air inside the connecting passage F10 to be discharged to the inner cabinet 40 through the nozzle 570.

Dry air may be supplied into the inner cabinet 40 by the blower 220.

The connecting passage F10 may be divided into a first section F10a, a second section F10b, and a third section FlOc. The first section F10a, the second section F10b, and the third section FlOc are sequentially connected to each other to form an air movement passage. Air in the connecting passage F10 may pass through the first section F10a, the second section F10b, and the third section FlOc in sequence.

The first section F10a may be an upstream section of the connecting passage F10, which is connected to the inlet 42.

The first section F10a may be connected to the inlet 42 and may be a section where the blower 220 is located.

The first section F10a may be a section through which humid air moves.

The second section F10b may be a section connected to the first section F10a and where the dehumidifying-material block 400 is located. The second section F10b may be a midstream section of the connecting passage F10.

The second section F10b may be a section where air is dehumidified by the dehumidifying-material block 400, or may be a section where the dehumidifying-material block 400 (the dehumidifying material 430) is regenerated.

The third section FlOc may be a downstream section of the connecting passage F10, which connects the second section F10b and the nozzle 570.

The third section FlOc may be a section through which dry air, from which moisture has been removed, moves.

The connecting passage F10 may be configured to include a suction duct 210 and a blowing duct 230. The suction duct 210 and the blowing duct 230 may be accommodated in the machine room 50.

The suction duct 210 forms part of the connecting passage F10. The suction duct 210 may be connected to the inlet 42 and may suck air from the inner cabinet 40.

The connecting passage F10 may be configured to include a dry air duct 530, a distribution housing 540, and a nozzle duct 560. The dry air duct 530 and distribution housing 540 may be fixedly coupled to the wall surface of the inner cabinet 40, and the nozzle duct 560 may be provided inside the inner cabinet 40.

The sump 250 has a structure for containing water. The sump 250 is configured to contain condensate water, generated from the shoe care device 1, therein. The sump 250 is configured to contain condensate water condensed in the condenser 800.

The sump 250 may be located below the dehumidifying material 430.

The sump 250 may be formed integrally with the suction duct 210. That is, the sump 250 may be configured to extend from the suction duct 210. The sump 250 may form the lowermost part of the suction duct 210 and may be configured as a container capable of containing water.

The sump 250 may extend downwards from the end of the horizontal duct 210b of the suction duct 210.

The sump 250 may be configured to extend from the suction duct 210 to a low position on the opposite side of the inlet 42. The sump 250 may be located to face the inlet 42 in the horizontal direction. Based on the vertical boundary surface RP, the condenser 800 and the sump 250 may be located on the opposite side of the inlet 42. In the case where the condenser 800 and the sump 250 are located at the front in the first direction X, the inlet 42 may be located at the rear in the first direction X.

The vertical length of the sump 250 may be greater than the vertical length of a vertical duct 210a of the suction duct 210.

The sump 250 have a first condensate inlet 251 and a second condensate inlet 252 formed as inlets through which condensate water flows into the sump 250.

The air introduced into the suction duct 210 may contain a relatively large amount of moisture in the process of refreshing shoes, and some of the air introduced into the suction duct 210 may be condensed in the suction duct 210. In addition, condensate water inside the inner cabinet 40 may be sucked into the suction duct 210 together with the air. In this case, the condensate water may flow along the suction duct 210, move to the sump 250 formed integrally with the suction duct 210 to be collected, and may then be discharged to the drain tank 70 or to the outside, or may be transported to the steam generator 600 or the like.

As described above, the shoe care device 1 according to the embodiment of the present disclosure may facilitate management and discharge of condensate water.

The blowing duct 230 configures part of the connecting passage F10. The blowing duct 230 configures a passage through which air is supplied to the dehumidifying material 430. The blowing duct 230 configures a passage through which the air passing through the blower 220 moves to the inner passage F11. The blowing duct 230 extends from the blower 220 to be connected to the housing inlet 311. The blowing duct 230 may extend horizontally from the blower 220 to be connected to the housing inlet 311.

The blower 220 may be connected between the suction duct 210 and the blowing duct 230, and a blowing fan 221 may be provided inside the blower 220. Air may be sucked from the inner cabinet 40 and the sucked air may be blown toward the blowing duct 230 by the operation of the blower 220 (rotation of the blowing fan 221).

The suction duct 210, the blower 220, and the blowing duct 230 may together form the first section F10a.

The blowing duct 230 may be connected to one side of the dehumidifying-material housing 300 accommodating the dehumidifying material 430 so that the air blown through the blowing duct 230 may come into contact with the dehumidifying material 430. Accordingly, moisture is removed from the air in contact with the dehumidifying material 430.

The dehumidifying-material housing 300 and the dehumidifying-material block 400 may together form the second section F10b.

The air passing through the dehumidifying-material housing 300 may be blown toward the damper housing 520, which is connected to the opposite side of the dehumidifying-material housing 300.

Dry air flowing into the damper housing 520 flows into the inner cabinet 40 while sequentially passing through the dry air duct 530, the distribution housing 540, the nozzle duct 560, and the nozzle 570.

The damper housing 520, the dry air duct 530, the distribution housing 540, the nozzle duct 560, and the nozzle 570 may form the third section FlOc.

In the embodiment of the present disclosure, the damper 510 may be configured in the form of a damper valve.

The damper 510 may be configured to open the regeneration passage F20 while blocking the third section FlOc, or to open the third section FlOc while blocking the regeneration passage F20.

The damper housing 520 is configured to accommodate the damper 510.

The damper housing 520 has a dry air outlet 523 forming the passage of the connecting passage F10, and a wet air outlet 524 forming the entrance of the regeneration passage F20.

The damper 510 may be configured to selectively close the dry air outlet 523 and the wet air outlet 524. The damper 510 may be configured to selectively seal the dry air outlet 523 and the wet air outlet 524.

In the shoe care device 1 according to an embodiment of the present disclosure, the damper 510 may be configured to be rotatable around a hinge shaft provided on one side. The hinge shaft of the damper 510 may be parallel to the second direction Y. In addition, the shoe care device 1 may be configured to include an actuator 515 that rotates the damper 510 around the hinge shaft of the damper 510. The actuator 515 may be configured as an electric motor and may be configured to rotate the damper 510 in both directions.

The dry air blown into the damper housing 520 may flow back into the inner cabinet 40 through the nozzle 570 to refresh the shoes.

As described above, the air inside the inner cabinet 40 moves into the connecting passage F10 while passing through the inlet 42, the suction duct 210, the blower 220, the blowing duct 230, the dehumidifying-material housing 300 (and the dehumidifying-material block 400), the damper housing 520, the dry air duct 530, the distribution housing 540, the nozzle duct 560, and the nozzle 570 in sequence.

The damper 510 is provided inside the damper housing 520 and controls the movement path of the air passing through the dehumidifying material 430. Upon operation of the damper 510, the air passing through the dehumidifying material 430 may move into the inner cabinet 40 through the nozzle 570, or may move to the regeneration passage F20.

The damper 510 may be configured to selectively close any one of the dry air outlet 523 and the wet air outlet 524. In the case where the damper 510 opens the dry air outlet 523 while closing the wet air outlet 524, the air passing through the dehumidifying material 430 may flow into the inner cabinet 40 through the nozzle 570, and in the case where the damper 510 opens the wet air outlet 524 while closing the dry air outlet 523, the air passing through the dehumidifying material 430 may be condensed while moving through the regeneration passage F20.

The moisture generated in the process of regenerating the dehumidifying material 430 needs to be discharged through a passage separated from the connecting passage (F10) (the third section F10c), which is the passage through which dry air flows. Accordingly, the shoe care device 1 according to the embodiment of the present disclosure is configured to include the regeneration passage F20, and when the dehumidifying material (zeolite) 430 is regenerated, the air passing through the dehumidifying material 430 moves through the regeneration passage F20, instead of being blown to the nozzle 570.

The regeneration passage F20 diverges from the connecting passage F10. The regeneration passage F20 may diverge from the third section FlOc of the connecting passage F10. The regeneration passage F20 leads to the sump 250.

The regeneration passage F20 configures a movement passage for fluid.

The regeneration passage F20 forms a passage through which air passing through the dehumidifying material 430 and/or condensed water flows when the dehumidifying material 430 is regenerated. The entirety or part of the regeneration passage F20 may be configured in the form of a pipe, a tube, a duct, or a combination thereof.

The moisture separated from the dehumidifying material 430 may move to the condenser 800 together with the air moving through the regeneration passage F20 and may then be condensed. In addition, the condensate water condensed in the condenser 800 may move to the sump 250 through the regeneration passage F20, may be collected in the lower portion of the sump 250, and may then be discharged to the drain tank 70 or to the outside or transported to the steam generator 600 or the like.

Referring to FIG. 7, if the damper 510 seals the wet air outlet 524 and opens the dry air outlet 523 in drying module A, and if the damper 510 opens the wet air outlet 524 and seals the dry air outlet 523 in drying module B, some of the air may flow toward drying module A and some may flow toward drying module B in the second section F10b. At this time, drying module A may operate in a dehumidifying mode and drying module B may operate in a regeneration mode.

On the other hand, in FIG. 7, if the damper 510 opens the wet air outlet 524 and seals the dry air outlet 523 in drying module A, and if the damper 510 seals the wet air outlet 524 and opens the dry air outlet 523 in drying module B, some of the air may flow toward drying module B and some may flow toward drying module A in the second section F10b. At this time, drying module B may operate in a dehumidifying mode and drying module A may operate in a regeneration mode.

If the damper 510 seals the wet air outlet 524 and opens the dry air outlet 523 in both drying module A and drying module B, both drying module A and drying module B may operate in a dehumidifying mode.

If the damper 510 seals the dry air outlet 523 and opens the wet air outlet 524 in both drying module A and drying module B, both drying module A and drying module B may operate in a regeneration mode.

The shoe care device 1 according to an embodiment of the present disclosure may further include a sensor unit 490 capable of measuring the amount of moisture adsorbed onto the dehumidifying material 430, and the controller 80 may perform control such that the regeneration mode is performed until the amount of moisture measured by the sensor unit 490 becomes less than or equal to a configured value.

In particular, the controller 80 may control all the heaters 710 to operate until the amount of moisture measured by the sensor unit 490 becomes less than or equal to a configured value.

In this case, the sensor unit 490 may include a moisture sensor that is installed adjacent to the dehumidifying material 430 and measures the amount of moisture adsorbed onto the dehumidifying material 430 as shown in FIG. 7, and the type and number thereof may be configured in various ways as necessary.

As described above, if it is detected that the amount of moisture adsorbed onto the dehumidifying material 430 exceeds a reference value, the shoe care device 1 according to the present embodiment may preferentially regenerate all the dehumidifying materials 430 until the amount of moisture becomes less than or equal to the reference value, thereby consistently maintaining the dehumidifying material 430 in an appropriate state for dehumidification even during the operation of the shoe care device 1 for refreshing shoes.

The inner cabinet 40 may have any shape and structure as long as shoes can be received in the accommodation space 41.

The machine room 50 may be located below the inner cabinet 40 (the inner space).

The dry air and steam supplied to the accommodation space 41 of the inner cabinet 40 tend to rise. In the shoe care device 1 according to the embodiment of the present disclosure, the machine room 50 may be located below the inner cabinet 40, and accordingly, the dry air and steam may naturally move upwards from the machine room 50 toward the inner cabinet 40, so that the dry air and steam may be effectively supplied.

In the shoe care device 1 according to the embodiment of the present disclosure, zeolite that is effective for dehumidification, deodorization, and humidification may be disposed in the machine room 50, and the air that has passed through the zeolite may flow back into the inner cabinet 40 to circulate through the connecting passage F10, and condensate water may be discharged through the regeneration passages F20 when the zeolite is regenerated, thereby effectively managing the shoes.

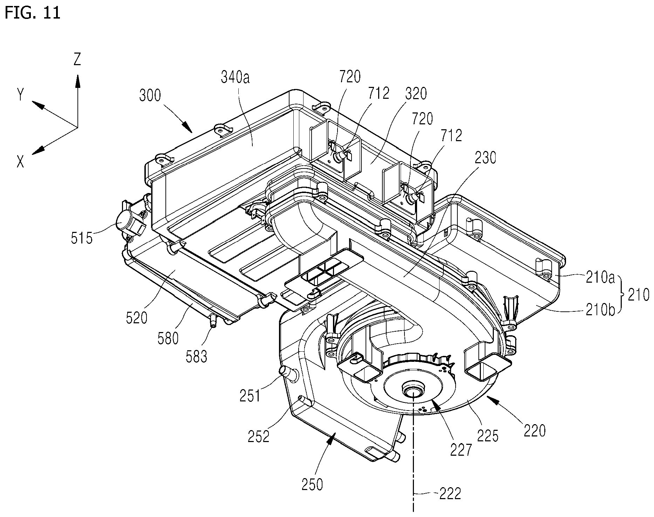

FIG. 11 is a bottom perspective view illustrating some elements provided inside a machine room 50 of a shoe care device 1.

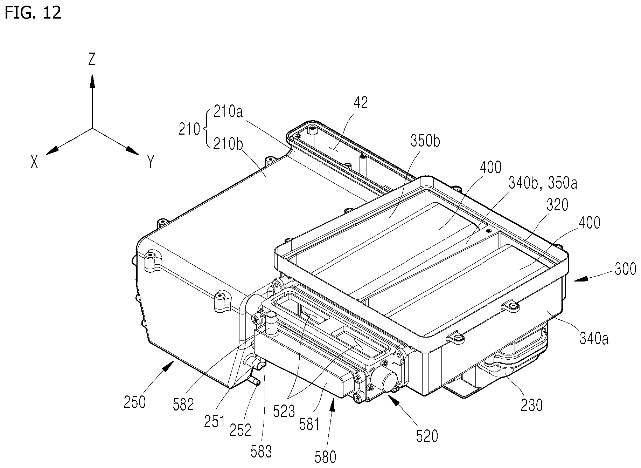

FIG. 12 is a perspective view illustrating some elements of the shoe care device 1 shown in FIG. 11 when viewed in another direction.

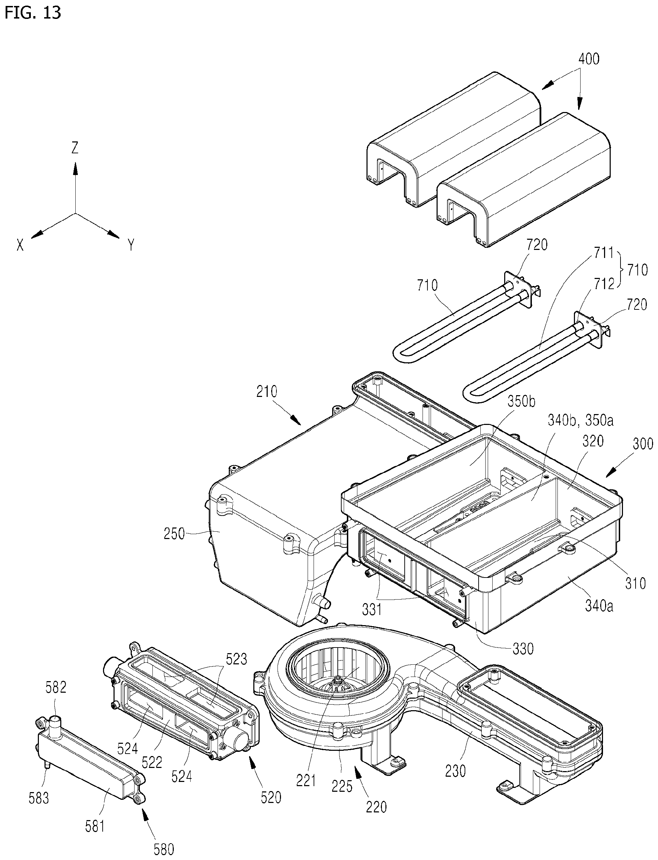

FIG. 13 is a perspective view illustrating the shoe care device 1 shown in FIG. 12 in which some elements are separated from each other.

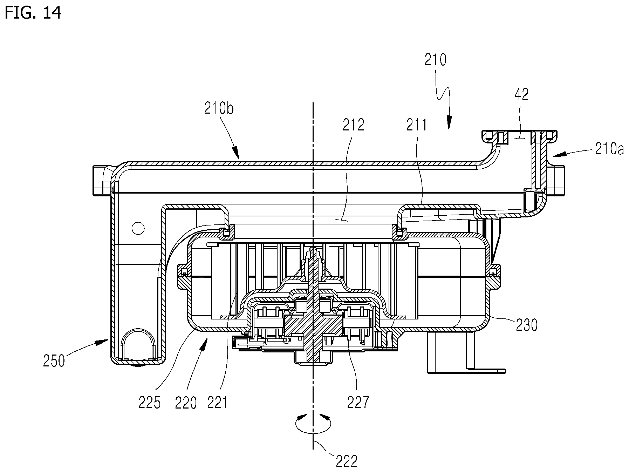

FIG. 14 is a cross-sectional view illustrating some configurations of the shoe care device 1 taken along line E-E' in FIG. 8.

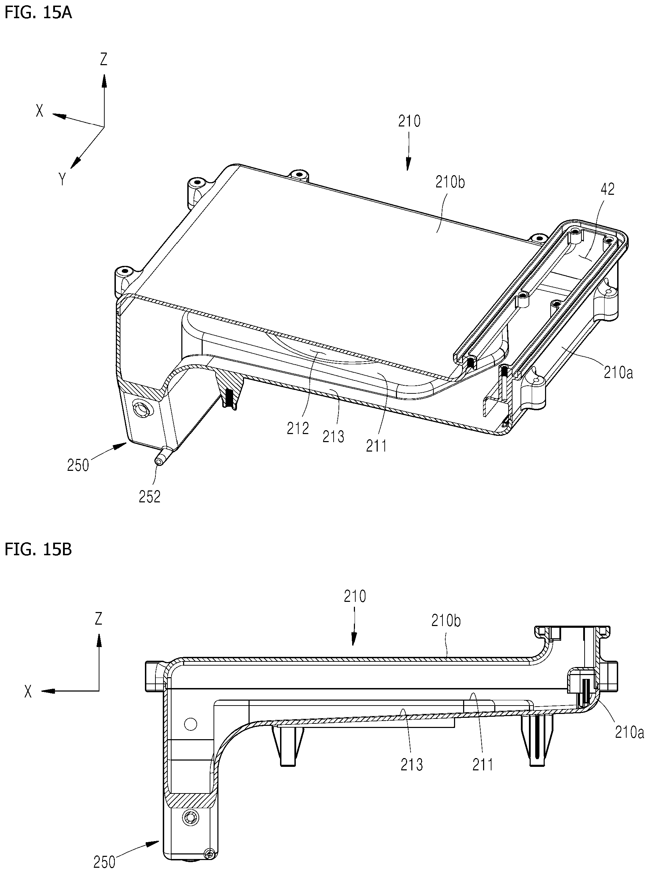

FIG. 15A is a perspective view illustrating a suction duct 210 according to an embodiment of the present disclosure. Here, the suction duct 210 is shown cut along line F-F' in FIG. 8 such that the interior of the suction duct 210 is visible.

FIG. 15B is a cross-sectional view illustrating the suction duct 210 taken along line F-F' in FIG. 8.

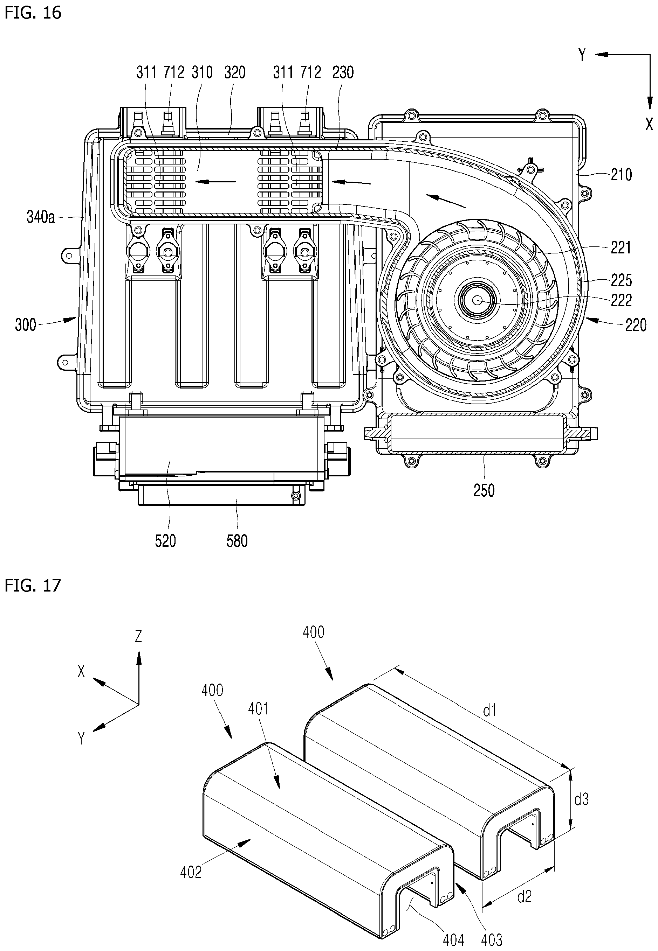

FIG. 16 is a diagram illustrating some configurations of the machine room 50 taken along line D-D' in FIG. 2B. In FIG. 16, the blowing housing 225, the blowing duct 230, and the sump 250 are shown in cross-section.

The suction duct 210 may be configured to extend from the inlet 42.

The suction duct 210 is provided inside the machine room 50.

The suction duct 210 may be configured to connect the inlet 42 and the sump 250 so as to communicate with each other. The upper end of the suction duct 210 is connected to the inlet 42, and the lower end of the suction duct 210 is connected to the sump 250, and the inlet 42 and the sump 250 communicate with each other through the suction duct 210. The air introduced through the suction duct 210 may move to the sump 250, and the air inside the sump 250 may move to the inlet 42.

The suction duct 210 may be configured to include a vertical duct 210a and a horizontal duct 210b.

The vertical duct 210a may be formed in the substantially vertical direction. The vertical duct 210a extends downwards from the inlet 42. The vertical duct 210a may be formed parallel to the third direction Z. The vertical duct 210a may be formed along a direction opposite the third direction Z from the inlet 42 so as to have a substantially constant cross-sectional area.

The horizontal duct 210b extends in the substantially horizontal direction from the lower end of the vertical duct 210a. The horizontal duct 210b may be formed along the first direction X. The horizontal duct 210b may be formed along the first direction X from the lower end of the vertical duct 210a so as to have a substantially constant cross-sectional area.

The length of the horizontal duct 210b in the horizontal direction (first direction X) may be twice the length of the vertical duct 210a in the vertical direction (third direction Z) or more. The length of the horizontal duct 210b in the horizontal direction (first direction X) may be 3 to 10 times the length of the vertical duct 210a in the vertical direction (third direction Z). Accordingly, the overall suction duct 210 is configured to be long in the horizontal direction and to lie in the horizontal direction.

The horizontal duct 210b may be configured to include a central bottom surface 211, a blowing inlet 212, and a condensate water drain 213.

The central bottom surface 211 forms a portion of the bottom surface inside the horizontal duct 210b. The central bottom surface 211 may form the central portion of the inner bottom surface of the horizontal duct 210b. The central bottom surface 211 forms an inner bottom surface of the horizontal duct 210b that protrudes upwards. The front portion (opposite the first direction X) of the central bottom surface 211 may have a tapered shape in which the width of the front portion is reduced as it is closer to the front (opposite the first direction X).

The tapered shape of the front portion of the central bottom surface 211 may enable water on the inner floor of the horizontal duct 210b to naturally move toward the condensate water drain 213.

The blowing inlet 212 is a hole that penetrates the central bottom surface 211 in the third direction Z. The blowing inlet 212 may be formed in the center of the central bottom surface 211. The blowing inlet 212 forms a passage communicating with the inside of the blowing housing 225 of the blower 220.

The blowing inlet 212 is configured in the form of a circular hole. The diameter of the blowing inlet 212 may be determined such that the air flowing in through the suction duct 210 may easily move therethrough. The cross-sectional area of the blowing inlet 212 may be more than 1/2 of the cross-sectional area of the suction duct 210, and the cross-sectional area of the blowing inlet 212 may be similar to or equal to the cross-sectional area of the suction duct 210.

The inner bottom surface of the horizontal duct 210b may be configured to be inclined downwards along the first direction X. The inner bottom surface of the horizontal duct 210b may be configured to be inclined downwards from the vertical duct 210a toward the sump 250.

The condensate water drain 213 is formed at the bottom of the inside of the horizontal duct 210b.

The condensate water drain 213 may form the lowest portion of the floor inside the horizontal duct 210b.

The condensate water drain 213 forms a portion of the inner bottom surface of the horizontal duct 210b. The condensate water drain 213 is located lower than the central bottom surface 211. The condensate water drain 213 may be configured in the form of a groove that is concave downwards from the central bottom surface 211. The condensate water drain 213 may be formed at the edge of the central bottom surface 211. The condensate water drain 213 may be formed on both left and right edges of the central bottom surface 211, respectively.

The condensate water drain 213 may be formed in the entire section of the horizontal duct 210b in the first direction X.

The condensate water drain 213 may be configured to be inclined downwards from the vertical duct 210a toward the sump 250.

The water flowing into the suction duct 210 through the inlet 42 and the vertical duct 210a may flow to the sump 250 along the inclined bottom surface of the horizontal duct 210b and flow into the sump 250. At this time, the water moves along the condensate water drain 213, and since the central bottom surface 211 is higher than the condensate water drain 213, the water is prevented from flowing into the blower 220 through the blowing inlet 212.

The blower 220 constitutes a portion of the connecting passage F10. The blower 220 is configured to generate air flow in the connecting passage F10.

The blower 220 may be connected between the suction duct 210 and the dehumidifying-material housing 300, based on the air movement direction of the connecting passage F10.

The blower 220 may be located between the suction duct 210 and the blowing duct 230, based on the air movement direction of the connecting passage F10, and may be configured to connect the suction duct 210 and the blowing duct 230.

The blower 220 may be configured to connect the horizontal duct 210b and the blowing duct 230 to each other.

The blower 220 may be located below the horizontal duct 210b.

The blower 220 may be configured to include a blowing fan 221, a blowing housing 225, and a motor 227.

The blowing fan 221 may be configured to rotate about the rotation axis 222 in the third direction Z, which is a vertical direction and is perpendicular to the first direction X. The motor 227 of the blower 220 rotates the blowing fan 221.

The blowing housing 225 is configured to accommodate the blowing fan 221. The blowing housing 225 may be formed in a round shape about the rotation axis 222 of the blowing fan 221. The blowing housing 225 may have a circular shape or a spiral shape with respect to the rotation axis 222 of the blowing fan 221.

The blowing housing 225 is configured to communicate with the suction duct 210 and the blowing duct 230, respectively, and configures a portion of the connecting passage F10.

The horizontal size of the blowing housing 225 may be greater than the vertical size of the blowing housing 225. The horizontal size of the blowing housing 225 may be twice the vertical size of the blowing housing 225 or more.

In the shoe care device 1 according to the embodiment of the present disclosure, since the blowing fan 221 is configured to rotate around the rotation axis in the vertical direction, even in the case of a machine room 50 with a smaller vertical height, the diameter of the blowing housing 225 and the diameter of the blowing fan 221 may be configured to be sufficiently large, and the flow rate of air transported by the blower 220 may be sufficiently increased.

The blowing housing 225 may be connected to and communicate with the suction duct 210 on the rotation axis 222 of the blowing fan 221, and may be connected to and communicate with the blowing duct 230 at its edge.

Therefore, the air inside the suction duct 210 flows into the blowing housing 225 near the rotation axis 222 of the blowing fan 221, and the air inside the blowing housing 225 is pressurized toward the edge of the blowing housing 225 as the blowing fan 221 rotates and moves to the blowing duct 230 along the circumferential direction of the blowing housing 225.

One side of the blowing duct 230 communicates with the blowing housing 225 of the blower 220, and the other side communicates with the dehumidifying-material housing 300.

The blowing duct 230 may extend in the horizontal direction from the blowing housing 225. The blowing duct 230 may be configured to be substantially long along the second direction Y. The blowing duct 230 may be coupled to the lower side of the dehumidifying-material housing 300 so as to communicate therewith.

The blowing housing 225 and the blowing duct 230 may form a spiral passage about the rotation axis 222 of the blowing fan 221 together such that the air inside the blowing housing 225 may naturally move to the blowing duct 230 when the blowing fan 221 rotates.

The radial distance from the rotation axis 222 of the blowing fan 221 gradually increases from blowing housing 225 to the blowing duct 230 along the rotational direction of the blowing fan 221. In an embodiment, as shown in FIG. 10, the distance from the rotation axis 222 of the blowing fan 221 to the outer edges of the blowing housing 225 and the blowing duct 230 may be configured to gradually increase in the clockwise direction.

Based on the first direction X, the rear end of the blowing housing 225 or the rear end of the blowing duct 230 may be configured to further protrude backwards in the first direction X, compared to the rear end of the dehumidifying-material block 400, or to be located at the same position as the same.

Accordingly, the air passing through the blowing housing 225 and the blowing fan 221 may flow into the dehumidifying-material housing 300 from the back in the first direction X and naturally move along the direction in which the inner passage F11 of the dehumidifying-material block 400 is formed.

In addition, the above configuration of the blower 220 and the blowing duct 230 enables to stably secure the flow rate of air supplied to the inner space (inner passage F11) of the dehumidifying-material block 400, and in particular, even if a pair of dehumidifying-material blocks 400 is provided, it is possible to supply sufficient air to the dehumidifying-material block 400.

In addition, it is possible to prevent the flow resistance of the air passing through the dehumidifying-material block 400 from unnecessarily increasing while allowing the air moving through the connecting passage F10 to smoothly pass through the dehumidifying-material block 400.

As described above, in the shoe care device 1, the suction duct 210 includes a vertical duct 210a and a horizontal duct 210b, and the blowing housing 225 is coupled to the lower side of the horizontal duct 210b so as to communicate therewith, and the sump 250 extends downwards from the end of the horizontal duct 210b. Therefore, the suction duct 210, the blower 220, and the sump 250 may be arranged inside the machine room 50 without wasting space, and even when the vertical height h2 of the machine room 50 is required to be designed to be relatively small, a sufficient blowing capacity of the blower 220 may be secured.

As described above, since the inlet 42 and the sump 250 communicate with each other through the suction duct 210, and since the condensate water drain 213 is formed on the suction duct 210, the condensate water inside the suction duct 210 may naturally move to the sump 250.

Meanwhile, the air inside the sump 250 is likely to move to the inlet 42 through the suction duct 210. The water vapor inside the sump 250 may move toward the inlet 42 through the suction duct 210. In the shoe care device 1 according to the embodiment of the present disclosure, since the suction duct 210 is configured to be long in the substantially horizontal direction, the water vapor is more likely to be condensed while moving through the suction duct 210, so that the water vapor may condense inside the suction duct 210 and move back to the sump 250. In addition, the water vapor moving from the sump 250 through the suction duct 210 may pass through the blowing inlet 212 of the horizontal duct 210b. At this time, the water vapor may flow into the blower 220 by the operation of the blower 220, thereby preventing the same from entering the inner cabinet 40.

The dehumidifying-material housing 300 is configured as a container capable of accommodating the dehumidifying-material block 400.

The dehumidifying-material housing 300 may be configured as a container of which the upper side is substantially open. In particular, the dehumidifying-material housing 300 may be configured such that the dehumidifying-material block 400 is to be put into or taken out of the dehumidifying-material housing 300 through the upper side thereof. The dehumidifying-material cover 47 may be coupled to the upper side of the dehumidifying-material housing 300 to open and close the upper opening of the dehumidifying-material housing 300.

The dehumidifying-material block 400 is accommodated inside the dehumidifying-material housing 300.

The inner space of the dehumidifying-material housing 300 configures a portion of the connecting passage F10.

The heater 710 is located on the connecting passage F10 and configured to heat the air in the connecting passage F10.

In addition, the heater 710 is configured to heat the dehumidifying-material block 400. The heater 710 is configured to heat the dehumidifying material 430 constituting the dehumidifying-material block 400. To this end, the heater 710 is disposed on the connecting passage F10 to be adjacent to the dehumidifying-material block 400.

Both the dehumidifying-material block 400 and the heater 710 may be accommodated in the dehumidifying-material housing 300.

The heater 710 may be located in the inner space formed by the dehumidifying-material housing 300 and the dehumidifying-material block 400.

The dehumidifying-material housing 300 and the heater 710 will be further described later.

FIG. 17 is a perspective view illustrating the dehumidifying-material block 400 shown in FIG. 13 viewed in another direction.

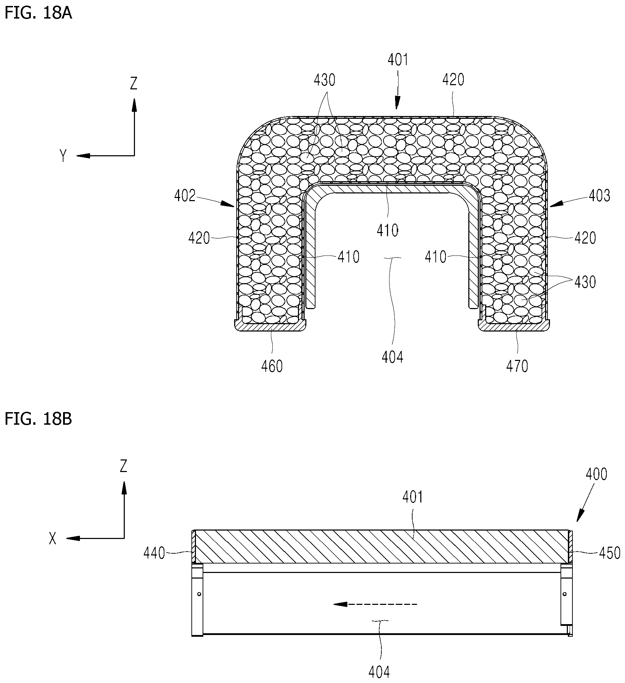

FIG. 18A is a transverse cross-sectional view illustrating a dehumidifying-material block 400 according to an embodiment of the present disclosure.

FIG. 18B is a longitudinal cross-sectional view illustrating a dehumidifying-material block 400 according to an embodiment of the present disclosure.

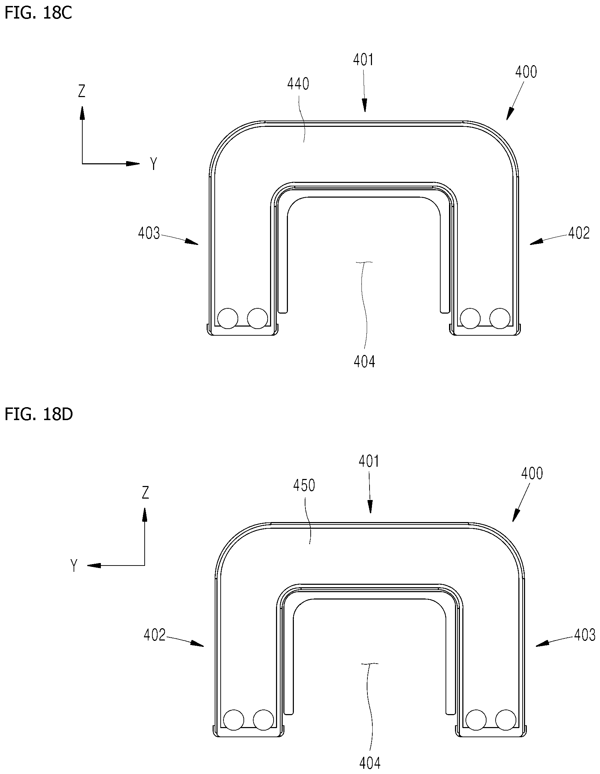

FIG. 18C is a diagram illustrating a dehumidifying-material block 400 viewed from the front in the first direction X according to an embodiment of the present disclosure, and FIG. 18D is a diagram illustrating a dehumidifying-material block 400 viewed from the back in the first direction X according to an embodiment of the present disclosure.

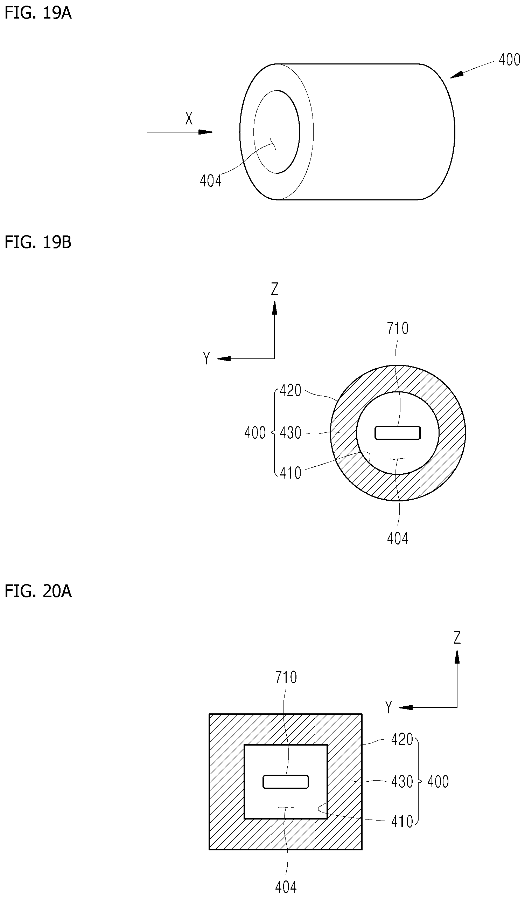

FIG. 19A is a perspective view illustrating a dehumidifying-material block 400 according to an embodiment of the present disclosure.

FIG. 19B is a transverse cross-sectional view illustrating a dehumidifying-material block 400 and a heater 710 according to an embodiment of the present disclosure.

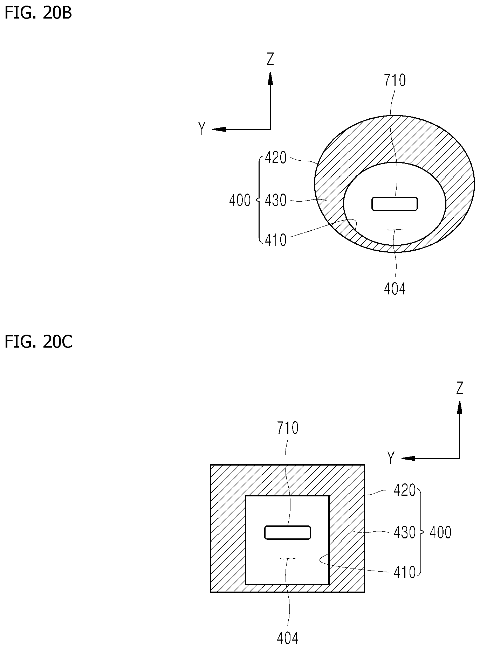

FIGS. 20A, 20B, and 20C are transverse cross-sectional views illustrating a dehumidifying-material block 400 and a heater 710, respectively, according to an embodiment of the present disclosure.

The dehumidifying-material block 400 may have a predetermined length in the first direction X. The dehumidifying-material block 400 may have a length d1 in the first direction X, which is longer than a length d2 in the second direction Y and a length d3 in the third direction Z. Here, the first direction X may indicate the longitudinal direction of the dehumidifying-material block 400.

A plurality of dehumidifying-material blocks 400 may be provided in the shoe care device 1.

A pair of dehumidifying-material blocks 400 may be provided in the shoe care device 1. In this case, the dehumidifying-material blocks 400 may be arranged in the second direction Y perpendicular to the first direction X.

The dehumidifying-material block 400 is located on the connecting passage F10 and is configured to remove moisture from the air passing through the connecting passage F10.

The dehumidifying-material block 400 is configured to form the inner space 404 and such that the air in the inner space 404 moves to pass through the dehumidifying-material block 400 over the entire area thereof. Accordingly, the contact area between the air passing through the connecting passage F10 and the dehumidifying material 430 may be increased.

The dehumidifying-material block 400 may be configured to include an inner mesh 410, an outer mesh 420, and a dehumidifying material 430. In addition, the dehumidifying-material block 400 may be configured to include a first frame 440.

In addition, the dehumidifying-material block 400 may be configured to include a second frame 450.

Each of the inner mesh 410 and the outer mesh 420 may be configured in the form of a mesh, and a plurality of holes may be formed in the inner mesh 410 and the outer mesh 420 over the entire area thereof. The holes of the inner mesh 410 and the outer mesh 420 may be formed smaller than the size of each grain of the dehumidifying material 430 to prevent the dehumidifying material 430 from escaping therefrom.

The inner mesh 410 and the outer mesh 420 may be made of a relatively hard material to maintain the shape of the dehumidifying-material block 400. Each of the inner mesh 410 and the outer mesh 420 may be made of or may be configured to include a material such as a metal, a heat-resistant synthetic resin, synthetic fiber, a carbon fiber, and the like.

Each of the inner mesh 410 and the outer mesh 420 may have a predetermined area, and may be formed in a curved shape, or they may be configured in the form of a combination of flat surfaces bent to each other.

The inner mesh 410 may have a structure to form the inner space 404 of the dehumidifying-material block 400. The heater 710 may be accommodated in the inner space 404 of the inner mesh 410.

The outer mesh 420 is positioned outside the inner mesh 410.

The inner mesh 410 configures the inner surface of the dehumidifying-material block 400, and the outer mesh 420 configures the outer surface of the dehumidifying-material block 400.

Each of the inner mesh 410 and the outer mesh 420 may have a constant section along the first direction X.

As described above, the dehumidifying material 430 is configured as a combination of a plurality of grains (or stones), and the space between the inner mesh 410 and the outer mesh 420 is filled with the dehumidifying material 430.

The dehumidifying-material block 400 may be configured in the form of a pipe or a tunnel extending in the horizontal direction.

The dehumidifying-material block 400 may have various shapes in the cross-section, such as a circle, an ellipse, a polygon, and the like.

The dehumidifying-material block 400 may have a shape that is opened to one side in the cross-section. The opening in the dehumidifying-material block 400 may be directed downwards (the opposite direction of the third direction Z).

The dehumidifying-material block 400 may have a cross-section in a " 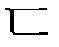 " shape or a "U" shape.

The dehumidifying-material block 400 may have a cross-section in a " 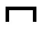 " shape (see FIG. 18A). The dehumidifying-material block 400 in the above structure expands the contact area between the dehumidifying material 430 and the air, enables uniform formation of the outer passage F12 over the entire outer area of the dehumidifying-material block 400, and facilitates separation and replacement of the dehumidifying-material block 400.

In the case where the dehumidifying-material block 400 has a cross-section in a " 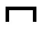 " shape, the inner mesh 410 and the outer mesh 420 may be connected to each other.

In the case where the dehumidifying-material block 400 has a cross-section in a " 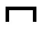 " shape, the dehumidifying-material block 400 may be divided into a ceiling part 401, a first side wall 402, and a second side wall 403.

In the case where the dehumidifying-material block 400 is configured to include the ceiling part 401, the first side wall 402, and the second side wall 403, the inner mesh 410 and the outer mesh 420 may be connected to each other.

The ceiling part 401 may be formed to be flat in the horizontal direction.

Each of the first side wall 402 and the second side wall 403 may be flat in the vertical direction. The first side wall 402 extends downwards from one side of the ceiling part 401, and the second side wall 403 extends downwards from the opposite side of the ceiling part 401. The second side wall 403 may be spaced apart from the first side wall 402, and may be configured to be parallel to the first side wall 402.

The ceiling part 401, the first side wall 402, and the second side wall 403 are configured to have predetermined thicknesses, respectively.

The ceiling part 401, the first side wall 402, and the second side wall 403 are configured to include an inner mesh 410, an outer mesh 420, and a dehumidifying material 430, respectively.

The ceiling part 401, the first side wall 402, and the second side wall 403 are configured to include an inner mesh 410, an outer mesh 420, a dehumidifying material 430, a first frame 440, and a second frame 450, respectively.

The first frame 440 may constitute a front surface of the dehumidifying-material block 400 in the first direction X. The first frame 440 may be configured in the form of a relatively hard plate, and may include a material such as metal, synthetic resin, ceramic, carbon fiber, or the like.

The first frame 440 may constitute front surfaces of the ceiling part 401, the first side wall 402, and the second side wall 403 in the first direction X.

The first frame 440 is coupled along the edges of an inner mesh 410 and an outer mesh 420. The inner mesh 410 and the outer mesh 420 may be fixed to the first frame 440.

The inner mesh 410 and the outer mesh 420 are fixed to the first frame 440 so that the overall structure of the dehumidifying-material block 400 may be stably maintained, thereby maintaining the stable state in which the dehumidifying-material block 400 is filled with the dehumidifying material 430.

The first frame 440 may form a surface perpendicular to or inclined to the first direction X. The first frame 440 may form a surface parallel to the second direction Y and the third direction Z.

The second frame 450 may constitute the rear surface of the dehumidifying-material block 400 in the first direction X. The second frame 450 may be configured in the form of a relatively hard plate, and may include a material such as metal, synthetic resin, ceramic, carbon fiber, or the like.

The second frame 450 may constitute the rear surfaces of the ceiling part 401, the first side wall 402, and the second side wall 403 in the first direction X.

The second frame 450 is coupled along the edges of the inner mesh 410 and the outer mesh 420. The inner mesh 410 and the outer mesh 420 may be fixed to the second frame 450.

The inner mesh 410 and the outer mesh 420 are fixed to the second frame 450 so that the overall structure of the dehumidifying-material block 400 may be stably maintained, thereby maintaining the stable state in which the dehumidifying-material block 400 is filled with the dehumidifying material 430.

The second frame 450 may form a surface perpendicular to or inclined to the first direction X. The second frame 450 may form a surface parallel to the second direction Y and the third direction Z.

The dehumidifying-material block 400 may be configured to include a third frame 460 and a fourth frame 470.

The third frame 460 may constitute a lower surface of the first side wall 402, and the fourth frame 470 may constitute a lower surface of the second side wall 403.

The third frame 460 may connect the inner mesh 410 and the outer mesh 420 of the first side wall 402 to each other, and the fourth frame 470 may connect the inner mesh 410 and the outer mesh 420 of the second side wall 403 to each other.

Each of the third frame 460 and the fourth frame 470 may be formed in the first direction X, may be configured in the form of a relatively hard plate, and may include a material such as metal, synthetic resin, ceramic, carbon fiber, or the like.

In an embodiment, assuming that the shortest distance from the inner mesh 410 to the outer mesh 420 in the dehumidifying-material block 400 is the thickness of the dehumidifying-material block 400, the ceiling part 401, the first side wall 402, and the second side wall 403 may be configured to have the same thickness as each other.

In another embodiment, assuming that the shortest distance from the inner mesh 410 to the outer mesh 420 in the dehumidifying-material block 400 is the thickness of the dehumidifying-material block 400, the thickness of the ceiling part 401 may be configured to be greater than the thickness of the first side wall 402 or second side wall 403.

The inner space 404 surrounded by the ceiling part 401, the first side wall 402, and the second side wall 403 configure an inner passage F11, and the outer space of the ceiling part 401, the first side wall 402, and the second side wall 403 configure an outer passage F12.

The heater 710 may be configured to be surrounded by the ceiling part 401, the first side wall 402, and the second side wall 403.

In the shoe care device 1, the inner passage F11, which is the inner space 404 of the dehumidifying-material block 400, may be configured to be closed or sealed at the rear and front thereof in the first direction X. The inner passage F11, which is the inner space of the dehumidifying-material block 400, may be closed or sealed at the foremost and rearmost thereof in the first direction X.

Therefore, all or most air introduced into the inner passage F11 may pass through the dehumidifying-material block 400, instead of escaping from the inner passage F11 through the rear or front in the first direction X.

As described above, the inner space 404 of the dehumidifying-material block 400 (the inner space of the inner mesh 410) constitutes the inner passage F11 that is a portion of the connecting passage F10. The heater 710 may be located on the inner passage F11.

The dehumidifying-material block 400 has a predetermined length in the first direction X, and the length of the inner passage F11 is the same as or similar to the total length d1 of the dehumidifying-material block 400 in the first direction X.

The inner passage F11 is configured to have a predetermined length in the first direction X. In the embodiment of the present disclosure, the length of the inner passage F11 in the first direction X is configured to be greater than the length (width) thereof in the second direction Y or the length (height) thereof in the third direction Z. That is, the longitudinal direction of the inner passage F11 may be parallel to the first direction X. In a specific embodiment, the length of the inner passage F11 in the first direction X may be configured to be double the length (width) thereof in the second direction Y or the length (height) thereof in the third direction Z.

If the first direction X is the longitudinal direction of the inner passage F11, if the second direction Y is the width direction of the inner passage F11, and if the third direction Z is the height direction of the inner passage F11, the length of the inner passage F11 may be greater than the width and the height of the inner passage F11.

The air introduced into the inlet 42 may flow while passing through the dehumidifying-material block 400 from the inside to the outside thereof.

The inner passage F11 configures the upstream of the second section F10b of the connecting passage F10. That is, when the air of the first section F10a flows to the second section F10b, the air first enters the inner passage F11, and then the air introduced into the inner passage F11 may pass through the dehumidifying-material block 400.

The inner passage F11 may be formed inside the dehumidifying-material block 400, and as described above, the inner passage F11 may be formed long in the first direction X, so that the air moving along the inner passage F11 may move in the direction to pass through the dehumidifying-material block 400 (the direction crossing the first direction X), so the contact area between the air of the connecting passage F10 and the grains of each dehumidifying material 430 may become sufficiently large, thereby increasing the dehumidifying efficiency.

Assuming that the distance between the inner mesh 410 and the outer mesh 420 in the dehumidifying-material block 400 is the thickness of the dehumidifying-material block 400, the thickness of the upper portion of the dehumidifying-material block 400 may be greater than the thickness of the lower portion (see FIG. 20B and FIG. 20C).

The air passing through the inner passage F11 may tend to flow upwards while being heated by the heater 710, and since the thickness of the upper portion of the dehumidifying-material block 400 is greater than the thickness of the lower portion, the air passing through the dehumidifying-material block 400 may come into contact with a large number of grains in the dehumidifying material 430, thereby further improving the dehumidifying efficiency.

In an embodiment, the dehumidifying-material block 400 may have a constant thickness along the first direction X (see FIG. 18B).

In another embodiment, the dehumidifying-material block 400 may have a thickness that varies along the first direction X. For example, the rear portion of the dehumidifying-material block 400 in the first direction X may be thicker, and the dehumidifying-material block 400 may become thinner toward the front thereof in the first direction X.

In particular, the rear portion of the ceiling part 401 of the dehumidifying-material block 400 in the first direction X may be thicker, and the ceiling part 401 may become thinner toward the front thereof in the first direction X. In addition, the ceiling part 401 of the dehumidifying-material block 400 may be configured such that the inner surface thereof is inclined upwards as it is closer to the front thereof in the first direction X.

As will be described later, the housing inlet 311 may face the bottom surface of the ceiling part 401 of the dehumidifying-material block 400, and the housing inlet 311 may be formed at the position close to the rear portion in the first direction X.

In this case, the air injected into the inner passage F11 through the housing inlet 311 may be directed to the ceiling part 401 at the rear in the first direction X, and the air introduced into the inner passage F11 may first come into contact with the dehumidifying material 430 in the thickest portion thereof, thereby improving the contact efficiency between the air and the dehumidifying material 430.

In addition, since the inner surface (bottom surface) of the ceiling part 401 of the dehumidifying-material block 400 is formed to be inclined upwards as it is closer to the front portion in the first direction X, the air passing through the blowing housing 225, the blowing duct 230, and the inner passage F11 may effectively move and unnecessary flow resistance may be minimized.

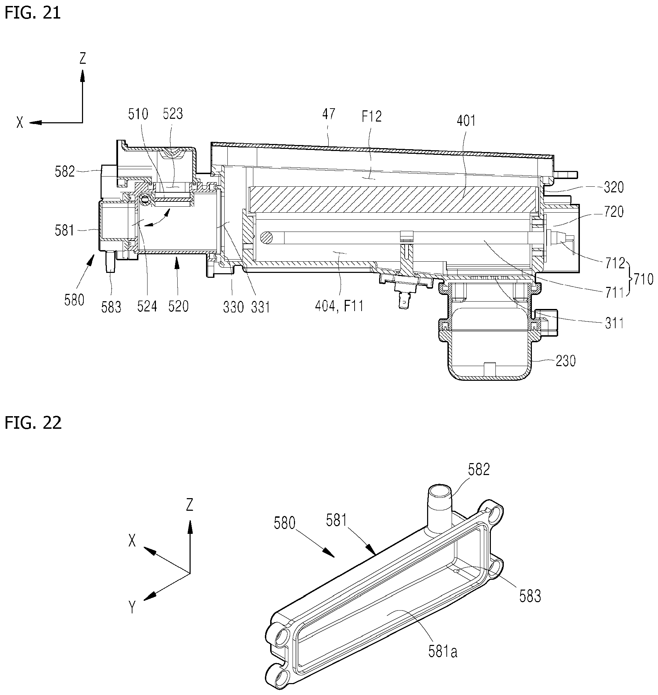

FIG. 21 is a cross-sectional view illustrating some elements provided in a machine room 50 of a shoe care device 1.

The dehumidifying-material block 400 and the dehumidifying material 430 may be located below the bottom (cabinet bottom plate 43 and the dehumidifying-material cover 47) of the inner cabinet 40, and the dehumidifying-material block 400 may be spaced apart from the bottom (cabinet bottom plate 43) of the inner cabinet 40.

The external space of the dehumidifying-material block 400 (the external space of the outer mesh 420) configures the outer passage F12 that is a portion of the connecting passage F10.

The space between the outer surface of the dehumidifying-material block 400 (the outer surface of the outer mesh 420) and the inner surface of the dehumidifying-material housing 300 may configure a portion of the outer passage F12.

The space between the cabinet bottom plate 43 of the inner cabinet 40 and the dehumidifying-material block 400 may configure a portion of the outer passage F12. In particular, the space between the dehumidifying-material cover 47 and the dehumidifying-material block 400 may configure a portion of the outer passage F12.

The air introduced into the inlet 42 passes through the suction duct 210, the blower 220, and the blowing duct 230 to flow into the inner space 404 of the inner mesh 410, and flows from the inner passage F11 to the outer passage F12 while passing through the dehumidifying-material block 400.

The dehumidifying-material housing 300 may be configured to include a housing bottom plate 310, a rear dehumidifying-material wall 320, a front dehumidifying-material wall 330, left dehumidifying-material walls 340a and 340b, and right dehumidifying-material walls 350a and 350b (see FIGS. 12 and 13).

The housing bottom plate 310 may be configured in the form of a plate on which the dehumidifying-material block 400 is placed. The housing bottom plate 310 may be configured in the form of a plate that is substantially flat in the horizontal direction.

A housing inlet 311 is formed on the housing bottom plate 310.

The housing inlet 311 is a hole formed on the housing bottom plate 310 and configures an inlet through which air is introduced into the inner space of the dehumidifying-material block 400. The housing inlet 311 configures an inlet through which air flows into the inner passage F11.

In the housing inlet 311, a net such as a grid type, a mesh type, or the like may be provided.

The housing inlet 311 may be formed to face the bottom surface of the ceiling part 401 of the dehumidifying-material block 400.

The housing inlet 311 may be formed to be closer to the rear portion of the dehumidifying-material housing 300 in the first direction X. The housing inlet 311 may be formed adjacent to the rear end of the housing bottom plate 310 in the first direction X.

The rear dehumidifying-material wall 320, the front dehumidifying-material wall 330, the left dehumidifying-material walls 340a and 340b, and the right dehumidifying-material walls 350a and 350b may respectively constitute wall surfaces erected in the vertical direction. In the dehumidifying-material housing 300, based on the first direction X, the rear dehumidifying-material wall 320 may configure a rear wall surface, the front dehumidifying-material wall 330 may configure a front wall surface, the left dehumidifying-material walls 340a and 340b may configure left wall surfaces, and the right dehumidifying-material walls 350a and 350b may configure right wall surfaces.

The rear dehumidifying-material wall 320 extends upwards from the housing bottom plate 310. In the state in which the dehumidifying-material block 400 is accommodated in the dehumidifying-material housing 300, the rear dehumidifying-material wall 320 is located behind the dehumidifying-material block 400 in the first direction X. In this case, the rear dehumidifying-material wall 320 may be configured to be in close contact with or close to the rear surface of the dehumidifying-material block 400 in the first direction X.

In the case where the rear dehumidifying-material wall 320 is close to the rear surface of the dehumidifying-material block 400 in the first direction X, the gap between the rear dehumidifying-material wall 320 and the dehumidifying-material block 400 may be configured to be very small, for example, about 1 mm or less.

The rear dehumidifying-material wall 320 may be configured to close or seal the inner passage F11, which is the inner space 404 of the dehumidifying-material block 400, at the rear thereof in the first direction X.

The front dehumidifying-material wall 330 extends upwards from the housing bottom plate 310. The front dehumidifying-material wall 330 is located in front of the dehumidifying-material block 400 in the first direction X. In the state in which the dehumidifying-material block 400 is accommodated in the dehumidifying-material housing 300, the front dehumidifying-material wall 330 is spaced forward apart from the dehumidifying-material block 400 in the first direction X. A housing outlet 331 through which the air in the outer passage F12 flows may be formed in the front dehumidifying-material wall 330 so as to penetrate the same in the first direction X.

The left dehumidifying-material walls 340a and 340b connect the rear dehumidifying-material wall 320 and the front dehumidifying-material wall 330 on the left side of the dehumidifying-material block 400 in the first direction X.

The right dehumidifying-material walls 350a and 350b connect the rear dehumidifying-material wall 320 and the front dehumidifying-material wall 330 on the right side of the dehumidifying-material block 400 in the first direction X.

In the case where a pair of dehumidifying-material blocks 400 is provided in the shoe care device 1 according to the embodiment of the present disclosure, the dehumidifying-material housing 300 may be configured to respectively accommodate the pair of dehumidifying-material blocks 400 in separate spaces.

In this case, the dehumidifying-material housing 300 may be configured to include a housing bottom plate 310, a rear dehumidifying-material wall 320, a front dehumidifying-material wall 330, a first left dehumidifying-material wall 340a, a first right dehumidifying-material wall 350a, a second left dehumidifying-material wall 340b, and a second right dehumidifying-material wall 350b.

Any one of the dehumidifying-material blocks 400 is accommodated between the first left dehumidifying-material wall 340a and the first right dehumidifying-material wall 350a, and the other dehumidifying-material block 400 is accommodated between the second left dehumidifying-material wall 340b and the second right dehumidifying-material wall 350b.

In the shoe care device 1 according to an embodiment of the present disclosure, the first right dehumidifying-material wall 350a and the second left dehumidifying-material wall 340b may be formed separately from each other, or may be formed integrally with each other.

In the state in which the dehumidifying-material blocks 400 are accommodated in the dehumidifying-material housing 300, the inner surfaces of the left dehumidifying-material walls 340a and 340b may face the outer surfaces of the first side walls 402 while being spaced apart from each other. Accordingly, predetermined gaps are formed between the left dehumidifying-material walls 340a and 340b and the first side walls 402, and these gaps configure portions of the outer passage F12.

In addition, in the state in which the dehumidifying-material blocks 400 are accommodated in the dehumidifying-material housing 300, the inner surfaces of the right dehumidifying-material walls 350a and 350b may face the outer surfaces of the second side walls 403 while being spaced apart from each other. Accordingly, predetermined gaps are formed between the right dehumidifying-material walls 350a and 350b and the second side walls 403, and these gaps configure portions of the outer passage F12.

In addition, as described above, gaps are formed between the dehumidifying-material cover 47 and the dehumidifying-material blocks 400, and these gaps configure the outer passage F12.

The space configuring the outer passage F12 communicates with the space between the vertical plate 722 and the housing outlet 331 of the front dehumidifying-material wall 330, and the air in the outer passage F12 moves forward in the first direction X through the housing outlet 331 to flow into the damper housing 520.

In the shoe care device 1 according to the embodiment of the present disclosure, based on the third direction Z opposite the direction of gravity, the dehumidifying-material block 400 is located at the same height as or at a higher position than the housing inlet 311, which is the entrance of the inner passage F11, and the inner passage F11 is formed upwards from the lower end of the dehumidifying-material block 400, and the outer passage F12 is formed upwards from the lower end of the dehumidifying-material block 400 so as to surround the inner passage F11.

In the inner passage F11, the air moves in a direction perpendicular to the first direction X while moving along the first direction X.

The pressure inside the inner passage F11 is greater than the pressure inside the outer passage F12, and the pressure at the rear of the inner passage F11 in the first direction X, in which the housing inlet 311 is formed, is greater than the front thereof. In addition, the pressure inside the dehumidifying-material housing 300 is greater than the pressure inside the damper housing 520.

Therefore, considering the movement direction of air and the applied direction of pressure in the connecting passage F10, when the air in the inner passage F11 passes through the dehumidifying-material block 400 to flow to the outer passage F12, the air is able to pass and flow through entire portion of the dehumidifying material 430, enabling maximum utilization of the dehumidifying material 430.

In addition, when the heater 710 is operated in regeneration of the dehumidifying material 430, the air in the inner passage F11 may move more effectively in the direction opposite the direction of gravity (the third direction Z), thereby effectively regenerating the dehumidifying material 430.

The heater 710 may be configured to be fixed to the machine room 50, and the dehumidifying-material block 400 may be configured to be replaceable. The heater 710 may be fixed to the dehumidifying-material housing 300.

The heater 710 may be configured as various devices and structures as long as it is capable of supplying heat to the dehumidifying material 430.

The heater 710 may be configured as an electric heater. In the embodiment of the present disclosure, the heater may include a heating element, and may be configured to supply heat to the surroundings while the heating element emits heat by the supplied electric energy. The heater may include a nichrome wire as a heating element.

The heater 710 may be configured to include a free end 711 and a fixed end 712.

The free end 711 may be formed in the first direction X. In the heater 710, the free end 711 is made of a heating element.

The fixed end 712 may extend from the free end 711 at the rear in the first direction X.

The fixed end 712 may be electrically connected to a power source, and as electric energy is supplied to the free end 711 through the fixed end 712, the free end 711 may emit heat.

When the shoe care device 1 according to the embodiment of the present disclosure is viewed in the second direction Y, the heater 710 may be substantially formed in a "-" shape.

In addition, since the dehumidifying-material block 400 according to the embodiment of the present disclosure is configured to include the ceiling part 401, the first side wall 402, and the second side wall 403, and has a " 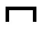 " shape in its cross-section, in the case where the dehumidifying-material block 400 is placed inside the dehumidifying-material housing 300, the heater 710 is accommodated in the inner passage F11 that is the inner space 404 of the dehumidifying-material block 400.

Accordingly, it is possible to provide the shoe care device 1 in which the heater 710 is located in the inner space 404 of the dehumidifying-material block 400 while the dehumidifying-material block 400 is easily attached and detached.

The shoe care device 1 may be configured to include a heater flange 720 to which the heater 710 is fixed.

The heater flange 720 may be made of metal.

The heater flange 720 is configured in a plate that is flat in the vertical direction. The heater flange 720 may be fixed to the front dehumidifying-material wall 330 at the rear in the first direction X.

The fixed end 712 of the heater 710 may be fixed to the heater flange 720.

FIG. 22 is a perspective view illustrating a condensate water separator 580 according to an embodiment of the present disclosure.

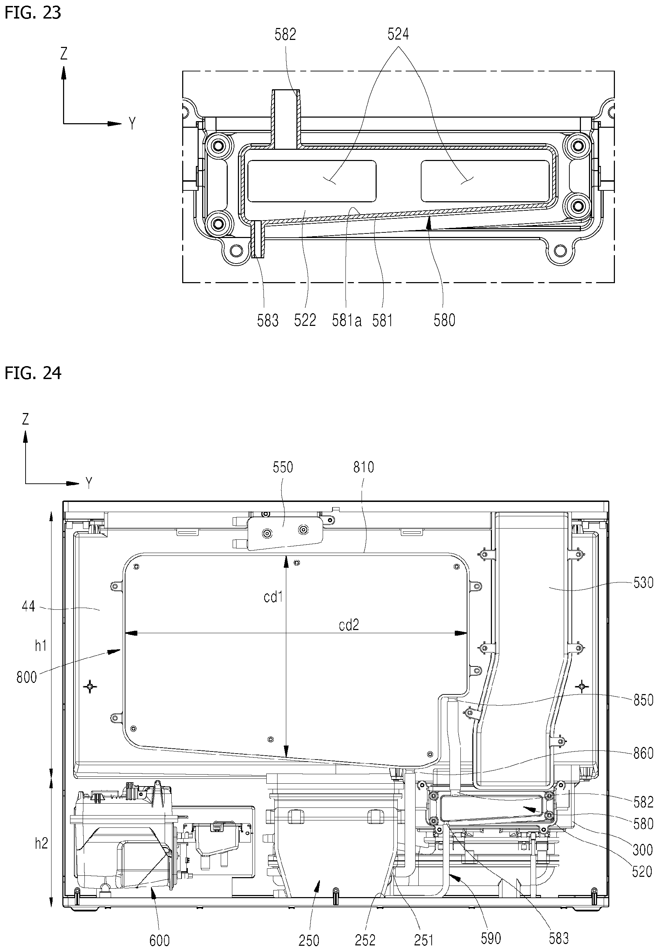

FIG. 23 is a diagram illustrating some configurations of a shoe care device 1 according to an embodiment of the present disclosure. In FIG. 23, the condensate water separator 580 is shown in cross-section so that its interior is visible.

As described above, a dry air outlet 523 constituting the connecting passage F10 is formed in the damper housing 520, and a wet air outlet 524 constituting the entrance of the regeneration passage F20 is also formed therein. In addition, the damper 510 may be configured to selectively seal the dry air outlet 523 and the wet air outlet 524.

When air is dehumidified by the dehumidifying material 430, the damper 510 seals the wet air outlet 524 and opens the dry air outlet 523, so that the dry air inside the damper housing 520 flows into the inner cabinet 40 through the nozzle 570.

When the dehumidifying material 430 is regenerated, the damper 510 seals the dry air outlet 523 and opens the wet air outlet 524, so that the water vapor inside the damper housing 520 flows into the condensate water separator 580.

The condensate water separator 580 constitutes part of the regeneration passage F20. During the regeneration of the dehumidifying material 430, the air passing through the dehumidifying material 430 flows into the condensate water separator 580.

The inside of the condensate water separator 580 may communicate with the inside of the damper housing 520, and the air inside the dehumidifying-material housing 300 may flow into the inside of the condensate water separator 580 through the damper housing 520.

The condensate water separator 580 may be configured to include a regenerated-air discharge housing 581, a steam outlet 582, and a condensate water outlet 583.

The regenerated-air discharge housing 581 forms the overall appearance of the condensate water separator 580 and is configured to have a space therein.

The regenerated-air discharge housing 581 may be coupled to the damper housing 520 at the front in the first direction X.

If two dehumidifying-material blocks 400 are provided in the shoe care device 1 according to the embodiment of the present disclosure and if drying module A and drying module B are provided as described above, both the air passing through drying module A and the air that passing through drying module B may be configured to flow into the regenerated-air discharge housing 581.

Meanwhile, inside the damper housing 520, a central boundary damper wall 521 may be formed to partition the space inside the damper housing 520. The central boundary damper wall 521 may be formed parallel to the first direction X and the third direction Z. The inner space of the damper housing 520 is divided into a first damping space DS1 and a second damping space DS2 by the central boundary damper wall 521, and the first damping space DS1 and the second damping space DS2 are isolated from each other (see FIG. 9).

In the case where the damper housing 520 includes the central boundary damper wall 521, the first damping space DS1, and the second damping space DS2, two dry air outlets 523 and two wet air outlets 524 are also provided. In this case, one dry air outlet 523 communicates with the first damping space DS1, instead of communicating with the second damping space DS2, and the other dry air outlet 523 communicates with the second damping space DS2, instead of communicating with the first damping space DS1. In addition, one wet air outlet 524 communicates with the first damping space DS1, instead of communicating with the second damping space DS2, and the other wet air outlet 524 communicates with the second damping space DS2, instead of communicating with the first damping space DS1.

In the case where two dehumidifying-material blocks 400 are provided in the shoe care device 1 according to the embodiment of the present disclosure as described above, the air passing through one dehumidifying-material block 400 (e.g., the dehumidifying-material block 400 constituting drying module A) flows into the first damping space DS1, and the air passing through the other dehumidifying-material block 400 (e.g., the dehumidifying-material block 400 constituting drying module B) flows into the second damping space DS2.

In addition, since the first damping space DS1 and the second damping space DS2 are provided to be partitioned from each other inside the damper housing 520 as described above, the air inside the first damping space DS1 and the air inside the second damping space DS2 may move independently of each other through the connecting passage F10 or the regeneration passage F20.

In the shoe care device 1 according to an embodiment of the present disclosure, the length of the regenerated-air discharge housing 581 in the second direction Y may be configured to be longer than the length thereof in the first direction X and the length thereof in the third direction Z. In addition, the regenerated-air discharge housing 581 is configured to communicate with both the first damping space DS1 and the second damping space DS2.

Accordingly, the water vapor inside the first damping space DS1 may flow into the regenerated-air discharge housing 581, and the water vapor inside the second damping space DS2 may flow into the regenerated-air discharge housing 581.

In addition, in the case where drying module A and drying module B are provided, both the air passing through drying module A and the air passing through drying module B may flow into the regenerated-air discharge housing 581.

The shoe care device 1 according to the embodiment of the present disclosure may be configured to include a regenerated-air parting wall 522.

The regenerated-air parting wall 522 is a wall that forms the boundary between the condensate water separator 580 and the damper housing 520. The regenerated-air parting wall 522 may be formed integrally with the condensate water separator 580, or may be formed integrally with the damper housing 520.

In the attached drawing illustrating the embodiment of the present disclosure, the regenerated-air parting wall 522 is shown as being integrated with the damper housing 520, and the following description will be made based on this.

The wet air outlet 524 has the regenerated-air parting wall 522 formed to pass through the same.

In addition, the lower edge of the wet air outlet 524 may be higher than the bottom surface 581a inside the condensate water separator 580.

Therefore, when the water vapor generated during the regeneration of the dehumidifying material 430 flows into the condensate water separator 580 and then condenses, the condensate water may be prevented from flowing back into the damper housing 520 and may flow into the sump 250. That is, since the regenerated-air parting wall 522 is formed between the condensate water separator 580 and the damper housing 520, the condensate water inside the condensate water separator 580 may be effectively discharged to the sump 250, instead of moving in the reverse direction of the discharge direction.

The shoe care device 1 according to the embodiment of the present disclosure has a structure that enables, when some of the water vapor separated from the dehumidifying material 430 during the regeneration of the dehumidifying material 430 is condensed before flowing into the condenser 800 so that both the water vapor and the condensate water exist inside the condensate water separator 580, a discharge path (first discharge path F21) of the water vapor to be separated from a discharge path (second discharge path F22) of the condensate water.

The steam outlet 582 forms an outlet through which the air inside the condensate water separator 580 is discharged. The air inside the condensate water separator 580 may move to the condenser 800 through the steam outlet 582. The steam outlet 582 may be formed on the upper side of the regenerated-air discharge housing 581. The steam outlet 582 may be formed on the regenerated-air discharge housing 581 so as to be open upwards in the vertical direction.

The steam outlet 582 may be connected to the condenser inlet 850 of the condenser 800 through a separate hose, pipe, or the like.

The condensate water outlet 583 forms an outlet through which the condensate water inside the condensate water separator 580 is discharged. The condensate water inside the condensate water separator 580 may move to the sump 250 through the condensate water outlet 583. The condensate water outlet 583 may be located lower than the steam outlet 582. The condensate water outlet 583 may be formed on the lower side of the regenerated-air discharge housing 581. The condensate water outlet 583 may be formed on the regenerated-air discharge housing 581 so as to be open downwards in the vertical direction.

The discharge pipe 590 may be configured in the form of a hose or pipe.

One end of the discharge pipe 590 is connected to the condensate water outlet 583, and the other end of the discharge pipe 590 is connected to the second condensate inlet 252 of the sump 250.

The condensate water outlet 583 may be formed at the lower end of the bottom 581a of the regenerated-air discharge housing 581. That is, the condensate water outlet 583 may be formed at the lowest portion of the bottom surface 581a of the regenerated-air discharge housing 581. The bottom surface 581a of the regenerated-air discharge housing 581 may be configured to be inclined downwards toward the condensate water outlet 583. In an embodiment, the bottom surface 581a of the regenerated-air discharge housing 581 may be formed to be inclined downwards along the opposite direction of the second direction Y, and the condensate water outlet 583 may be formed on the bottom surface 581a of the regenerated-air discharge housing 581 at the rear end in the second direction Y.

The water vapor introduced into the condensate water separator 580 may be in the state of being heated to a predetermined temperature and may have ordinary temperature (e.g., 20 ± 5 degrees C) or more. Since this water vapor has a strong tendency to rise, it may naturally move through the steam outlet 582 formed on the upper side of the regenerated-air discharge housing 581 in the upward direction.

The water vapor inside the condensate water separator 580 may flow into the condenser 800 through the steam outlet 582 to be condensed, and the condensate water condensed in the condenser 800 may move to the sump 250. In this case, the path leading to the steam outlet 582 of the condensate water separator 580, the condenser 800, and the sump 250 corresponds to the first discharge path F21.

Meanwhile, some of the water vapor introduced into the condensate water separator 580 may be cooled and condensed inside the regenerated-air discharge housing 581. The condensate water formed as described above may flow along the bottom surface 581a of the regenerated-air discharge housing 581 and may be discharged to the outside of the condensate water separator 580 through the condensate water outlet 583.

The condensate water inside the condensate water separator 580 may move to the sump 250 through the condensate water outlet 583 and the discharge pipe 590. That is, the condensate water inside the condensate water separator 580 may be discharged directly to the sump 250 without passing through the condenser 800. In this case, the path leading to the condensate water outlet 583 of the condensate water separator 580, the discharge pipe 590, and the sump 250 corresponds to the second discharge path F22.

As described above, in the shoe care device 1 according to the embodiment of the present disclosure, since the steam outlet 582 and the condensate water outlet 583 are separately provided in the condensate water separator 580, the water vapor and the condensate water, which may exist together, may respectively move through separate paths (the first discharge path F21 and the second discharge path F22).

In addition, since the steam outlet 582 is formed on the upper side of the condensate water separator 580, and since the condensate water outlet 583 is formed on the lower side of the condensate water separator 580, the water vapor and the condensate water may be discharged in opposite directions according to the properties of water vapor and condensate water, and a stable flow of air (water vapor) may be obtained when regenerating the dehumidifying material 430, and it is possible to prevent residual water from being generated in the condensate water separator 580 and regeneration passage F20.

FIG. 24 is a diagram illustrating a shoe care device 1 from which an outer back plate 21 of an outer cabinet 20 is removed.

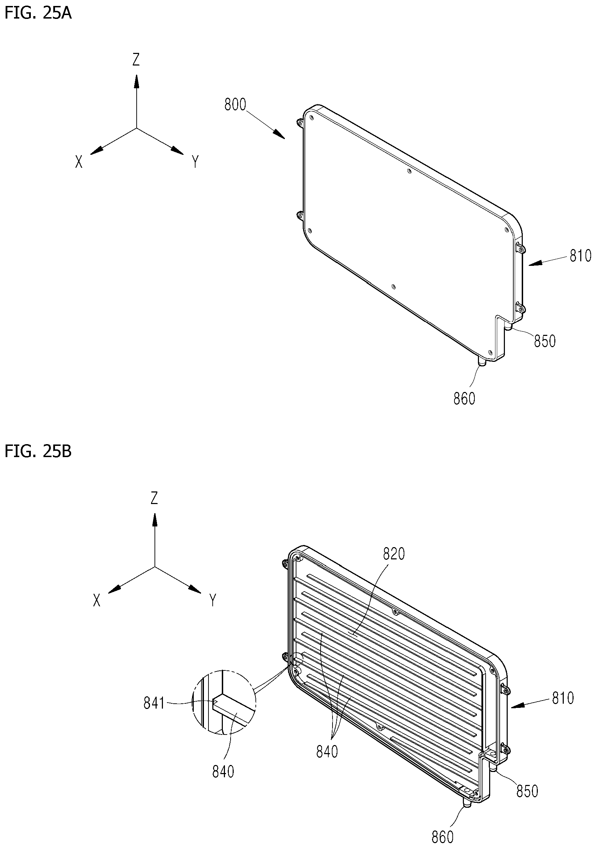

FIG. 25A is a perspective view separately illustrating the condenser 800 shown in FIG. 24.

FIG. 25B is a diagram illustrating the inside of the condenser 800 in FIG. 25A.

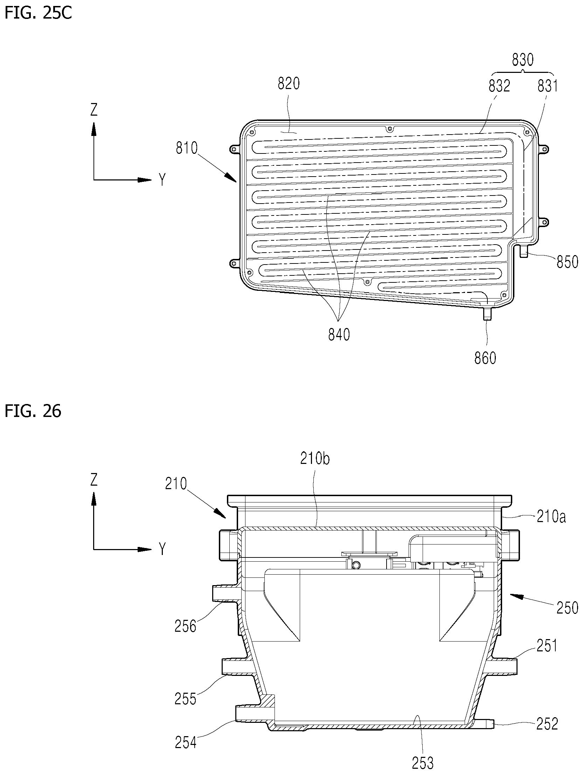

FIG. 25C is a front view illustrating the condenser 800 in FIG. 25B.

The condenser 800 forms part of the regeneration passage F20.

The condenser 800 is configured such that the air passing through the condensate water separator 580 flows into the same and condenses. The condenser 800 is configured such that the air passing through the steam outlet 582 flows into the same, condenses, and then moves to the sump 250.

The condenser 800 is configured to include a condenser housing 810, a passage guide wall 840, a condenser inlet 850, and a condenser outlet 860.

The condenser 800 may be entirely made of metal. The condenser 800 may be made of a metal with excellent thermal conductivity. The condenser 800 may be made of aluminum.

In the embodiment of the present disclosure, the condenser 800 is located between the inner cabinet 40 and the outer cabinet 20. The condenser 800 may be located between the inner back plate 44 and the outer back plate 21.

The thickness of the condenser 800 in the forward and backward directions may be 0.5 to 1 times the distance between the inner back plate 44 and the outer back plate 21. The width of the condenser 800 in the left and right directions and the height thereof in the vertical direction may be equal to or greater than 10 times the thickness of the condenser 800 in the forward and backward directions and equal to or less than 30 times the same, respectively.

The condenser 800 may be in close contact with the inner back plate 44 and/or the outer back plate 21. That is, the condenser 800 may be in close contact with the outer surface of the inner back plate 44 or the inner surface of the outer back plate 21.

The inner back plate 44 and/or the outer back plate 21 may be made of metal with excellent thermal conductivity. In the case where the condenser 800 is coupled to the inner back plate 44 in close contact with each other, the inner back plate 44 may be made of a metal with excellent thermal conductivity, and in the case where the condenser 800 is coupled to the outer back plate 21 in close contact with each other, the outer back plate 21 may be made of a metal with excellent thermal conductivity.

Accordingly, the water vapor moving through the condenser 800 may be effectively condensed.

In the shoe care device 1 according to the embodiment of the present disclosure, the machine room 50 is provided on the lower side of the inner cabinet 40, and the condenser 800 is located between the inner cabinet 40 and the outer cabinet 20. That is, the condenser 800 is located outside the limited space of the machine room 50, instead of inside the machine room 50.

In addition, the condenser 800 may be located between the inner cabinet 40 and the outer cabinet 20, making full use of the space between the inner cabinet 40 and the outer cabinet 20 and thereby obtaining a large area.

In the shoe care device 1 according to the embodiment of the present disclosure, the condenser 800 and the dry air duct 530 may be disposed between the inner back plate 44 of the inner cabinet 40 and the outer back plate 21 of the outer cabinet 20.

The condenser 800 may be formed in a size equivalent to the area of the inner back plate 44 of the inner cabinet 40, excluding the area occupied by the dry air duct 530, and accordingly, the condenser 800 may be configured to have a relatively large area.

In an embodiment, the vertical height cd1 of the condenser 800 may be 0.5 to 1 times the vertical height h1 of the inner cabinet 40, and the lateral width cd2 of the condenser 800 may greater than or similar to the vertical height h1 of the inner cabinet 40.

Depending on the embodiment, the vertical height of the machine room 50 in the shoe care device 1 may configured to be smaller, and the vertical height h2 of the machine room 50 may be less than the vertical height h1 of the inner cabinet 40.

In this case, the space inside the machine room 50 becomes relatively narrow, causing restrictions on the arrangement and design of respective components in consideration of various components constituting the shoe care device 1, which are accommodated in the machine room 50. Furthermore, unlike the present disclosure, if the condenser 800 is disposed inside the machine room 50, the shape and location of the condenser 800 may be restricted, and the heat exchange efficiency of the condenser 800 may be reduced.

In the embodiment of the present disclosure, since the condenser 800 is located between the inner cabinet 40 and the outer cabinet 20, it is possible to effectively arrange the respective components even in the case where the vertical height of the machine room 50 is required to be relatively small.

In addition, since the condenser 800 is located between the inner cabinet 40 and the outer cabinet 20, the condenser 800 may be formed in a relatively thin and wide shape to increase the heat exchange area of the condenser 800, thereby facilitating heat exchange between the external air of the shoe care device 1 and the condenser 800. Accordingly, condensation of water vapor passing through the condenser 800 may be effectively performed.

As the condenser 800 is disposed between the inner back plate 44 and the outer back plate 21, the condenser 800 is located higher than the condensate water separator 580 and the sump 250 disposed inside the machine room 50.

Accordingly, the water vapor inside the condensate water separator 580 may naturally rise and flow into the condenser 800, and the condensate water condensed inside the condenser 800 may naturally descend by gravity to flow into the sump 250, so that water vapor may be effectively condensed and discharged when regenerating the dehumidifying material 430.

The condenser housing 810 forms the overall appearance of the condenser 800. The condenser housing 810 has a condensation space 820, which is an inner space of the condenser 800, therein.

The condenser inlet 850 forms the entrance of the condenser 800 and also forms the entrance of the condensation space 820.

The condenser inlet 850 is connected to the steam outlet 582 so as to communicate with the same. The condenser inlet 850 is connected to the steam outlet 582 582 by a separate pipe or hose constituting the regeneration passage F20.

The condenser inlet 850 may be located higher than the steam outlet 582. Therefore, the water vapor inside the condensate water separator 580 may move upwards through the steam outlet 582 and the condenser inlet 850, so that the water vapor may naturally move from the condensate water separator 580 to the condenser 800.

The condenser outlet 860 forms the exit of the condenser 800 and also forms the exit of the condensation space 820.

The condenser outlet 860 may be formed at the lower end of the condenser 800. That is, the condenser outlet 860 may be formed at the lowest portion of the condensation space 820.

The condenser outlet 860 is connected to the sump 250. The condenser outlet 860 is connected to the first condensate inlet 251 through a separate pipe or hose constituting the regeneration passage F20.

The passage guide wall 840 is configured in the form of a narrow and long plate. The passage guide wall 840 may be provided in the inside (condensation space 820) of the condenser housing 810 and a plurality of passage guide walls may be provided.

A plurality of passage guide walls 840 is arranged to be spaced apart from each other and cross each other, thereby forming a path through which water vapor and condensate water move. The passage guide wall 840 forms a condenser 800 passage, which is a path leading from the condenser inlet 850 to the condenser outlet 860 in the condensation space 820. The condenser passage 830 is a passage for air (or water) provided inside the condenser 800 and connects the condenser inlet 850 and the condenser outlet 860.

The condenser 800 passage may be configured to include an introduction section 831 and a condensation section 832.

As the plurality of passage guide walls 840 is spaced apart from each other in parallel, a condensation section 832 may be provided between the passage guide walls 840.

The condensation section 832 may be formed in a zigzag shape to extend from the uppermost of the condensation space 820 to the lower portion.

In the present disclosure, the angle between the passage guide wall 840 forming the condensation section 832 and the horizontal plane may be 1 to 10 degrees. The passage guide wall 840 forming the condensation section 832 may be formed substantially in the second direction Y, and may be disposed to be inclined upwards along the second direction Y.

In this case, a drainage groove 841, which is a passage vertically passing through the passage guide wall 840, is formed at the lower end of the passage guide wall 840.

In the embodiment of the present disclosure, the lengths of the passage guide walls 840 forming the condensation section 832 may be 200 to 400 mm, the widths of the passage guide walls 840 may be 5 to 20 mm, and the spacing between the passage guide walls 840 may be 10 to 25 mm.

According to the above-described configuration, the water vapor may move along the zigzag-shaped condensation section 832 in the condensation space 820, thereby increasing the contact area and contact time between the water vapor and the condenser 800, so that heat exchange between the water vapor and the condenser 800 and condensation of the water vapor may be effectively performed.

In addition, since the passage guide wall 840 is formed to be inclined and since a drainage groove 841 is formed at the lower end of the passage guide wall 840, the condensate water formed in the condensation space 820 may move downwards along the surface of the passage guide wall 840 and move smoothly in the direction of gravity, instead of pooling inside the condenser 800, thereby effectively preventing residual water from occurring inside the condenser 800.

The introduction section 831 may lead upwards from the condenser inlet 850 to the upper starting point of the condensation section 832.

FIG. 26 is a diagram illustrating a sump 250 according to an embodiment of the present disclosure. In FIG. 26, the sump 250 is shown in cross-section to show its internal configuration.

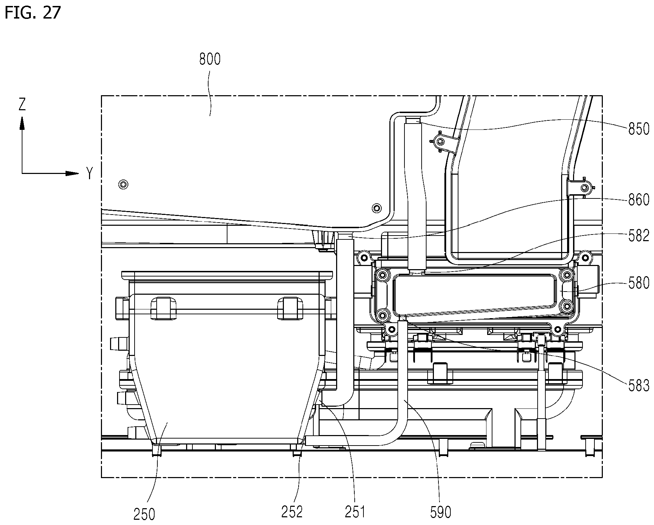

FIG. 27 is a diagram illustrating some configurations of a shoe care device 1 according to an embodiment of the present disclosure, which shows the connection and positional relationships of a condensate water separator 580, a condenser 800, and a sump 250.

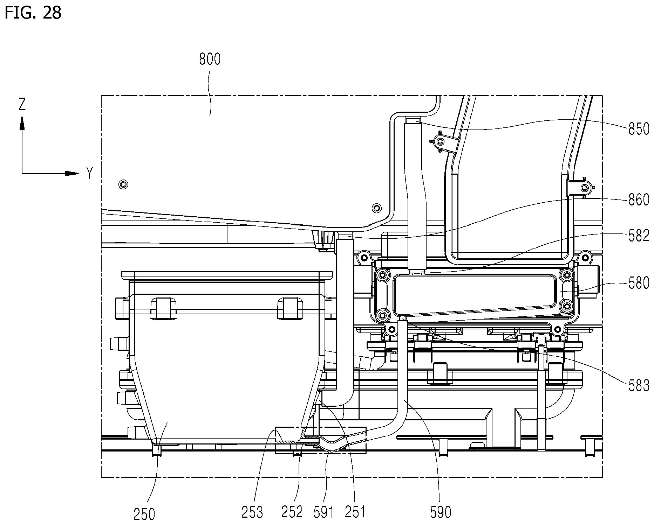

FIG. 28 is a diagram illustrating some configurations of a shoe care device 1 according to an embodiment different from that in FIG. 27.

As described above, the sump 250 and the suction duct 210 are located in the machine room 50, and the sump 250 extends downwards from the suction duct 210. In addition, the condenser 800 may be located above the machine room 50.

In the shoe care device 1 according to the embodiment of the present disclosure, the sump 250 is located lower than the condenser 800 so that the condensate water generated inside the condenser 800 may flow downwards into the sump 250, and the condensate water may naturally move in the regeneration passage F20.

The condensate water inside the sump 250 may be discharged directly to the sump 250 through the discharge pipe 590.

The sump 250 is configured to include a first condensate inlet 251 and a second condensate inlet 252.

The first condensate inlet 251 and the second condensate inlet 252 respectively form an inlet through which condensate water flows into the sump 250.

The first condensate inlet 251 is connected to the condenser outlet 860 so as to communicate with the same. The first condensate inlet 251 may be located lower than the condenser outlet 860. The first condensate inlet 251 may be connected to the condenser outlet 860 by a separate hose constituting the regeneration passage F20. The condensate water discharged from the condenser outlet 860 flows into the sump 250 through the first condensate inlet 251.

The second condensate inlet 252 is located lower than the condensate water separator 580. The second condensate inlet 252 is located lower than the condensate water outlet 583.

The second condensate inlet 252 is connected to the condensate water outlet 583 so as to communicate with the same. The second condensate inlet 252 is connected to the condensate water outlet 583 through a discharge pipe 590. The condensate water discharged from the condensate water outlet 583 flows into the sump 250 through the discharge pipe 590 and the second condensate inlet 252.

The first condensate inlet 251 may be located lower than the condensate water outlet 583.

The second condensate inlet 252 may be located lower than the first condensate inlet 251.

The second condensate inlet 252 may be located at the same level or lower than the bottom 253 of the sump 250. Accordingly, when some water is stored inside the sump 250, the second condensate inlet 252 may be filled with this water.

The sump 250 is configured to include a sump outlet 254.

The sump outlet 254 forms an outlet through which the condensate water inside the sump 250 is drained. The sump outlet 254 may be formed adjacent to the bottom 253 of the sump 250. The sump outlet 254 may be configured to be equal to or lower than the bottom 253 of the sump 250.

The sump outlet 254 may be connected to the drain tank 70 by a hose or the like.

A third condensate inlet 255 and a fourth condensate inlet 256 may be formed in the sump 250.

The third condensate inlet 255 may be connected to the drain tank 70 or the second water pump 71 through a separate hose or the like, and water exceeding the storage capacity of the drain tank 70 may flow back into the sump 250 through the third condensate inlet 255.

The fourth condensate inlet 256 may be connected to the steam separator 550 through a separate hose or the like, and the condensate water inside the steam separator 550 may flow into the sump 250 through the fourth condensate inlet 256.

The discharge pipe 590 connects the condensate water outlet 583 and the second condensate inlet 252 such that the condensate water inside the condensate water separator 580 is discharged directly to the sump 250.

As described above, in the shoe care device 1 according to the embodiment of the present disclosure, if both water vapor and condensate water exist inside the condensate water separator 580, the water vapor inside the condensate water separator 580 moves through the first discharge path F21 to be condensed and then stored in the sump 250, and the condensate water inside the condensate water separator 580 moves directly to the sump 250 through the second discharge path F22 to be stored therein.

However, since the condensate water separator 580 is connected to the sump 250 through the discharge pipe 590, water vapor, instead of condensate water, is likely to be discharged through the discharge path (second discharge path F22) for discharge of condensate water. In this case, if water vapor before condensation flows into the sump 250, the water vapor may move into the inner cabinet 40 through the suction duct 210, so unintentional residual water may be generated in a specific portion inside the shoe care device 1, thereby reducing the regeneration efficiency of the dehumidifying material 430.

In the shoe care device 1 according to the embodiment of the present disclosure, the discharge pipe 590 may be configured to include a trap section 591, thereby effectively avoiding the above-mentioned problems.

The trap section 591 is curved to be convex downwards from the discharge pipe 590. Accordingly, when water moves through the discharge pipe 590, the water may be stored in the trap section 591.

In the shoe care device 1 according to the embodiment of the present disclosure, the trap section 591 may be located lower than the condensate water outlet 583. Additionally, the trap section 591 may be located lower than the bottom surface 253 of the sump 250.

When regenerating the dehumidifying material, the water vapor separated from the dehumidifying material 430 moves into the condensate water separator 580, and a significant portion thereof is condensed while passing through the condenser 800, and the condensed water is stored in the sump 250.

In the shoe care device 1 according to the embodiment of the present disclosure, if water vapor generated during the regeneration of the dehumidifying material is condensed while passing through the condenser 800, the condensate water moves to the trap section 591 of the discharge pipe 590 in a close contact with the bottom surface 253 inside the sump 250 and fills the trap section 591. In addition, even if the water vapor is condensed inside the condensate water separator 580 before passing through the condenser 800, the condensate water moves to the trap section 591 of the discharge pipe 590 through the discharge pipe 590 and fills the trap section 591.

As described above, even if a very small amount of condensate water is generated during the regeneration of the dehumidifying material 430, this condensate water may fill the trap section 591, and the condensate water filled into the trap section 591 may block the movement of water vapor through the discharge pipe 590.

The shoe care device 1 according to an embodiment of the present disclosure may be configured to include an inner cabinet 40, a steam generator 600, an inlet 42, a nozzle 570, an air supplier 10, and a controller 80.

The inner cabinet 40 has an accommodation space 41 formed to accommodate shoes, and the inlet 42 and the nozzle 570 may be provided therein.

The steam generator 600 is configured to supply steam into the inner cabinet 40. Since steam may be supplied to the inner cabinet 40 by the steam generator 600 to perform steam treatment on the shoes, it is possible to exert the effect of sterilization by the high temperature steam and the refreshing effect by inflating the material of the shoes.

The inlet 42 may be formed on one inner side of the inner cabinet 40 to suck the air from the accommodation space 41, and the air inside the inner cabinet 40 may move to the connecting passage F10 through the inlet 42.

The nozzle 570 may be installed inside the inner cabinet 40 so as to be inserted into the shoe and spray at least one of steam or air into the shoe, and the air passing through the connecting passage F10 and the steam supplied from the steam generator 600 may be sprayed into the shoes through the nozzle 570 inside the inner cabinet 40.

In particular, the nozzle 570 may spray at least one of steam and air while being inserted into the shoe, thereby effectively injecting the steam and air into the shoe.

The air supplier 10 blows the air in the accommodation space 41, and the dehumidifying material 430 is disposed on the path of the blown air and heated by the same.

In this case, as the dehumidifying material 430 is heated, the moisture adsorbed onto the dehumidifying material 430 is separated so that the dehumidifying material 430 may be regenerated into the state capable of performing a dehumidifying function.

To this end, the air supplier 10 may be configured to include a duct, a blower 220, a dehumidifying-material housing 300, a heater 710, a damper housing 520, and a damper 510, which are disposed inside the machine room 50.

In particular, in the air supplier 10, a connecting passage F10 through which air circulates between the inlet 42 and the nozzle 570 and a regeneration passages F20 through which the air passing through the dehumidifying material 430 is blown to portions other than the nozzle 570 may be formed.

Accordingly, the air in the accommodation space 41 may be blown by the air supplier 10 to move to the connecting passage F10 or the regeneration passages F20 while passing through the dehumidifying material 430.

The controller 80 may control the air supplier 10 such that the connecting passage F10 and the regeneration passages F20 are selectively opened and closed depending on whether or not the dehumidifying material 430 is heated.

That is, the connecting passage F10 and the regeneration passages F20 may be selectively opened and closed by the controller 80 depending on whether or not the dehumidifying material 430 is in the state of being regenerated.

As described above, in the shoe care device 1 according to the present embodiment, the dehumidifying material 430 may be disposed in the air supplier 10 to capture moisture and bacteria from the blown air, and the dehumidifying material 430 may be heated to regenerate in the air supplier 10, thereby adequately maintaining the performance for processing shoes.

In addition, in the shoe care device 1 according to the present embodiment, since the connecting passage F10 through which the air circulates is formed between the inlet 42 and the nozzle 570 provided inside the inner cabinet 40, it is possible to prevent the user from being exposed to the air used in dehumidification and deodorization of shoes.

In addition, in the shoe care device 1 according to the present embodiment, a pair of dehumidifying materials 430 may be disposed in the air supplier 10, and the connecting passage F10 and the regeneration passages F20 may be formed in the respective dehumidifying materials 430, and the connecting passage F10 and the regeneration passages F20 may be selectively opened and closed depending on the need for a dehumidifying mode and a regeneration mode, so the shoe care device 1 may be operated in an optimal state depending on the situation, thereby further improving the efficiency of processing shoes.

Specifically, in the shoe care device 1 according to an embodiment of the present disclosure, the air supplier 10 may include a chamber 15, a heater 710, a dry air outlet 523, a wet air outlet 524, and a damper 510, and may further include a condenser 800.

A pair of chambers 15 may be provided as separated spaces on the connecting passage F10 to separately accommodate the respective dehumidifying materials 430. This chamber 15 may be configured to include a portion of a dehumidifying-material housing 300 and a portion of a damper housing 520.

The heater 710 may be installed in each chamber 15 to heat the dehumidifying material 430, and may be disposed in the connecting passage F10 so as to be adjacent to the dehumidifying material 430.

The dry air outlet 523 may be formed in each chamber 15 to discharge the air passing through the dehumidifying material 430 toward the nozzle 570 and may be formed in the damper housing 520 to be opened and closed by the damper 510.

The wet air outlet 524 may be formed in each chamber 15 separately from the dry air outlet 523 to discharge the air passing through the dehumidifying material 430 in a direction other than the direction to the nozzle 570 and may be formed in the damper housing 520 to be opened and closed by the damper 510.

The damper 510 may be installed in each chamber 15 to selectively open and close the dry air outlet 523 and the wet air outlet 524 and may be installed in the damper housing 520.

In this case, the controller 80 may control the damper 510 to selectively open and close the dry air outlet 523 and the wet air outlet 524 depending on whether or not each heater 710 operates.

As described above, in the shoe care device 1 according to the present embodiment, since the air supplier 10 includes the chamber 15, the heater 710, the dry air outlet 523, the wet air outlet 524, and the damper 510, the controller 80 may control the damper 510 to selectively execute the dehumidifying mode and the regeneration mode.

The condenser 800 may be connected to the wet air outlet 524 and condense moisture in the air discharged through the wet air outlet 524, and may constitute a portion of the regeneration passages F20 so that the condensate water condensed in the condenser 800 may flow through the regeneration passages F20.

As described above, in the shoe care device 1 according to the present embodiment, since the air supplier 10 further includes the condenser 800, it is possible to condense moisture generated during the regeneration of the dehumidifying material 430.

The shoe care device 1 according to an embodiment of the present disclosure may be configured to include an inner cabinet 40, a steam generator 600, an inlet 42, a nozzle 570, a connecting passage F10, a blowing fan 221, and a dehumidifying material 430.

The connecting passage F10 is a portion through which air circulates between the inlet 42 and the nozzle 570 so that the inlet 42 configures the entrance of the connecting passage F10 and the nozzle 570 configures the exit of the connecting passage F10.

That is, the connecting passage F10 may be an air path through which the air is sucked into the air supplier 10 from the inside of the inner cabinet 40, blown to be dehumidified by passing through the dehumidifying material 430, and then supplied back to the inside of the inner cabinet 40.

The blowing fan 221 may be installed on the connecting passage F10 to blow air from the inlet 42 to the nozzle 570. The air may be sucked from the inner cabinet 40 and the sucked air may be blown through the connecting passage F10 by the operation of the blowing fan 221.

In the shoe care device 1 according to an embodiment of the present disclosure, the nozzle 570 may have a spray hole 571 formed at the end thereof and may be installed to adjust the direction in which the spray hole 571 is inserted into the shoe.

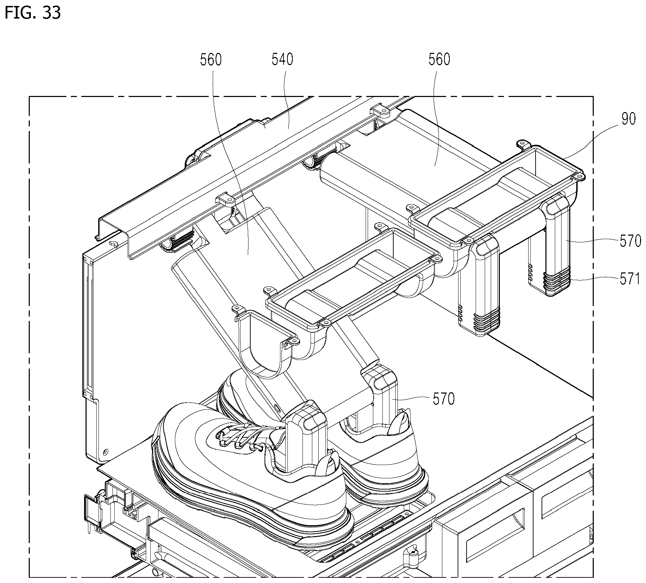

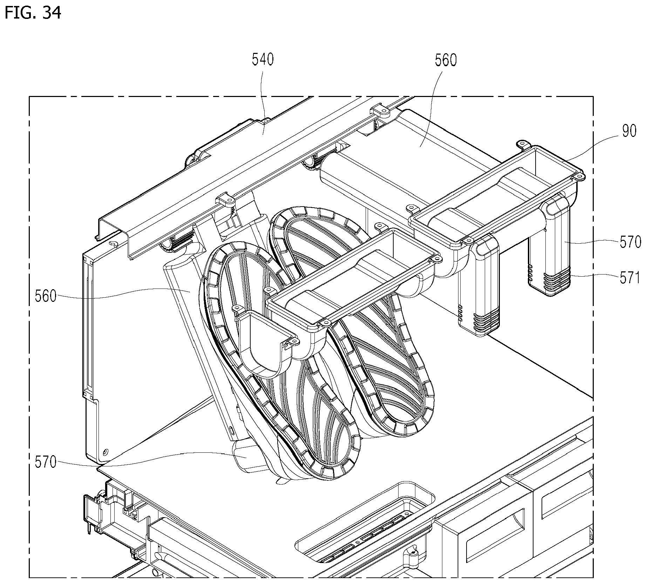

That is, as shown in FIGS. 33 and 34, the insertion direction of the nozzle 570 into the shoe may be adjusted. Accordingly, the shoes may be processed in the state in which various types of shoes are accommodated inside the inner cabinet 40 in various directions.

As described above, in the shoe care device 1 according to this embodiment, since the nozzle 570 is installed to be inserted into the shoe and spray at least one of steam and air into the shoe, and since the insertion direction of the nozzle 570 is adjustable, the shoe care device 1 may be operated to adjust the receiving direction of the shoes as needed, thereby improving shoe treatment efficiency.

In the shoe care device 1 according to an embodiment of the present disclosure, the nozzle 570 may spray both steam and air into the shoe in the case where the spray hole 571 is inserted into the shoe from above.

That is, as shown in FIG. 33, after the shoe is received in the inner cabinet 40 in a normal state, the nozzle 570 may be inserted into the shoe from above.

This case may be a normal condition that does not require special care for the shoes, a general mode may be performed to spray both steam and air into the shoes.

As described above, in the shoe care device 1 according to the present embodiment, since the nozzle 570 is inserted into the shoe from above and sprays both steam and air to the shoe, the steam and dry air may be effectively provided into the shoes when the shoes require general refreshing.

On the other hand, in the shoe care device 1 according to an embodiment of the present disclosure, if the nozzle 570 is inserted into the shoe from the bottom, only air may be sprayed into the shoe.

That is, as shown in FIG. 34, the shoe may be received in the inner cabinet 40 in an inverted state, and the nozzle 570 may be inserted into the shoe from the bottom.

This case may correspond to the case where a large amount of moisture existing inside the shoe is required to be discharged to the outside of the shoe, so that a drying mode may be performed to spray only air into the shoe, instead of steam.

As described above, in the shoe care device 1 according to this embodiment, since the nozzle 570 is inserted into the shoe from the bottom and sprays only air into the shoe, only dry air may be sprayed into the shoe when drying the shoes is more necessary, thereby effectively discharge the moisture inside the shoes.

The shoe care device 1 according to an embodiment of the present disclosure may further include a nozzle duct 560 that constitutes a portion of the connecting passage F10 and is installed to protrude to the inside of the inner cabinet 40 to form a passage for steam and air.

That is, one end of the nozzle duct 560 is connected to the inner cabinet 40 and the other end is connected to the nozzle 570, thereby forming a passage through which steam and air move to the nozzle 570. In this case, one end of the nozzle duct 560 may be connected to the distribution housing 540.

In particular, the nozzle duct 560 may be installed to protrude to the inside of the inner cabinet 40, so that the nozzle 570 may be disposed at various positions inside the inner cabinet 40.

As described above, the shoe care device 1 according to this embodiment may further include the nozzle duct 560 installed to protrude into the inner cabinet 40 between the distribution housing 540 and the nozzle 570, so that air may move stably and effectively from the distribution housing 540 to the nozzle 570.

In the shoe care device 1 according to an embodiment of the present disclosure, the nozzle 570 may be coupled to the nozzle duct 560 by a hinge. That is, the nozzle 570 may be coupled to the hinge shaft formed at the other end of the nozzle duct 560, so that the nozzle 570 may rotate around the hinge shaft.

As described above, in the shoe care device 1 according to this embodiment, since the nozzle 570 is hinge-coupled to the nozzle duct 560, the nozzle 570 may be rotated with respect to the nozzle duct 560 around the hinge as needed, thereby adjusting the angle thereof.

In the shoe care device 1 according to an embodiment of the present disclosure, the nozzle duct 560 may be coupled to the inner cabinet 40 by a hinge. That is, the hinge shaft formed at one end of the nozzle duct 560 may be coupled to the inner cabinet 40, so that the nozzle duct 560 may rotate around the hinge shaft. In this case, the nozzle duct 560 may be hinge-coupled to the distribution housing 540.

As described above, in the shoe care device 1 according to this embodiment, since the nozzle duct 560 is hinge-coupled to the inner cabinet 40, the nozzle duct 560 may be rotated with respect to the inner cabinet 40 around the hinge as needed, thereby adjusting the angle thereof.

In the shoe care device 1 according to an embodiment of the present disclosure, the nozzle duct 560 may be installed to protrude from one upper side of the inner cabinet 40.

That is, the nozzle duct 560 may be coupled to the upper portion of the inner cabinet 40 and protrude to extend downwards. Additionally, the nozzle duct 560 may be coupled to one side of the inner cabinet 40 and protrude to extend toward the other side.

Therefore, the nozzle duct 560 may be located in the upper portion of the inner cabinet 40 when the nozzle 570 is not in use, thereby avoiding occupying the inner space of the inner cabinet 40. Additionally, in order to use the nozzle 570, the nozzle duct 560 may be moved to place the nozzle 570 at various positions in the inner space of the inner cabinet 40.

As described above, the shoe care device 1 according to this embodiment may effectively utilize the inner space of the inner cabinet 40 because the nozzle duct 560 is installed on one upper side of the inner cabinet 40.

In the shoe care device 1 according to an embodiment of the present disclosure, the spray holes 571 may be formed in the opposite directions at the end of the nozzle 570. That is, steam and air may be sprayed from the end of the nozzle 570 in the opposite directions.

In order for the steam and air to be effectively sprayed into the shoe, the steam and air need to be sprayed toward the front face of the shoe. However, as described above, if the direction in which the nozzle 570 is inserted into the shoe varies as needed, the steam and air are likely to be sprayed to the faces of the shoe, other than the front face, depending on the direction of the nozzle 570 inserted into the shoe.

For example, the spray holes 571 are formed to face only the front face of the shoe in the state shown in FIG. 33, whereas the spray holes 571 face the rear face of the shoe according to the nozzle 570 inserted in the opposite direction in the state shown in FIG. 34.

Therefore, in order to ensure that the steam and air are consistently sprayed toward the front face of the shoe even if the direction of the nozzle 570 inserted into the shoe is changed, the steam and air are required to be sprayed in the opposite directions from the end of the nozzle 570.

As described above, since the shoe care device 1 according to this embodiment performs spraying in the opposite directions from the end of the nozzle 570, the spray to the shoe may be effectively performed, regardless of the direction of the nozzle 570 inserted into the shoe.

In the shoe care device 1 according to an embodiment of the present disclosure, a pair of nozzles 570 may be installed to branch off from the nozzle duct 560.

Considering that shoes are generally configured by a pair of shoes and that the pair of shoes is exposed to relatively the same environment when used, the pair of shoes may require substantially the same treatment.

Therefore, in order to ensure relatively uniform treatment on a pair of shoes, a pair of nozzles 570 may be installed to branch off from one nozzle duct 560.

As described above, in the shoe care device 1 according to this embodiment, since a pair of nozzles 570 is installed to branch off from the nozzle duct 560, a pair of shoes may be simultaneously treated.

The shoe care device 1 according to an embodiment of the present disclosure may further include a distribution housing 540 that constitutes a portion of the connecting passage F10 and is disposed to extend along one direction from the connecting passage F10 to form a passage for steam and air, and a plurality of nozzle ducts 560 may be installed to branch off from the distribution housing 540.

Through this, the nozzles 570 connected to the nozzle duct 560 may be disposed to branch off from the distribution housing 540.

In order to process multiple shoes in the inner cabinet 40, a plurality of nozzle ducts 560 needs to be provided. It may be structurally very complex and inefficient to form a connecting passage F10 for each of the plurality of nozzle ducts 560 described above.

Therefore, it may be desirable to install a plurality of nozzle ducts 560 that branch off from one connecting passage F10.

However, if a plurality of nozzle ducts 560 is simply installed to branch, the amount of steam and air distributed may vary depending on the position of each nozzle duct 560 on the connecting passage F10.

In this case, processing performance may vary for each nozzle duct 560, which may cause inconvenience to the user, such as difficulty in predicting. In addition, the shoe care device 1 may have load concentrated on a specific part, causing functional and structural problems.

Accordingly, it is desirable to install a distribution housing 540 extending in one direction on the connecting passage F10 so as to serve as a buffer space before steam and air are supplied to the respective nozzle ducts 560.

That is, the steam and air may flow into the distribution housing 540, be sufficiently dispersed inside the distribution housing 540, and then be supplied to the respective nozzle ducts 560. Accordingly, the steam and air may be supplied uniformly to the respective nozzle ducts 560 and the nozzles 570 coupled thereto, regardless of positions where they are disposed.

As described above, since the shoe care device 1 according to the present embodiment has a plurality of nozzles 570 disposed to branch off from the distribution housing 540 disposed to extend in one direction on the connecting passage F10, it is possible to distribute a relatively uniform amount of steam or air to the respective nozzles 570.

In the shoe care device 1 according to an embodiment of the present disclosure, the distribution housing 540 may be disposed on one outer upper side of the inner cabinet 40.

As described above, in order for the nozzle duct 560 to protrude from one upper side of the inner cabinet 40, the distribution housing 540 to which the nozzle duct 560 is coupled may be disposed on one upper side of the inner cabinet 40.

However, since the distribution housing 540 is intended to evenly distribute steam and air to the respective nozzle ducts 560, the user does not need to directly operate the distribution housing 540, so it is not required to be exposed to the user.

On the other hand, if the distribution housing 540 is exposed to the user inside the inner cabinet 40, the structure inside the inner cabinet 40 may become complicated and may be undesirable in terms of good-looking appearance.

Therefore, the distribution housing 540 is preferably disposed on one upper side of the inner cabinet 40 between the inner cabinet 40 and the outer cabinet 20, which corresponds to the outside of the inner cabinet 40.

As described above, in the shoe care device 1 according to this embodiment, the distribution housing 540 may be disposed on one outer upper side of the inner cabinet 40, thereby effectively utilizing the inner space of the inner cabinet 40.

The shoe care device 1 according to an embodiment of the present disclosure may further include a dry air duct 530 that forms a portion of the connecting passage F10 and is connected to a part of the distribution housing 540 to guide the air passing through the dehumidifying material 430 to the distribution housing 540.

That is, the dry air duct 530 may have one end connected to the dehumidifying material 430 and the other end connected to the distribution housing 540 so as to form a passage through which the air passing through the dehumidifying material 430 moves to the distribution housing 540.

As described above, since the shoe care device 1 according to the present embodiment further includes the dry air duct 530 coupled to a part of the distribution housing 540 on the connecting passage F10, the air passing through the dehumidifying material 430 may move stably and effectively to the distribution housing 540.

In the shoe care device 1 according to an embodiment of the present disclosure, the dry air duct 530 may be disposed on the outer side of the inner cabinet 40 so as to extend along the longitudinal direction.

Since dry air and steam supplied to the accommodation space 41 of the inner cabinet 40 tend to rise, it is preferable that the machine room 50 is located at the lower portion of the inner cabinet 40.

In this case, since the air must be dehumidified through the air supplier 10 disposed inside the machine room 50, the dehumidifying material 430 also needs to be disposed inside the machine room 50.

In addition, since the distribution housing 540 is preferably disposed on one upper side of the inner cabinet 40 as described above, the dry air duct 530 needs to be disposed to connect the lower portion and upper portion of the inner cabinet 40.

Accordingly, the dry air duct 530 may be installed to extend in the longitudinal direction so as to connect the portion where the dehumidifying material 430 is installed and the distribution housing 540.

However, since the dry air duct 530 is merely a movement passage for air, the user does not need to directly operate the dry air duct 530, and thus it does not need to be exposed to the user.

On the other hand, if the dry air duct 530 is exposed to the user inside the inner cabinet 40, the structure inside the inner cabinet 40 may become complicated and may be undesirable in terms of good-looking appearance.

Therefore, it is desirable to dispose the dry air duct 530 in the longitudinal direction between the inner cabinet 40 and the outer cabinet 20, which corresponds to the outside of the inner cabinet 40.

As described above, in the shoe care device 1 according to this embodiment, the dry air duct 530 may be disposed on the outer side of the inner cabinet 40 in the longitudinal direction, thereby effectively utilizing the inner space of the inner cabinet 40 and stably and effectively moving the air to the distribution housing 540 located in the upper portion of the inner cabinet 40.

In the shoe care device 1 according to an embodiment of the present disclosure, a steam inlet 541 may be formed in another portion of the distribution housing 540, and the steam generator 600 may be connected to the steam inlet 541.

It is desirable that the steam supplied from the steam generator 600 is formed separately from the connecting passage F10 and that the steam and air are mixed inside the distribution housing 540 and then moved to the nozzle duct 560 and nozzle 570.

If steam flows into the connecting passage F10, for example, into the dry air duct 530, prior to the distribution housing 540, there is a risk that the dehumidifying performance of the shoe care device 1 deteriorates.

Accordingly, it is preferable that the portion of the distribution housing 540 where the dry air duct 530 is coupled is separately formed to be spaced apart from the steam inlet 541 into which steam flows.

As described above, in the shoe care device 1 according to this embodiment, since the steam generator 600 is connected to the steam inlet 541 formed in another portion of the distribution housing 540, steam and air may effectively move to the distribution housing 540 without interfering with each other.

The shoe care device 1 according to an embodiment of the present disclosure may further include a steam separator 550 installed on the steam inlet 541 to prevent condensate water in the steam from flowing into the distribution housing 540.

Although most of the steam supplied from the steam generator 600 is in a gaseous state, condensate water in a liquid state may be generated due to condensation thereof during the movement.

If the condensate water in the steam is supplied to the nozzle 570, the drying efficiency of the shoes may deteriorate. Additionally, if the condensate water in the steam flows into the distribution housing 540, a separate configuration for discharging the condensate water must be provided in the distribution housing 540, which makes the structure complicated.

Therefore, it is desirable to remove the condensate water from the steam through the steam separator 550 installed in the steam inlet 541 before the condensate water in the steam flows into the distribution housing 540.

As described above, since the shoe care device 1 according to this embodiment prevents condensate water in steam from flowing into the distribution housing 540 through the steam separator 550, it is possible to prevent residual water inside the distribution housing 540 and minimize movement of the residual water to the nozzle.

In the shoe care device 1 according to an embodiment of the present disclosure, the steam separator 550 may include a separating base 551, a separating inlet 552, a first stopper 553, and a separating outlet 554.

Specifically, the separating base 551 may be formed larger than the steam inlet 541 and disposed on one side of the distribution housing 540 where the steam inlet 541 is formed so as to cover the steam inlet 541, constituting the primary appearance of the steam separator 550.

Accordingly, the steam supplied from the steam generator 600 may flow into the separating base 551 and then move into the distribution housing 540 through the steam inlet 541.

The separating inlet 552 may be formed on the side of the separating base 551 such that steam flows into the same, and the steam generator 600 may be connected to the separating inlet 552.

Accordingly, the steam supplied from the steam generator 600 may flow into the separating base 551 through the separating inlet 552. In this case, since the steam is supplied from a separate supply device, the steam flowing in through the separating inlet 552 may move linearly to a certain extent in the travelling direction.

The first stopper 553 may be formed to extend in the longitudinal direction inside the separating base 551 so as to block the side portion of the steam inlet 541 adjacent to the separating inlet 552, and may be formed on the path of steam flowing in through the separating inlet 552.

Accordingly, the steam flowing in through the separating inlet 552 may collide with the first stopper 553, instead of moving straight to the steam inlet 541, and make a detour to the lower portion of the separating base 551.

During this process, the condensate water in the steam may fall onto the lower portion of the separating base 551 by the first stopper 553, so that the condensate water may be initially separated from the steam.

The separating outlet 554 may be formed at the lower portion of the separating inlet 552 on the side of the separating base 551, and may be a passage through which the condensate water falling onto the lower portion of the separating base 551 is discharged.

That is, the steam that collides with the first stopper 553 as described above may make a detour to the lower portion of the first stopper 553 and then move to the steam inlet 541, and the condensate water in the steam may fall onto the lower portion of the separating base 551 to be discharged through the separating outlet 554.

In this case, the separating outlet 554 may be connected to the sump 250 or the like, so that the condensate water may be treated separately.

As described above, in the shoe care device 1 according to this embodiment, since the steam separator 550 includes the separating base 551, the separating inlet 552, the first stopper 553, and the separating outlet 554, the condensate water in steam may be prevented from flowing into the steam inlet 541 when it is supplied.

In the shoe care device 1 according to an embodiment of the present disclosure, the steam separator 550 may further include a second stopper 555 formed to extend in the transverse direction inside the separating base 551 so as to block the lower portion of the steam inlet 541.

As described above, the steam that collides with the first stopper 553 and takes a detour to the lower portion of the first stopper 553 and then moves to the steam inlet 541 may still have condensate water of a liquid element.

Therefore, it is necessary to secondarily separate the condensate water from the steam by causing the steam to re-collide with the second stopper 555.

In particular, condensate water in the steam that collides with the first stopper 553 may fall onto the lower portion of the separating base 551. During this process, some condensate water may scatter and flow into the steam inlet 541, so the second stopper 555 may be formed at the lower portion of the steam inlet 541 to block the scattering condensate water.

As described above, in the shoe care device 1 according to this embodiment, since the steam separator 550 further includes the second stopper 555, condensate water in steam may be prevented from scattering inside the steam separator 550 and flowing into the steam inlet 541.

In the shoe care device 1 according to an embodiment of the present disclosure, the bottom surface of the separating base 551 may be formed to be inclined downwards in the direction in which the separating outlet 554 is formed.

As described above, the condensate water in the steam must fall onto the lower portion of the separating base 551 and then be discharged through the separating outlet 554, so it is necessary to ensure that the condensate water is collected toward the separating outlet 554.

If the collected condensate water remains inside the steam separator 550, instead of being effectively discharged, it is not desirable because there is a risk of contamination and bacterial growth.

Therefore, it is necessary to form a slope on the bottom surface of the separating base 551 so that the dropped condensate water moves smoothly toward the separating outlet 554.

As described above, in the shoe care device 1 according to this embodiment, since the bottom surface of the separating base 551 is formed to be inclined, it is possible to prevent residual water inside the steam separator 550.

The shoe care device 1 according to an embodiment of the present disclosure may further include a heater 710 installed to heat the dehumidifying material 430 and a regeneration passage F20 through which the air passing through the dehumidifying material 430 while the dehumidifying material 430 is heated is blown to a portion other than the nozzle 570.

The regeneration passage F20 may form a passage that branches off from the connecting passage F10 and allows the air passing through the dehumidifying material 430 and/or the condensate water to move therethrough.

That is, the regeneration passages F20 may be a path through which air moves in the process of heating and regenerating the dehumidifying material 430 when the dehumidifying function is not effectively performed due to excessive moisture adsorbed onto the dehumidifying material 430.

Dehumidification of the air is not performed while the dehumidifying material 430 is being regenerated. On the contrary, moisture separated from the dehumidifying material 430 in the regeneration process is contained in the air passing through the dehumidifying material 430, resulting in higher humidity.

Therefore, it is inappropriate to supply air with high humidity back to the inner cabinet 40, so it is necessary to blow the air to the regeneration passages F20 separated from the connecting passage F10.

As described above, the shoe care device 1 according to this embodiment may heat the dehumidifying material 430 by the heater 710, and the regeneration passage F20 may be formed for the air generated according thereto to move, thereby preventing air from moving to the inner cabinet 40 in the regeneration mode.

The shoe care device 1 according to an embodiment of the present disclosure may further include a dehumidifying-material housing 300 disposed on the connecting passage F10 and having the dehumidifying material 430 accommodated therein and the heater 710 installed therein.

Specifically, the inner space of the dehumidifying-material housing 300 may configure a portion of the connecting passage F10, and the dehumidifying material 430 may be located inside the dehumidifying-material housing 300. In this case, the heater 710 may be disposed in the connecting passage F10 to be adjacent to the dehumidifying material 430, thereby heating the dehumidifying material 430.

As described above, since the shoe care device 1 according to the present embodiment further includes the dehumidifying-material housing 300 in which the dehumidifying material 430 is accommodated, the dehumidifying material 430 may be stably accommodated to appropriately execute a function thereof.

In this case, the dehumidifying material 430 may be disposed in a shape extending in the first direction X on the plane of the dehumidifying-material housing 300, and the air may be blown in the first direction X on the connecting passage F10.

Specifically, the dehumidifying material 430 may be accommodated in the dehumidifying-material housing 300. That is, the dehumidifying-material housing 300 may be installed in the machine room 50, and the dehumidifying material 430 may be accommodated in the dehumidifying-material housing 300, thereby executing dehumidification and regeneration functions.

One side of the dehumidifying-material housing 300 accommodating the dehumidifying material 430 may be connected to the blowing duct 230 to supply air to the dehumidifying material 430.

In addition, the air supplied to the inside of the dehumidifying-material housing 300 may come into contact with the dehumidifying material 430, and may then be blown toward the connecting passage F10 or the regeneration passages F20. To this end, the opposite side of the dehumidifying-material housing 300 may be connected to the damper housing 520 so that the air passing through the inside may be blown toward the damper housing 520.

In particular, it is desirable in terms of dehumidifying efficiency to allow the air to come into contact and collide with the dehumidifying material 430 on the longest possible path. Accordingly, it is preferable to blow the air along the extended longitudinal direction of the dehumidifying material 430.

In the shoe care device 1 according to an embodiment of the present disclosure, the external space of the dehumidifying material 430 may form the outer passage F12, which is a portion of the connecting passage F10, while accommodated in the dehumidifying-material housing 300, and the dehumidifying-material housing 300 may be formed such that the cross-sectional area of the outer passage F12 increases as it is closer to the front in the first direction X.

Specifically, the air flowing into the dehumidifying-material housing 300 needs to pass through the dehumidifying material 430 and then move smoothly to the damper housing 520 and the dry air duct 530.

To this end, since air must move effectively in the first direction X on the plane inside the dehumidifying-material housing 300, it is preferable that flow resistance does not increase as it is closer to the front in the first direction X.

Therefore, the cross-sectional area of the outer passage F12 of the dehumidifying material 430 may increase as it is closer to the front in the first direction X inside the dehumidifying-material housing 300, thereby preventing flow resistance from increasing.

As described above, in the shoe care device 1 according to the present embodiment, since the cross-sectional area of the outer passage F12 of the dehumidifying material 430 accommodated in the dehumidifying-material housing 300 is configured to increase toward the front, it is possible to prevent the flow resistance inside the dehumidifying-material housing 300 from increasing.

In the shoe care device 1 according to an embodiment of the present disclosure, the dehumidifying-material housing 300 may be formed such that the width of the outer passage F12 increases as it is closer to the front in the first direction X.

That is, inside the dehumidifying-material housing 300, the distance between the left dehumidifying-material wall 340 and the right dehumidifying-material wall 350 increases toward the front in the first direction X.

As described above, in the shoe care device 1 according to the present embodiment, since the width of the outer passage F12 of the dehumidifying material 430 accommodated in the dehumidifying-material housing 300 is configured to increase toward the front, it is possible to prevent the flow resistance from increasing in the width direction inside the dehumidifying-material housing 300.

In the shoe care device 1 according to an embodiment of the present disclosure, the dehumidifying-material housing 300 may be formed such that the height of the outer passage F12 increases as it is closer to the front in the first direction X.

That is, inside the dehumidifying-material housing 300, the distance between the housing bottom plate 310 and the dehumidifying-material cover 47 increases toward the front in the first direction X.

As described above, in the shoe care device 1 according to the present embodiment, since the height of the outer passage F12 of the dehumidifying material 430 accommodated in the dehumidifying-material housing 300 is configured to increase toward the front, it is possible to prevent the flow resistance from increasing in the height direction inside the dehumidifying-material housing 300.

In the shoe care device 1 according to an embodiment of the present disclosure, the dehumidifying-material housing 300 may be disposed at the lower portion of the inner cabinet 40, and the bottom of the inner cabinet 40 may be formed to be inclined corresponding to the height of the outer passage F12 formed in the dehumidifying-material housing 300.

In the process of using the shoe care device 1, condensate water may also be generated inside the inner cabinet 40, and this condensate water may flow down along the inner wall surfaces of the inner cabinet 40 to gather on the bottom surface of the inner cabinet 40.

If the condensate water gathering above leaks to the outside of the inner cabinet 40, the user may feel inconvenience, causing problems in usability.

In particular, since the door 30 is installed in the front portion of the inner cabinet 40, it is necessary to prevent leakage of condensate water through the gap of the door 30.

Therefore, the inner cabinet 40 needs to have an inclined bottom, and it is desirable for the inlet 42 to be disposed at the lowest portion of the inclined bottom.

In addition, the outer passage F12 formed in the dehumidifying-material housing 300 may be configured to have a height corresponding to the bottom of the inclined inner cabinet 40 as described above, thereby preventing unnecessary wasting of the space in the machine room 50.

As described above, in the shoe care device 1 according to the present embodiment, since the inclined surface of the bottom of the inner cabinet 40 is configured to correspond to the height of the outer passage F12 of the dehumidifying material 430 accommodated in the dehumidifying-material housing 300, the dehumidifying-material housing 300 may be efficiently disposed in the lower portion of the inner cabinet 40.

In the shoe care device 1 according to an embodiment of the present disclosure, the dehumidifying-material housing 300 may include a housing inlet 311, formed at the rear in the first direction X, into which the air headed for the dehumidifying material 430 flows and a housing outlet 331, formed at the front in the first direction X, from which the air passing through the dehumidifying material 430 is discharged.

In order to allow the air passing through the interior of the dehumidifying-material housing 300 to more frequently come into contact and collide with the dehumidifying material 430, it is necessary to maximize the air movement path inside the dehumidifying-material housing 300.

Accordingly, the housing inlet 311 and the housing outlet 331 are preferably disposed on the opposite sides on the plane of the dehumidifying-material housing 300.

As described above, in the shoe care device 1 according to the present embodiment, since the dehumidifying-material housing 300 includes the housing inlet 311 and the housing outlet 331, the air may stably and effectively pass through the interior of the dehumidifying-material housing 300 accommodating the dehumidifying material 430.

In the shoe care device 1 according to an embodiment of the present disclosure, the dehumidifying-material housing 300 may further include a housing bottom plate 310 configuring the bottom surface, a rear dehumidifying-material wall 320 configuring the rear surface in the first direction X, a front dehumidifying-material wall 330 configuring the front surface in the first direction X, and left dehumidifying-material walls 340a and 340b and right dehumidifying-material walls 350a and 350b configuring side surfaces connected between the rear dehumidifying-material wall 320 and the front dehumidifying-material wall 330.

The rear dehumidifying-material wall 320, the front dehumidifying-material wall 330, the left dehumidifying-material walls 340a and 340b, and the right dehumidifying-material walls 350a and 350b may configure wall surfaces erected in the vertical direction, respectively. Based on the first direction X, the rear dehumidifying-material wall 320 may configure a rear wall surface, the front dehumidifying-material wall 330 may configure a front wall surface, the left dehumidifying-material walls 340a and 340b may configure left wall surfaces, and the right dehumidifying-material walls 350a and 350b may configure right wall surfaces in the dehumidifying-material housing 300.

As described above, in the shoe care device 1 according to the present embodiment, since the dehumidifying-material housing 300 further includes the housing bottom plate 310, the rear dehumidifying-material wall 320, the front dehumidifying-material wall 330, the left dehumidifying-material walls 340a and 340b, and the right dehumidifying-material walls 350a and 350b, the space for accommodating the dehumidifying materials 430 may be stably partitioned.

In the shoe care device 1 according to an embodiment of the present disclosure, the dehumidifying-material housing 300 may further include first guide protrusions 360 that protrude inward from the inner surfaces of the left dehumidifying-material walls 340a and 340b and the inner surfaces of the right dehumidifying-material walls 350a and 350b to support the dehumidifying material 430.

As the first guide protrusions 360 are formed, predetermined gaps are stably formed between the left dehumidifying-material walls 340a and 340b and the first side walls 402, and predetermined gaps are stably formed between the right dehumidifying-material walls 350a and 350b and the second side walls 403. Accordingly, the dehumidifying-material block 400 is prevented from moving in the horizontal direction inside the dehumidifying-material housing 300 and the outer passage F12 is stably maintained.

As described above, since the shoe care device 1 according to the present embodiment supports the dehumidifying material 430 through the first guide protrusion 360 formed on the left dehumidifying-material walls 340a and 340b and the right dehumidifying-material walls 350a and 350b, the dehumidifying material 430 may be accommodated more stably inside the dehumidifying-material housing 300.

In the shoe care device 1 according to an embodiment of the present disclosure, the housing inlet 311 may be formed on the housing bottom plate 310, and the housing outlet 331 may be formed on the front dehumidifying-material wall 330.

It is preferable that the dehumidifying material 430 and the dehumidifying-material housing 300 are disposed at the uppermost portion of the machine room 50, and the connection of the blowing duct 230 with the dehumidifying-material housing 300 may facilitate blowing and minimize interference between members.

Accordingly, the housing inlet 311 may be formed on the lower surface of one side of the dehumidifying-material housing 300.

In addition, in the case where the air passing through the dehumidifying-material housing 300 is immediately introduced into the inner cabinet 50, it is impossible to selectively blow the air to the connecting passage F10 or the regeneration passages F20, so the housing outlet 331 is required to be formed on the side surface of the dehumidifying-material housing 300, instead of on the upper surface thereof.

Accordingly, the housing outlet 331 may be formed on the side surface opposite the portion where the housing inlet 311 is formed on the plane.

As described above, in the shoe care device 1 according to the present embodiment, since the housing inlet 311 is formed on the housing bottom plate 310 and since the housing outlet 331 is formed on the front dehumidifying-material wall 330, it is possible to effectively blow the air and minimize interference between members.

In the shoe care device 1 according to an embodiment of the present disclosure, the heater 710 may include a fixed end 712 installed on the rear dehumidifying-material wall 320 and a free end 711 that extends in the first direction X from the fixed end 712.

In this case, the fixed end 712 may be electrically connected to a power source, and the free end 711 may generate heat when electric energy is supplied to the free end 711 through the fixed end 712.

As described above, in the shoe care device 1 according to the present embodiment, since the heater 710 includes the fixed end 712 and the free end 711, the heater 710 may be effectively installed in the dehumidifying-material housing 300.

The shoe care device 1 according to an embodiment of the present disclosure may be configured to include an inner cabinet 40, an inlet 42, a nozzle 570, a heater 710, a blowing fan 221, a dehumidifying-material block 400, and a dehumidifying-material housing 300.

The dehumidifying-material block 400 has an inner space configured to accommodate the heater 710, includes the dehumidifying material 430 surrounding the heater 710, and is installed on the connecting passage F10 to dehumidify the blown air.

In this case, the dehumidifying-material housing 300 may include a dehumidifying-material fixing groove 313 formed on the bottom surface such that the lower end of the dehumidifying-material block 400 is inserted thereto.

That is, the dehumidifying-material housing 300 may have a dehumidifying-material fixing groove 313 formed on the bottom surface so as to correspond to the shape of the lower end of the dehumidifying-material block 400, and the lower end of the dehumidifying-material block 400 may be inserted into the dehumidifying-material fixing groove 313.

Accordingly, the lower end of the dehumidifying-material block 400 may be more stably supported on the bottom surface of the dehumidifying-material housing 300. In addition, airtightness may be improved between the lower end of the dehumidifying-material block 400 and the bottom surface of the dehumidifying-material housing 300, thereby preventing the air flowing into the inner space of the dehumidifying-material block 400 from escaping through the lower end of the dehumidifying-material block 400.

As described above, in the shoe care device 1 according to the present embodiment, since the dehumidifying-material fixing groove 313 is formed on the bottom surface of the dehumidifying-material housing 300, and since the lower end of the dehumidifying-material block 400 is inserted into the dehumidifying-material fixing groove 313, the dehumidifying material 430 may be stably accommodated and may appropriately execute its function.

In the shoe care device 1 according to an embodiment of the present disclosure, the inner space of the dehumidifying-material block 400 may form an inner passage F11, which is a portion of the connecting passage F10, and the dehumidifying-material housing 300 may further include a front fixed plate 315 formed to block the front of the inner passage F11.

In order to improve the dehumidifying efficiency of air by the dehumidifying material 430, the air flowing into the dehumidifying-material housing 300 needs to further come into contact and collide with the dehumidifying material 430.

However, if the air flowing into the inner space of the dehumidifying-material block 400 moves in the first direction X as it goes, the frequency at which the air comes into contact and collides with the dehumidifying material 430 may be greatly reduced.

Therefore, it is desirable to block the front of the inner passage F11 using the front fixed plate 315 to prevent the air flowing into the inner space of the dehumidifying-material block 400 from escaping in the first direction X without any interference.

As described above, in the shoe care device 1 according to the present embodiment, since the front fixed plate 315 formed in the dehumidifying-material housing 300 blocks the front of the inner passage F11 of the dehumidifying-material block 400 accommodated in the dehumidifying-material housing 300, the air in the dehumidifying-material housing 300 may be prevented from moving only along the inner passage F11.

In the shoe care device 1 according to an embodiment of the present disclosure, the dehumidifying-material block 400 may be configured in the form of a pipe or tunnel formed in the horizontal direction, and the air flowing into the inner passage F11 may flow by passing through the dehumidifying-material block 400 from the inside to the outside.

The inner passage F11 may be formed inside the dehumidifying-material block 400, and the inner passage F11 may be formed long in the first direction X, so that the air moving along the inner passage F11 may move in the direction to pass through the dehumidifying-material block 400 (the direction crossing the first direction X), and accordingly, the contact area between the air of the connecting passage F10 and the grains of each dehumidifying material 430 may become sufficiently large, thereby increasing the dehumidifying efficiency.

As described above, in the shoe care device 1 according to the present embodiment, since the air is able to flow by passing through the dehumidifying-material block 400, which is configured in the form of a tube or tunnel, from the inside to the outside, the air may more frequently come into contact with the dehumidifying material 430, thereby improving dehumidification efficiency.

In the shoe care device 1 according to an embodiment of the present disclosure, the dehumidifying-material block 400 may be accommodated in the dehumidifying-material housing 300 such that the rear surface thereof is in close contact with the rear dehumidifying-material wall 320.

As described above, even in the case where the front of the inner passage F11 is blocked by the front fixed plate 315 to prevent the air flowing into the inner space of the dehumidifying-material block 400 from escaping in the first direction X without any interference, if the air escapes to the rear of the inner passage F11, dehumidification efficiency may still deteriorate.

Therefore, it is desirable to keep the rear surface of the dehumidifying-material block 400 in close contact with the rear dehumidifying-material wall 320 to prevent air from escaping to the rear of the inner passage F11.

As described above, in the shoe care device 1 according to the present embodiment, since the rear surface of the dehumidifying-material block 400 accommodated in the dehumidifying-material housing 300 is in close contact with the rear dehumidifying-material wall 320, the air introduced into the inner passage F11 may be forced to flow through the dehumidifying-material block 400.

In the shoe care device 1 according to an embodiment of the present disclosure, a pair of dehumidifying materials 430 may be disposed to be separate on the branching connecting passages F10, and the heater 710 may be installed to heat each of the pair of dehumidifying materials 430.

That is, since a pair of dehumidifying materials 430 is disposed inside the shoe care device 1 and since the connecting passage F10 and the regeneration passage F20 are provided in each dehumidifying material 430, the shoe care device 1 may be operated in an optimal state depending on the situation, thereby further improving the efficiency of processing the shoes.

In addition, in the case where a pair of dehumidifying materials 430 is disposed on the connecting passage F10, a pair of left dehumidifying-material walls 340a and 340b and a pair of right dehumidifying-material walls 350a and 350b may be formed, in which any one dehumidifying material 430 may be accommodated between a first left dehumidifying-material wall 340a and a first right dehumidifying-material wall 350a and in which the remaining dehumidifying material 430 may be accommodated between a second left dehumidifying-material wall 340b and a second right dehumidifying-material wall 350b.

In this case, the shoe care device 1 according to an embodiment of the present disclosure may further include heaters 710 that are respectively installed between the first left dehumidifying-material wall 340a and the first right dehumidifying-material wall 350a and between the second left dehumidifying-material wall 340b and the second right dehumidifying-material wall 350b, respectively, to heat the pair of dehumidifying material 430.

In the shoe care device 1 according to an embodiment of the present disclosure, a first rotation-angle adjusting unit 561 may be formed at the coupling portion between the nozzle duct 560 and the inner cabinet 40, which is relatively rotatable by cogwheel coupling. In this case, the first rotation-angle adjusting unit 561 may be formed at a portion where the nozzle duct 560 and the distribution housing 540 are coupled.

Specifically, the first rotation-angle adjusting unit 561 may include an inner plate having cogs formed on the outer circumferential surface, and an outer plate surrounding the outer circumferential surface of the inner plate and having cogs formed on the inner circumferential surface to mesh with the cogs of the inner plate.

Accordingly, the inner plate and the outer plate may be supported in the state of meshing with each other, so that the nozzle duct 560 may remain at a predetermined angle with respect to the inner cabinet 40.

In addition, if an external force greater than the pressure at which the inner plate and the outer plate mesh with each other is applied, the inner plate and the outer plate may rotate relative to each other so that the angle of the nozzle duct 560 with respect to the inner cabinet 40 may vary.

As described above, since the shoe care device 1 according to the present embodiment adjusts the angle of the nozzle duct 560 protruding into the inner cabinet 40 by the first rotation-angle adjusting unit 561, the rotation angle of the nozzle duct 560 with respect to the inner cabinet 40 may be adjusted more precisely and stably.

In the shoe care device 1 according to an embodiment of the present disclosure, a second rotation-angle adjusting unit 562 may be formed at the coupling portion between the nozzle duct 560 and the nozzle 570, which is relatively rotatable by cogwheel coupling therebetween.

Specifically, the second rotation-angle adjusting unit 562 may also include an inner plate having cogs formed on the outer circumferential surface, and an outer plate surrounding the outer circumferential surface of the inner plate and having cogs formed on the inner circumferential surface to mesh with the cogs of the inner plate.

Accordingly, the inner plate and the outer plate may be supported in the state of meshing with each other, so that the nozzle 570 may remain at a predetermined angle with respect to the nozzle duct 560.

In addition, if an external force greater than the pressure at which the inner plate and the outer plate mesh with each other is applied, the inner plate and the outer plate may rotate relative to each other so that the angle of the nozzle 570 with respect to the nozzle duct 560 may vary.

As described above, since the shoe care device 1 according to the present embodiment adjusts the angle of the nozzle 570 coupled to the nozzle duct 560 by the second rotation-angle adjusting unit 562, the rotation angle of the nozzle 570 with respect to the nozzle duct 560 may be adjusted more precisely and stably.

The shoe care device 1 according to an embodiment of the present disclosure may further include a holder 90 installed in the upper portion of the inner cabinet 40 and capable of holding the nozzle duct 560.

As described above, in the case where the nozzle duct 560 is installed to protrude from one upper side of the inner cabinet 40, the nozzle duct 560 may be placed in the upper portion of the inner cabinet 40 when the nozzle 570 is not used, thereby avoiding occupying the inner space of the inner cabinet 40.

In this case, the nozzle duct 560 may be maintained to be positioned in the upper portion of the inner cabinet 40 by the first rotation-angle adjusting unit 561.

However, if an unintended external force is applied to the nozzle duct 560 or if the performance of the first rotation-angle adjusting unit 561 deteriorates due to long-term use, the nozzle duct 560 may sag downwards even when the nozzle 570 is not used.

Therefore, when the nozzle 570 is not used, it is desirable to hold the nozzle duct 560 by the holder 90 installed on the upper portion of the inner cabinet 40.

As described above, since the shoe care device 1 according to this embodiment holds the nozzle duct 560 using the holder 90 installed on the upper portion of the inner cabinet 40, it is possible to prevent the nozzle duct 560 from unnecessarily occupying the inner space of the inner cabinet 40.

In the shoe care device 1 according to an embodiment of the present disclosure, the holder 90 may include a holding base 91 disposed on the upper portion of the inner cabinet 40, and a holding wall 92 protruding downwards from the holding base 91 and having a holding protrusion 93 formed in a portion thereof.

In this case, a holding groove 563 corresponding to the holding protrusion 93 may be formed on the side of the nozzle duct 560.

As described above, when holding the nozzle duct 560 on the holder 90, the holding protrusion 93 may be engaged with the holding groove 563, thereby further improving the holding force.

In this case, a holding groove 563 may be formed on the holding wall 92, and a holding protrusion 93 may be formed on the side of the nozzle duct 560, thereby improving the holding force.

As described above, in the shoe care device 1 according to the present embodiment, since the holder 90 includes the holding base 91 and the holding wall 92, the nozzle duct 560 may be more stably held in the holder 90.

The shoe care device 1 according to an embodiment of the present disclosure may be configured to include an inner cabinet 40, a steam generator 600, an inlet 42, a nozzle 570, a connecting passage F10, a blowing fan 221, a dehumidifying material 430, a dry air duct 530, a distribution housing 540, and a backflow prevention flip 900.

The backflow prevention flip 900 may be installed in the dry air duct 530 to limit the inflow of steam from the distribution housing 540 to the dry air duct 530, and may perform a check-valve function in which the air is allowed to flow only in one direction, instead of in the opposite direction.

That is, the backflow prevention flip 900 may enable the flow of air from the dry air duct 530 to the distribution housing 540 while restricting the flow of air from the distribution housing 540 to the dry air duct 530.

As described above, if steam flows into the connecting passage F10, for example, into the dry air duct 530, prior to the distribution housing 540, there is a risk that the dehumidifying performance of the shoe care device 1 deteriorates. On the other hand, the air having moved to the dry air duct 530 needs to smoothly move to the distribution housing 540.

Therefore, it is desirable to install a backflow prevention flip 900 on the dry air duct 530 to limit the air flow from the distribution housing 540 to the dry air duct 530.

As described above, since the shoe care device 1 according to the present embodiment limits the inflow of steam from the distribution housing 540 to the dry air duct 530 by the backflow prevention flip 900 installed on the dry air duct 530, it is possible to prevent steam from flowing into unintended areas and deteriorating the performance of the shoe care device 1.

In the shoe care device 1 according to an embodiment of the present disclosure, the backflow prevention flip 900 may include a flip hinge shaft 910 that is hinge-coupled to one side of the inner surface of the dry air duct 530 and a flip plate 920 formed to extend from the flip hinge shaft 910 to cover the internal cross-section of the dry air duct 530.

Specifically, the backflow prevention flip 900 is required to cover the internal cross-section of the dry air duct 530 in order to restrict air flow inside the dry air duct 530.

Accordingly, the flip plate 920 may be installed inside the dry air duct 530 to cover the internal cross-section of the dry air duct 530. In this case, the flip plate 920 may be coupled to the flip hinge shaft 910 so as to be rotatable around a hinge.

Accordingly, as the flip plate 920 hinge-rotates in one direction to be disposed parallel to the internal cross-section of the dry air duct 530, the passing air flow may be limited, and as the flip plate 920 hinge-rotates in the opposite direction to intersect the inner cross-section of the dry air duct 530, the more air flow may pass through the same.

As described above, in the shoe care device 1 according to this embodiment, since the backflow prevention flip 900 includes a flip hinge shaft 910 and a flip plate 920, it is easy to open and close the internal cross-section of the dry air duct 530 through the hinge-rotation of the flip plate 920.

In the shoe care device 1 according to an embodiment of the present disclosure, the flip plate 920 may be disposed to be inclined upwards as it is far away from the flip hinge shaft 910. In this case, the planar area of the flip plate 920 may be greater than the internal cross-sectional area of the dry air duct 530.

As described above, the backflow prevention flip 900 must restrict the air flow from the distribution housing 540 to the dry air duct 530 while allowing the air flow from the dry air duct 530 to the distribution housing 540.

In this case, since the distribution housing 540 is disposed at a relatively high position, the backflow prevention flip 900 must allow air to move upwards inside the dry air duct 530 while limiting air to move downwards.

To this end, a separate limit structure may be needed to allow the flip plate 920 to hinge-rotate only upwards around the flip hinge shaft 910 and prevent the flip plate 920 from hinge-rotating downwards.

However, even without a separate limit structure, if the flip plate 920 is disposed to be inclined upwards as it is far away from the flip hinge shaft 910, the air flow moving upwards may presses the lower surface of the flip plate 920, so that the flip plate 920 may hinge-rotate.

On the other hand, even if the air flow moving downwards presses the upper surface of the flip plate 920, the flip plate 920 may hit the side wall of the dry air duct 530, so that hinge rotation thereof may be limited.

Therefore, even without a separate limit structure, the flip plate 920 disposed to be inclined may hinge-rotate only upwards around the flip hinge shaft 910 and may not hinge-rotate downwards.

As described above, in the shoe care device 1 according to this embodiment, since the flip plate 920 is disposed to be inclined, the upward air flow may be allowed, whereas the downward air flow may be effectively restricted.

The shoe care device 1 according to an embodiment of the present disclosure may further include a blowing duct 230 that forms a portion of the connecting passage F10, forms a portion of the suction duct 210 connected to the inlet 42 and the connecting passage F10, and forms a passage for air supplied to the dehumidifying material 430.

Specifically, the suction duct 210 may configure a portion of the connecting passage F10, and the suction duct 210 may be connected to the inlet 42 to suck air from the inner cabinet 40.

The blowing duct 230 may be connected to one side of the dehumidifying-material housing 300 accommodating the dehumidifying material 430 so that the air blown through the blowing duct 230 may come into contact with the dehumidifying material 430.

As described above, since the shoe care device 1 according to the present embodiment further includes the suction duct 210 and the blowing duct 230, the air may stably and effectively flow between the inlet 42 and the dehumidifying material 430.

The shoe care device 1 according to an embodiment of the present disclosure may further include a damper housing 520 that is connected between the dehumidifying-material housing 300 and the dry air duct 530 to guide the blown air.

The damper housing 520 may be coupled to the front side of the dehumidifying-material housing 300 in the first direction X.

Specifically, the damper housing 520 may be coupled to a housing outlet 331 formed in the dehumidifying-material housing 300 so that the air passing through the dehumidifying material 430 may be blown to the damper housing 520. In addition, the air may be blown to the connecting passage F10 or the regeneration passages F20 depending on the opening/closing direction of the damper 510 installed in the damper housing 520.

In this case, one side surface of the damper housing 520 coupled with the housing outlet 331 may be configured to be open.

In addition, in the case where the air introduced into the damper housing 520 flows through the connecting passage F10, the air must be blown into the inner cabinet 40 through the nozzle duct 560 and the nozzle 570, so the opposite side of the damper housing 520 may be connected to the dry air duct 530.

As described above, since the shoe care device 1 according to the present embodiment further includes the damper housing 520 installed between the dehumidifying-material housing 300 and the dry air duct 530, the air may move stably and effectively between the dehumidifying-material housing 300 and the dry air duct 530.

In the shoe care device 1 according to an embodiment of the present disclosure, the damper housing 520 may include a dry air outlet 523 formed to discharge the air passing through the dehumidifying material 430 toward the dry air duct 530 and a wet air outlet 524 formed to discharge the air passing through the dehumidifying material 430 in a direction other than the direction to the dry air duct 530.

Specifically, the dry air outlet 523 may be formed in the damper housing 520 to discharge the air passing through the dehumidifying material 430 toward the dry air duct 530, and may be formed in the damper housing 520 so as to be opened and closed by the damper 510.

The wet air outlet 524 may be formed in the damper housing 520 separately from the dry air outlet 523 to discharge the air passing through the dehumidifying material 430 in a direction other than the direction to the dry air duct 530, and may be formed in the damper housing 520 so as to be opened and closed by the damper 510.

As described above, in the shoe care device 1 according to the present embodiment, since the damper housing 520 includes the dry air outlet 523 and the wet air outlet 524, the connecting passage F10 and regeneration passages F20 may be properly separated based on the damper housing 520.

The shoe care device 1 according to an embodiment of the present disclosure may further include a damper 510 installed on the damper housing 520 to selectively open and close the dry air outlet 523 and the wet air outlet 524.

Specifically, the damper 510 may be installed on the air blowing path to selectively open and close the dry air outlet 523 and the wet air outlet 524, and may be installed in the damper housing 520.

In this case, the damper 510 may close the dry air outlet 523 and open the wet air outlet 524 when the dehumidifying material 430 is heated. That is, when the dehumidifying material 430 is heated for regeneration thereof, the air passing through the dehumidifying material 430 may be discharged together with the moisture separated from the dehumidifying material 430 through the wet air outlet 524.

As described above, since the shoe care device 1 according to this embodiment further includes the damper 510 installed in the damper housing 520, a dehumidifying mode and a regeneration mode may be selectively operated by controlling the damper 510.

In the foregoing, although specific embodiments of the present disclosure have been described and illustrated, the present disclosure is not limited to the described embodiments, and it will be understood by those of ordinary skill in the art that there may be various changes and modifications into other specific embodiments without departing from the spirit and scope of the present disclosure. Accordingly, the scope of the present disclosure should be defined by the technical idea described in the claims, instead of by the described embodiments.

### Reference Numerals

| | | | |
|---|---|---|---|
| 1: | Shoe care device | 10: | Air supplier |
| 20: | Outer cabinet | 30: | Door |
| 40: | Inner cabinet | 41: | Accommodation space |
| 42: | Inlet | 43: | Cabinet bottom plate |
| 44: | Inner back plate | 45: | Inner left plate |
| 46: | Inner right plate | 47: | Cabinet bottom plate |
| 48: | Rack | 50: | Machine room |
| 51: | First wall | 54: | Second wall |
| 55: | Third wall | 60: | Water supply tank |
| 61: | First water pump | 70: | Drain tank |
| 71: | Second water pump | 80: | Controller |
| 210: | Suction duct | 210a: | Vertical duct |
| 210b: | Horizontal duct | 211: | Central bottom surface |
| 212: | Blowing inlet | 213: | Condensate water drain |
| 220: | Blower | 221: | Blowing fan |
| 222: | Rotation axis | 225: | Blowing housing |
| 227: | Blowing fan motor | 230: | Blowing duct |
| 250: | Sump | 251: | First condensate inlet |
| 252: | Second condensate inlet | | |
| 300: | Dehumidifying-material housing | 310: | Housing bottom plate |
| 311: | Housing inlet | 320: | Rear dehumidifying-material wall |
| 321: | Rear hole | 330: | Front dehumidifying-material wall |
| 331: | Housing outlet | 340a and 340b: | Left dehumidifying-material wall |
| 350a and 350b: | Right dehumidifying-material wall | | |
| 400: | Dehumidifying-material block | 401: | Ceiling part |
| 402: | First side wall | 403: | Second side wall |
| 404: | Inner space of dehumidifying-material block | 410: | Inner mesh |
| 420: | Outer mesh | 430: | Dehumidifying material |
| 440: | First frame | 450: | Second frame |
| 460: | Third frame | 470: | Fourth frame |
| 510: | Damper | | |
| 520: | Damper housing | 521: | Central boundary damper wall |
| 522: | Regenerated-air parting wall | 523: | Dry air outlet |
| 524: | Wet air outlet | | |
| 530: | Dry air duct | 540: | Distribution housing |
| 550: | Steam separator | 560: | Nozzle duct |
| 570: | Nozzle | 600: | Steam generator |
| 710: | Heater | 711: | Free end |
| 712: | Fixed end | 720: | Heater flange |
| 800: | Condenser | 810: | Condenser housing |
| 820: | Condensation space | 830: | Condenser passage |
| 831: | Introduction section | 832: | Condensation section |
| 840: | Passage guide wall | 850: | Condenser inlet |
| 860: | Condenser outlet | | |
| F10: | Connecting passage | F10a: | First section |
| F10b: | Second section | F10c: | Third section |
| F11: | Inner passage | F12: | Outer passage |
| F20: | Regeneration passage | F21: | First discharge path |
| F22: | Second discharge path | X: | First direction |
| Y: | Second direction | Z: | Third direction |

### INDUSTRIAL APPLICABILITY

In some examples, since the dehumidifying material is disposed in the air supplier to capture moisture and bacteria in the blown air and since the air supplier heats the dehumidifying material to be regenerated, it can be possible to always maintain appropriate performance of processing shoes.

In addition, since the connection path through which air circulates is formed between the inlet and the outlet formed inside the inner cabinet, it can possible to help to prevent the user from being exposed to the air used in dehumidification and deodorization of shoes.

In addition, according to at least one of the embodiments of the present disclosure, since the nozzle is installed to be inserted into the shoe so as to spray at least one of steam and air into the shoe, and since the insertion direction of the nozzle is adjustable, the shoe care device can be operated to adjust the receiving direction of shoes as needed, thereby further improving the efficiency of processing shoes.

In addition, according to at least one of the embodiments of the present disclosure, since the nozzle is inserted into the shoe from above and sprays both steam and air into the shoe for treatment, steam and dry air can be effectively injected into the shoe when general refreshing is required for the shoe.

In addition, according to at least one of the embodiments of the present disclosure, since the nozzle is inserted into the shoe from the bottom and sprays only air into the shoe for treatment, only the dry air can be injected into the shoe when drying the shoe is more necessary, thereby effectively discharging moisture inside the shoe.

In addition, according to at least one of the embodiments in the present disclosure, since the nozzle is hinge-coupled to the nozzle duct installed to protrude to the inside of the inner cabinet on the connecting passage, the nozzle can be hinge-rotated relative to the nozzle duct as needed, thereby adjusting the angle thereof.

In addition, according to at least one of the embodiments of the present disclosure, since the nozzle duct is hinge-coupled to the inner cabinet, the nozzle duct can be hinge-rotated relative to the inner cabinet, thereby adjusting the angle thereof.

In addition, according to at least one of the embodiments of the present disclosure, since the nozzle duct is installed on one upper side of the inner cabinet, the inner space of the inner cabinet can be effectively utilized.

In addition, according to at least one of the embodiments of the present disclosure, since spraying is performed in the opposite directions from the end of the nozzle, spraying into the shoe can be effectively performed, regardless of the direction of the nozzle inserted into the shoe.

In addition, according to at least one of the embodiments of the present disclosure, since a pair of nozzles is installed to branch off from the nozzle duct, a pair of shoes can be processed simultaneously.

In addition, according to at least one of the embodiments of the present disclosure, since a plurality of nozzles is disposed to branch off from the distribution housing disposed to extend in one direction on the connecting passage, the steam or air supplied to the respective nozzles can be relatively evenly distributed.

In addition, according to at least one of the embodiments of the present disclosure, since a dry air duct coupled to a portion of the distribution housing on the connecting passage is further included, the air passing through the dehumidifying material can stably and effectively move to the distribution housing.

In addition, according to at least one of the embodiments of the present disclosure, since the steam generator is connected to the steam inlet formed in another portion of the distribution housing, steam and air can smoothly move to the distribution housing without interfering with each other.

In addition, according to at least one of the embodiments of the present disclosure, since the dehumidifying material is heated by the heater and since the regeneration passage is formed to allow the air generated according thereto to move therethrough, the air can be prevented from moving to the inner cabinet in the regeneration mode.

In addition, according to at least one of the embodiments of the present disclosure, since a dehumidifying-material housing accommodating the dehumidifying material is further included, the dehumidifying material can be stably accommodated and perform its function properly.

In addition, according to at least one of the embodiments of the present disclosure, since a pair of dehumidifying materials is disposed and since the connecting passage and the regeneration passage are formed in each dehumidifying material, the shoe care device can be operated in an optimal state depending on the situation, thereby further improving the efficiency of processing the shoes.

In addition, according to at least one of the embodiments of the present disclosure, since the angle of the nozzle duct protruding to the inside of the inner cabinet is adjusted by the first rotation-angle adjusting unit, the rotation angle of the nozzle duct with respect to the inner cabinet can be adjusted more precisely and stably.

In addition, according to at least one of the embodiments of the present disclosure, since the angle of the nozzle coupled to the nozzle duct is adjusted by the second rotation-angle adjusting unit, the rotation angle of the nozzle with respect to the nozzle duct can be adjusted more precisely and stably.

In addition, according to at least one of the embodiments of the present disclosure, since the nozzle duct is held in a holder installed in the upper portion of the inner cabinet, it is possible to prevent the nozzle duct from unnecessarily occupying the inner space of the inner cabinet.

In addition, according to at least one of the embodiments of the present disclosure, since the holder includes a holding base and a holding wall, the nozzle duct can be held more stably in the holder.

## Claims

1. A shoe care device comprising:
an inner cabinet configured to accommodate shoes therein;
a steam generator configured to supply steam into the inner cabinet;
an inlet through which air is sucked from the inner cabinet;
a nozzle installed inside the inner cabinet to be insertable into the shoe and configured to spray at least one of steam and air into the shoe;
a connecting passage connected from the inlet to the nozzle;
a blowing fan installed on the connecting passage to blow air from the inlet toward the nozzle; and
a dehumidifying material installed on the connecting passage to dehumidify the blown air.

2. The shoe care device according to claim 1, wherein the nozzle has a spray hole formed at an end thereof and is installed to adjust the direction in which the spray hole is inserted into the shoe.

3. The shoe care device according to claim 2, wherein the nozzle is configured to spray steam and air into the shoe in the case where the nozzle is inserted into the shoe from above.

4. The shoe care device according to claim 2, wherein the nozzle is configured to spray only air into the shoe in the case where the nozzle is inserted into the shoe from the bottom.

5. The shoe care device according to one of claims 1 to 4, further comprising a nozzle duct configured to form a portion of the connecting passage and installed to protrude to the inside of the inner cabinet so as to form a passage for steam and air,
wherein the nozzle is hinge-coupled to the nozzle duct.

6. The shoe care device according to claim 5, wherein the nozzle duct is hinge-coupled to the inner cabinet.

7. The shoe care device according to claim 6, wherein the nozzle duct is installed to protrude from one upper side of the inner cabinet.

8. The shoe care device according to one of claims 2 to 4, wherein the nozzle is formed in the opposite directions at the end of the nozzle.

9. The shoe care device according to claim 5, wherein a pair of nozzles is installed to branch off from the nozzle duct.

10. The shoe care device according to claim 5, further comprising a distribution housing configured to form a portion of the connecting passage and disposed to extend in one direction on the connecting passage so as to form a passage for steam and air,
wherein a plurality of nozzle ducts is installed to branch off from the distribution housing.

11. The shoe care device according to claim 10, further comprising a dry air duct configured to form a portion of the connecting passage and coupled to a portion of the distribution housing so as to guide air passing through the dehumidifying material to the distribution housing.

12. The shoe care device according to claim 11, wherein the steam inlet is formed in another portion of the distribution housing, and
wherein the steam generator is connected to the steam inlet.

13. The shoe care device according to one of claims 1 to 4, further comprising:
a heater installed to heat the dehumidifying material; and
a regeneration passage through which air passing through the dehumidifying material is blown to a portion other than the nozzle while the dehumidifying material is heated.

14. The shoe care device according to claim 13, further comprising a dehumidifying-material housing disposed on the connecting passage to accommodate the dehumidifying material and having the heater installed therein.

15. The shoe care device according to claim 13, wherein a pair of dehumidifying materials is disposed separately on branching connecting passages, and
wherein the heater is installed to heat each of the pair of dehumidifying materials.

16. The shoe care device according to claim 6, wherein a first rotation-angle adjusting unit is formed at a coupling portion between the nozzle duct and the inner cabinet so as to be relatively rotatable by cogwheel coupling therebetween.

17. The shoe care device according to claim 6, wherein a second rotation-angle adjusting unit is formed at a coupling portion between the nozzle duct and the nozzle so as to be relatively rotatable by cogwheel coupling therebetween.

18. The shoe care device according to claim 7, further comprising a holder installed on an upper portion of the inner cabinet and configured to hold the nozzle duct.

19. The shoe care device according to claim 18, wherein the holder comprises:
a holding base disposed on the upper portion of the inner cabinet; and
a holding wall formed to protrude downwards from the holding base and having a holding protrusion formed in a portion thereof, and
wherein a holding groove corresponding to the holding protrusion is formed on the side of the nozzle duct.
